(19) Europäisches Patentamt / European Patent Office / Office européen des brevets

(11) EP 3 774 774 B1

(12) **EUROPÄISCHE PATENTSCHRIFT**

(45) Veröffentlichungstag und Bekanntmachung des Hinweises auf die Patenterteilung:
**13.07.2022 Patentblatt 2022/28**

(21) Anmeldenummer: **19721540.3**

(22) Anmeldetag: **09.04.2019**

(51) Internationale Patentklassifikation (IPC):
***C07D 401/14*** *(2006.01)* ***C07D 487/04*** *(2006.01)*
***C07D 491/04*** *(2006.01)* ***C07D 495/04*** *(2006.01)*
***C09K 11/06*** *(2006.01)* ***H01L 51/00*** *(2006.01)*
***H01L 51/50*** *(2006.01)* ***H01L 29/786*** *(2006.01)*

(52) Gemeinsame Patentklassifikation (CPC):
**C07D 401/14; C07D 487/04; C07D 491/04; C07D 495/04; C09K 11/06; H01L 51/0067; H01L 51/0072;** C09K 2211/1007; C09K 2211/1029; C09K 2211/1033; C09K 2211/1037; C09K 2211/1059; H01L 51/5012; Y02E 10/549; Y02P 70/50

(86) Internationale Anmeldenummer:
**PCT/EP2019/058959**

(87) Internationale Veröffentlichungsnummer:
**WO 2019/197407 (17.10.2019 Gazette 2019/42)**

(54) **PYRIDIN-SUBSTITUIERTE 2,2'-DI-9H-CARBAZOL-9-YL-[1,1'-BIPHENYL]-DICARBONITRIL-DERIVATE UND VERWANDTE VERBINDUNGEN ZUR VERWENDUNG IN OPTOELEKTRONISCHEN VORRICHTUNGEN**

PYRIDINE SUBSTITUTED 2,2'-DI-9H-CARBAZOL-9-YL-[1,1'-BIPHENYL]-DICARBONITRILE DERIVATIVES AND RELATED COMPOUNDS FOR USE IN OPTOELECTRONIC DEVICES

DÉRIVÉS 2,2'-DI-9H-CARBAZOL-9-YL-[1,1'-BIPHÉNYL]-DICARBONITRILE SUBSTITUÉS PAR PYRIDINE ET COMPOSÉS SIMILAIRES À ÊTRE UTILISÉES DANS DES DISPOSITIFS OPTOÉLECTRONIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.04.2018 DE 102018108793**

(43) Veröffentlichungstag der Anmeldung:
**17.02.2021 Patentblatt 2021/07**

(73) Patentinhaber: **cynora GmbH**
**76646 Bruchsal (DE)**

(72) Erfinder: **FRIEDRICHS, Jan-Simon**
**76627 Karlsruhe (DE)**

(74) Vertreter: **Hoppe, Georg Johannes**
**Darani Anwaltskanzlei**
**Beuckestrasse 20**
**14163 Berlin (DE)**

(56) Entgegenhaltungen:
**EP-A1- 3 263 569 EP-A1- 3 287 451**

- **YONG JOO CHO ET AL: "Donor Interlocked Molecular Design for Fluorescence-like Narrow Emission in Deep Blue Thermally Activated Delayed Fluorescent Emitters", CHEMISTRY OF MATERIALS, Bd. 28, Nr. 15, 9. August 2016 (2016-08-09), Seiten 5400-5405, XP055305731, ISSN: 0897-4756, DOI: 10.1021/acs.chemmater.6b01484**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 774 774 B1

**Beschreibung**

Die Erfindung betrifft rein organische Moleküle und deren Verwendung in organischen lichtemittierenden Dioden (OLEDs) und in anderen optoelektronischen Vorrichtungen.

**Beschreibung**

Der vorliegenden Erfindung lag die Aufgabe zu Grunde, Moleküle bereitzustellen, die sich zur Verwendung in optoelektronischen Vorrichtungen eignen.

Diese Aufgabe wird mit der Erfindung, die eine neue Klasse von organischen Molekülen bereitstellt, gelöst.

Die erfindungsgemäßen organischen Moleküle sind rein organische Moleküle, weisen also keine Metallionen auf und grenzen sich so von den zur Verwendung in optoelektronischen Vorrichtungen bekannten Metallkomplexverbindungen ab.

Die erfindungsgemäßen organischen Moleküle zeichnen sich durch Emissionen im blauen, himmelblauen oder grünen Spektralbereich aus. Die Photolumineszenzquantenausbeuten der erfindungsgemäßen organischen Moleküle betragen insbesondere 60 % und mehr. Die erfindungsgemäßen Moleküle zeigen insbesondere thermisch aktivierte verzögerte Fluoreszenz (TADF). Die Verwendung der erfindungsgemäßen Moleküle in einer optoelektronischen Vorrichtung, beispielsweise einer organischen lichtemittierenden Diode (OLED), führt zu höheren Effizienzen der Vorrichtung. Entsprechende OLEDs weisen eine höhere Stabilität auf als OLEDs mit bekannten Emittermaterialien und vergleichbarer Farbe.

Unter dem blauen Spektralbereich wird hier der sichtbare Bereich von kleiner als 470 nm verstanden. Unter dem himmelblauen Spektralbereich wird hier der Bereich von 470 nm bis 499 nm verstanden. Unter dem grünen Spektralbereich wird hier der Bereich von 500 nm bis 599 nm verstanden. Dabei liegt das Emissionsmaximum im jeweiligen Bereich.

Die organischen Moleküle weisen eine Struktur gemäß Formel I auf oder bestehen aus einer Struktur gemäß Formel I:

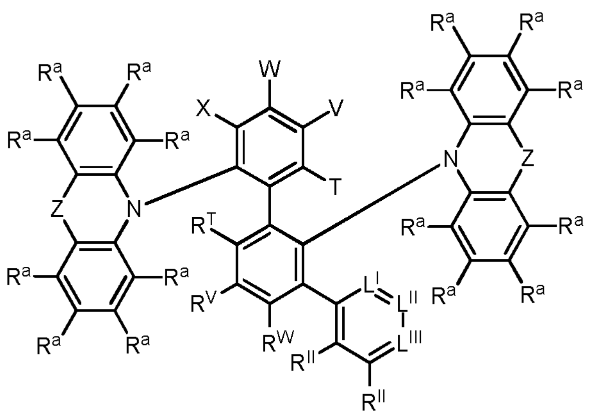

Formel I

Dabei gilt:

T ist ausgewählt aus einer Gruppe bestehend aus CN und $R^1$.
V ist ausgewählt aus einer Gruppe bestehend aus CN und $R^1$.
W ist ausgewählt aus einer Gruppe bestehend aus CN und $R^1$.
X ist ausgewählt aus einer Gruppe bestehend aus CN und $R^1$.
$R^T$ ist ausgewählt aus einer Gruppe bestehend aus CN und $R^2$.
$R^V$ ist ausgewählt aus einer Gruppe bestehend aus CN und $R^2$.
$R^W$ ist ausgewählt aus einer Gruppe bestehend aus CN und $R^2$.
L' ist ausgewählt aus einer Gruppe bestehend aus N und $CR^I$.
L" ist ausgewählt aus einer Gruppe bestehend aus N und $CR^I$.
$L^{III}$ ist ausgewählt aus einer Gruppe bestehend aus N und $CR^I$.

Z ist bei jedem Auftreten gleich oder verschieden eine direkte Bindung oder ausgewählt aus der Gruppe bestehend aus $CR^3R^4$, $C{=}CR^3R^4$, C=O, $C{=}NR^3$, $NR^3$, O, $SiR^3R^4$, S, S(O) und $S(O)_2$.

$R^1$, $R^2$, $R^I$, $R^{II}$ ist bei jedem Auftreten gleich oder verschieden H, Deuterium, eine lineare Alkylgruppe mit 1 bis 5 C-Atomen, eine lineare Alkenyl- oder Alkinylgruppe mit 2 bis 8 C-Atomen, eine verzweigte oder cyclische Alkyl-, Alkenyl- oder Alkinylgruppe mit 3 bis 10 C-Atomen, wobei ein oder mehrere H-Atome durch Deuterium ersetzt sein können, oder ein aromatisches oder heteroaromatisches Ringsystem mit 5 bis 15 aromatischen Ringatomen, das jeweils durch einen oder mehrere Reste $R^6$ substituiert sein kann.

$R^a$, $R^3$ und $R^4$ ist bei jedem Auftreten gleich oder verschieden H, Deuterium, $N(R^5)_2$, OH, $Si(R^5)_3$, $B(OR^5)_2$, $OSO_2R^5$, $CF_3$, CN, F, Br, I, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können; oder ein aromatisches oder heteroaromatisches Ringsystem mit 5 bis 60 aromatischen Ringatomen, das jeweils durch einen oder mehrere Reste $R^5$ substituiert sein kann, oder eine Aryloxy- oder Heteroaryloxygruppe mit 5 bis 60 aromatischen Ringatomen, die durch einen oder mehrere Reste $R^5$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^5$ substituiert sein kann.

$R^5$ ist bei jedem Auftreten gleich oder verschieden H, Deuterium, $N(R^6)_2$, OH, $Si(R^6)_3$, $B(OR^6)_2$, $OSO_2R^6$, $CF_3$, CN, F, Br, I, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^6$ substituiert sein kann, wobei eine oder mehrere nicht benachbarte $CH_2$-Gruppen durch $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sein können und wobei ein oder mehrere H-Atome durch Deuterium, CN, $CF_3$ oder $NO_2$ ersetzt sein können; oder ein aromatisches oder heteroaromatisches Ringsystem mit 5 bis 60 aromatischen Ringatomen, das jeweils durch einen oder mehrere Reste $R^6$ substituiert sein kann, oder eine Aryloxy- oder Heteroaryloxygruppe mit 5 bis 60 aromatischen Ringatomen, die durch einen oder mehrere Reste $R^6$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^6$ substituiert sein kann.

$R^6$ ist bei jedem Auftreten gleich oder verschieden H, Deuterium, OH, $CF_3$, CN, F, eine lineare Alkyl-, Alkoxy- oder Thioalkoxygruppe mit 1 bis 5 C-Atomen oder eine lineare Alkenyl- oder Alkinylgruppe mit 2 bis 5 C-Atomen oder eine verzweigte oder cyclische Alkyl-, Alkenyl-, Alkinyl-, Alkoxy- oder Thioalkoxygruppe mit 3 bis 5 C-Atomen, wobei ein oder mehrere H-Atome durch Deuterium, CN, $CF_3$ oder $NO_2$ ersetzt sein können; oder ein aromatisches oder heteroaromatisches Ringsystem mit 5 bis 60 aromatischen Ringatomen oder eine Aryloxy- oder Heteroaryloxygruppe mit 5 bis 60 aromatischen Ringatomen oder eine Diarylaminogruppe, Diheteroarylaminogruppe oder Arylheteroarylaminogruppe mit 10 bis 40 aromatischen Ringatomen.

Erfindungsgemäß kann jeder der Reste $R^a$, $R^3$, $R^4$ oder $R^5$ auch mit einem oder mehreren weiteren Resten $R^a$, $R^3$, $R^4$ oder $R^5$ ein mono- oder polycyclisches, aliphatisches, aromatisches und/oder benzoanelliertes Ringsystem bilden.

Erfindungsgemäß ist genau ein Rest ausgewählt aus T, V, W und X gleich CN, genau ein Rest ausgewählt aus $R^T$, $R^V$ und $R^W$ gleich CN, und genau ein Rest ausgewählt aus $L^I$, L" und $L^{III}$ gleich N.

In einer Ausführungsform des organischen Moleküls ist $R^1$, $R^2$, $R^{II}$ und $R^I$ bei jedem Auftreten gleich oder verschieden H, Methyl oder Phenyl.

In einer Ausführungsform des organischen Moleküls ist $R^1$ gleich H bei jedem Auftreten.

In einer Ausführungsform des organischen Moleküls ist $R^2$ gleich H bei jedem Auftreten.

In einer Ausführungsform des organischen Moleküls ist $R^I$ gleich H bei jedem Auftreten.

In einer Ausführungsform des organischen Moleküls ist $R^{II}$ gleich H bei jedem Auftreten.

In einer Ausführungsform des organischen Moleküls ist W gleich CN.

In einer Ausführungsform des organischen Moleküls ist V gleich CN.

In einer Ausführungsform des organischen Moleküls ist X gleich CN.

In einer Ausführungsform des organischen Moleküls ist T gleich CN.

In einer Ausführungsform des organischen Moleküls ist $R^V$ gleich CN.

In einer Ausführungsform des organischen Moleküls ist $R^W$ gleich CN.

In einer Ausführungsform des organischen Moleküls ist $R^T$ gleich CN.

In einer Ausführungsform des organischen Moleküls sind V und $R^V$ jeweils gleich CN.

In einer Ausführungsform des organischen Moleküls sind W und $R^V$ jeweils gleich CN.

In einer Ausführungsform des organischen Moleküls ist $L^I$ gleich N.

In einer Ausführungsform des organischen Moleküls ist L" gleich N.

In einer Ausführungsform des organischen Moleküls ist $L^{III}$ gleich N.

In einer Ausführungsform des organischen Moleküls weisen die organischen Moleküle eine Struktur gemäß

Formel II auf oder bestehen aus einer Struktur gemäß Formel II:

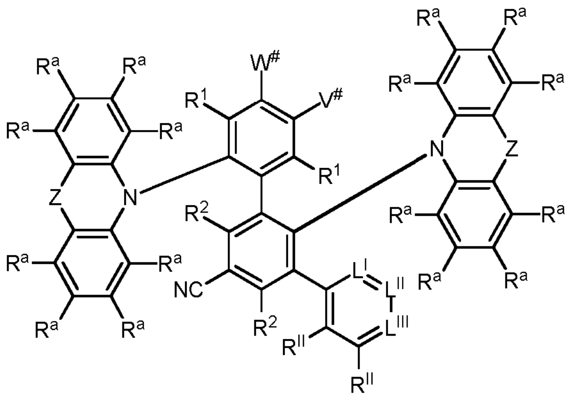

Formel II

wobei $W^{\#}$ ausgewählt ist aus einer Gruppe bestehend aus CN und $R^1$;
und $V^{\#}$ ausgewählt ist aus einer Gruppe bestehend aus CN und $R^1$;
wobei genau ein Rest ausgewählt aus $W^{\#}$ und $V^{\#}$ gleich CN ist;
und wobei ansonsten die für Formel I genannten Definitionen gelten.

In einer weiteren Ausführungsform des organischen Moleküls weist das organische Molekül bei jedem Auftreten gleich oder verschieden eine Struktur der Formel IIa auf oder besteht aus einer Struktur der Formel IIa:

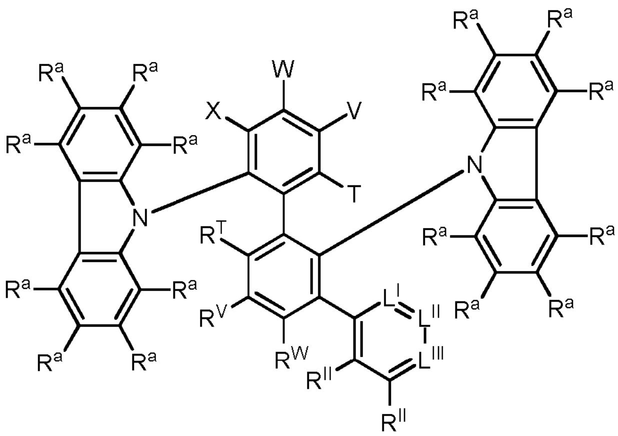

Formel IIa

wobei die für Formel I genannten Definitionen gelten.

In einer weiteren Ausführungsform des organischen Moleküls weist das organische Molekül bei jedem Auftreten gleich oder verschieden eine Struktur der Formel IIaa auf oder besteht aus einer Struktur der Formel IIaa:

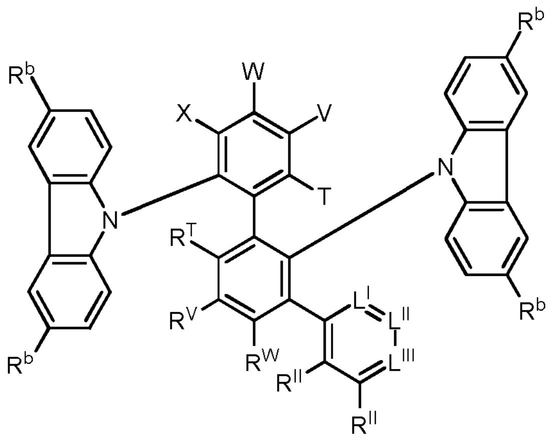

Formel IIaa

wobei

$R^b$ bei jedem Auftreten gleich oder verschieden ist $N(R^5)_2$, OH, $Si(R^5)_3$, $B(OR^5)_2$, $OSO_2R^5$, $CF_3$, CN, F, Br, I, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^5$ substituiert sein kann, wobei eine oder mehrere nicht benachbarte $CH_2$-Gruppen durch $R^5C{=}CR^5$, C=C, $Si(R^5)_2$, $Ge(R^5)_2$, $Sn(R^5)_2$, C=O, C=S, C=Se, $C{=}NR^5$, $P({=}O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können; oder ein aromatisches oder heteroaromatisches Ringsystem mit 5 bis 60 aromatischen Ringatomen, das jeweils durch einen oder mehrere Reste $R^5$ substituiert sein kann, oder eine Aryloxy- oder Heteroaryloxygruppe mit 5 bis 60 aromatischen Ringatomen, die durch einen oder mehrere Reste $R^5$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^5$ substituiert sein kann. Ansonsten gelten die oben genannten Definitionen.

In einer weiteren Ausführungsform des organischen Moleküls weist das organische Molekül bei jedem Auftreten gleich oder verschieden eine Struktur der Formel IIaaa auf oder besteht aus einer Struktur der Formel IIaaa:

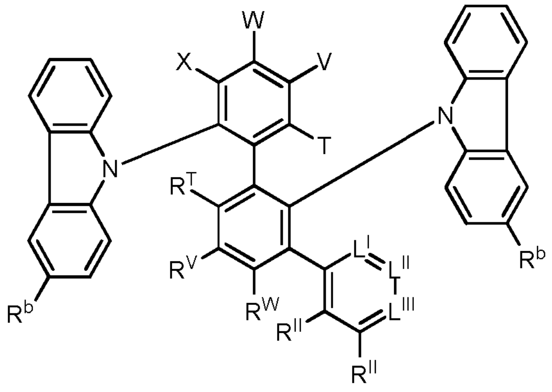

Formel IIaaa

wobei die oben genannten Definitionen gelten.

In einer weiteren Ausführungsform der erfindungsgemäßen organischen Moleküle weisen die erfindungsgemäßen organischen Moleküle eine Struktur ausgewählt aus der Gruppe der Formel IIb, der Formel IIb-2, der Formel IIb-3 oder der der Formel IIb-4 auf oder bestehen daraus:

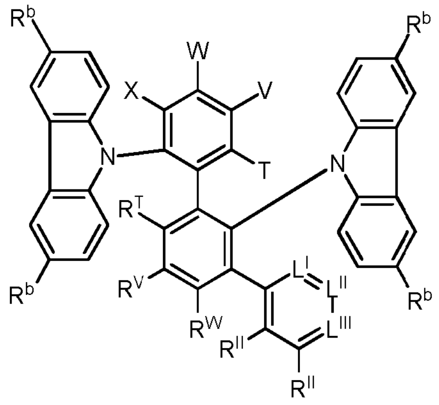

Formel IIb

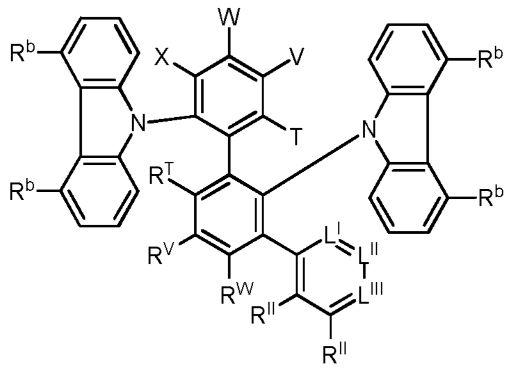

Formel IIb-2

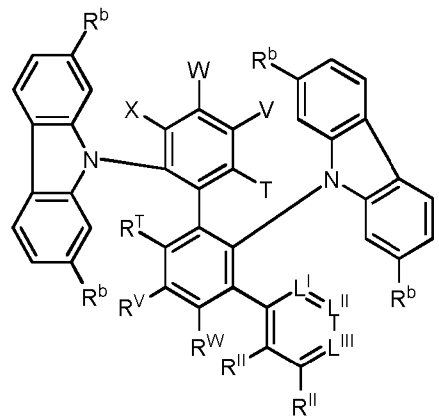

Formel IIb-3

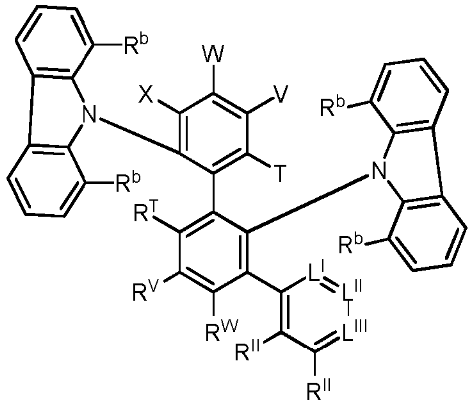

Formel IIb-4

wobei die oben genannten Definitionen gelten.

In einer weiteren Ausführungsform der erfindungsgemäßen organischen Moleküle weisen die erfindungsgemäßen organischen Moleküle eine Struktur ausgewählt aus der Gruppe der Formel IIc, der Formel IIc-2, der Formel IIc-3 oder der Formel IIc-4 auf oder bestehen daraus:

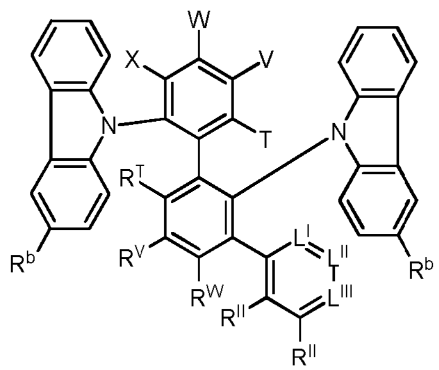

Formel IIc

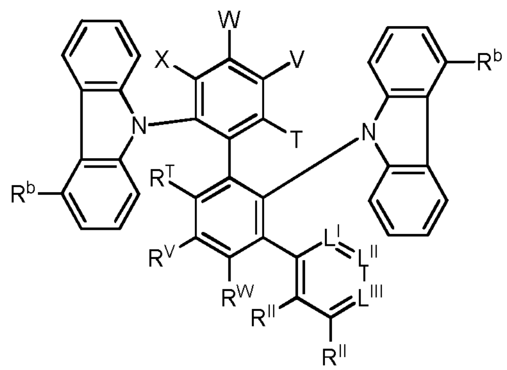

Formel IIc-2

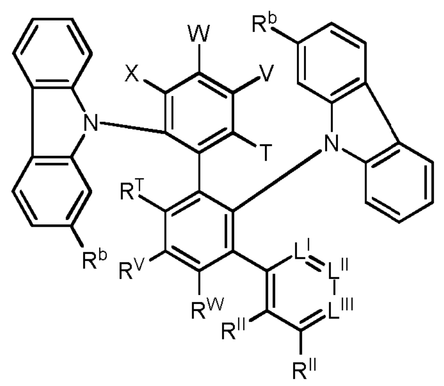

Formel IIc-3

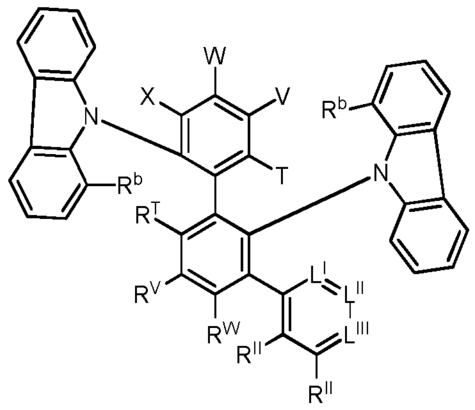

Formel IIc-4

wobei die oben genannten Definitionen gelten.

In einer weiteren Ausführungsform der erfindungsgemäßen organischen Moleküle ist $R^b$ bei jedem Auftreten unabhängig voneinander ausgewählt aus der Gruppe bestehend aus Me, $^i$Pr, $^t$Bu, CN, $CF_3$, Ph (phenyl), das jeweils durch einen oder mehrere Reste ausgewählt aus Me, $^i$Pr, $^t$Bu, CN, $CF_3$ oder Ph substituiert sein kann, Pyridinyl, das jeweils durch einen oder mehrere Reste ausgewählt aus Me, $^i$Pr, $^t$Bu, CN, $CF_3$ oder Ph substituiert sein kann, Pyrimidinyl, das jeweils durch einen oder mehrere Reste ausgewählt aus Me, $^i$Pr, $^t$Bu, CN, $CF_3$ oder Ph substituiert sein kann, Carbazolyl, das jeweils durch einen oder mehrere Reste ausgewählt aus Me, $^i$Pr, $^t$Bu, CN, $CF_3$ oder Ph substituiert sein kann, Triazinyl, das jeweils durch einen oder mehrere Reste ausgewählt aus Me, $^i$Pr, $^t$Bu, CN, $CF_3$ oder Ph substituiert sein kann, und $N(Ph)_2$.

Im Folgenden sind beispielhaft Ausführungsformen der erfindungsgemäßen organischen Moleküle gezeigt:

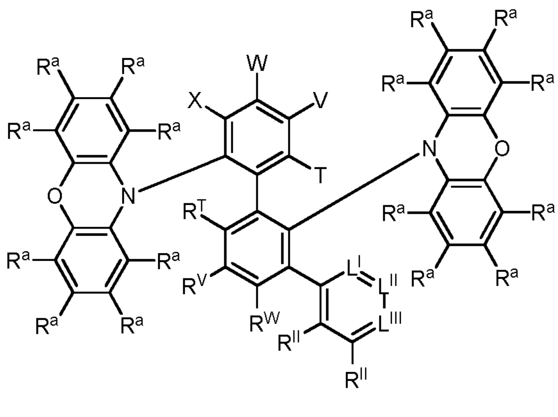

,

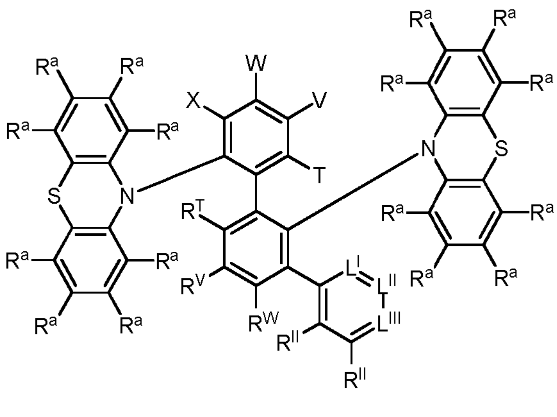

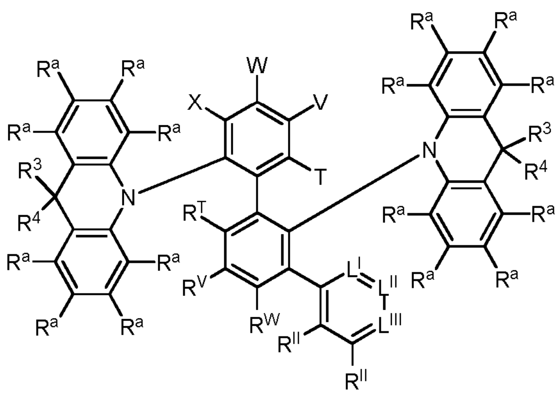

,

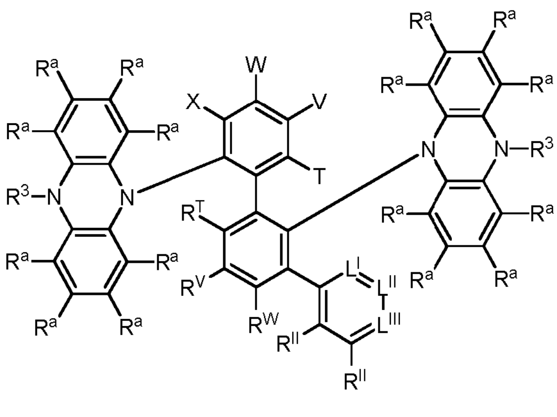

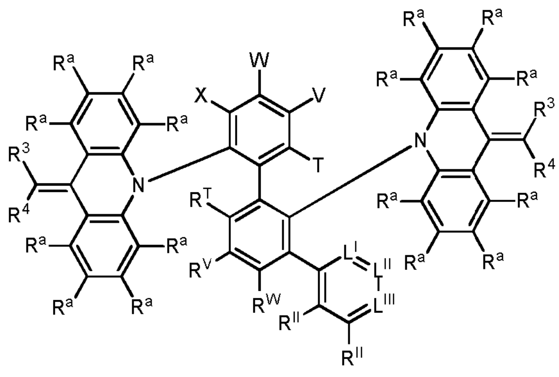
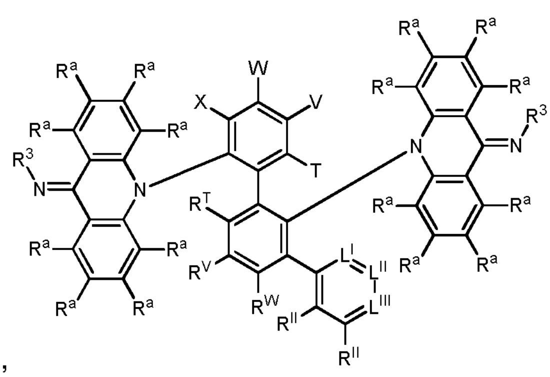
,

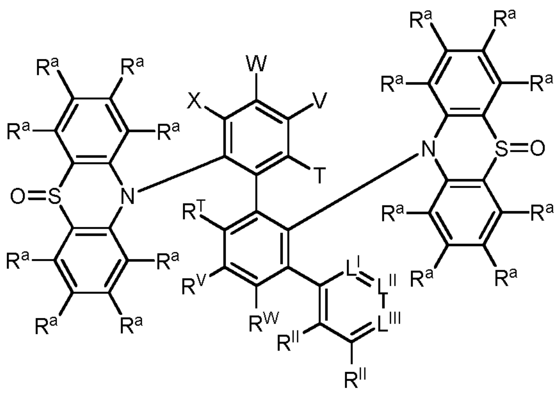

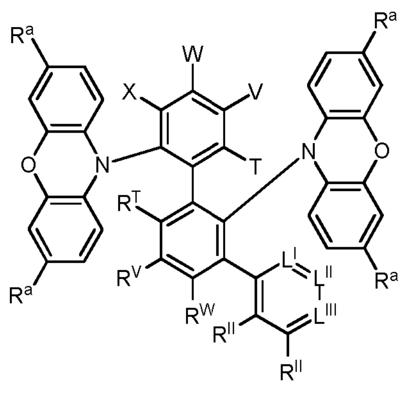
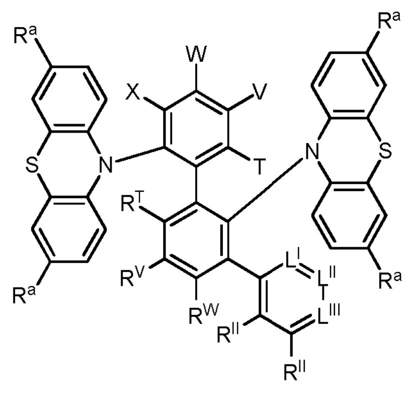

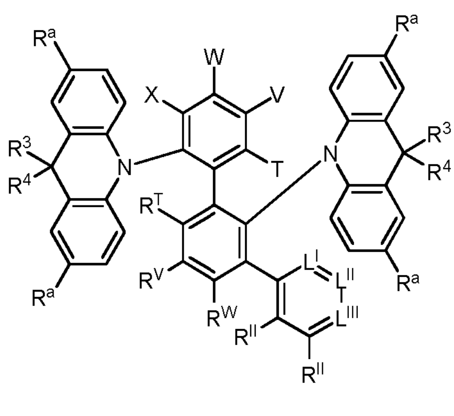
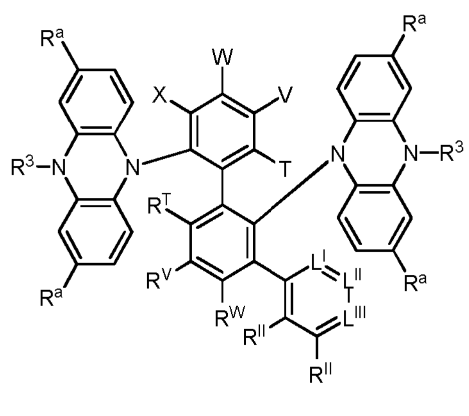

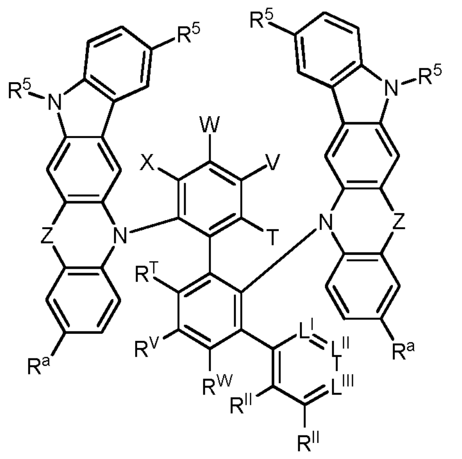

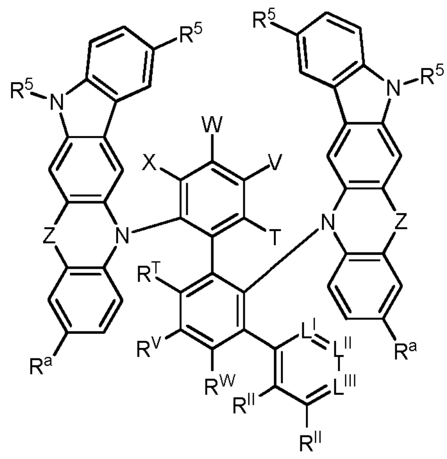

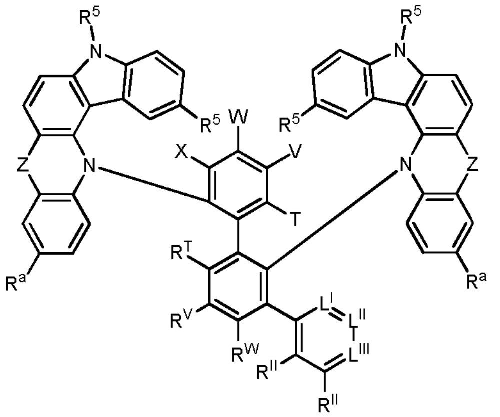

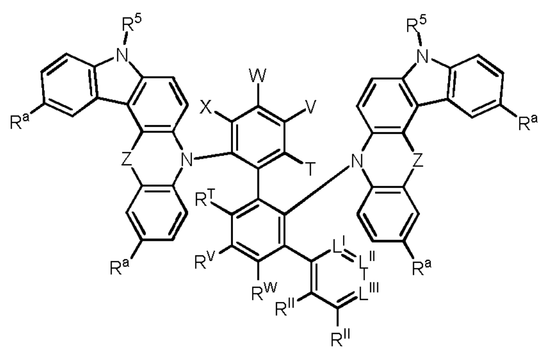

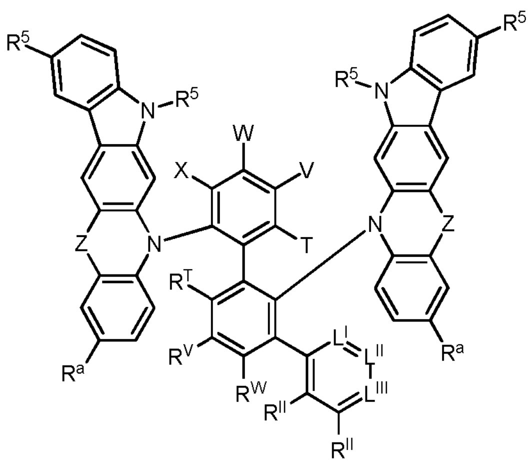

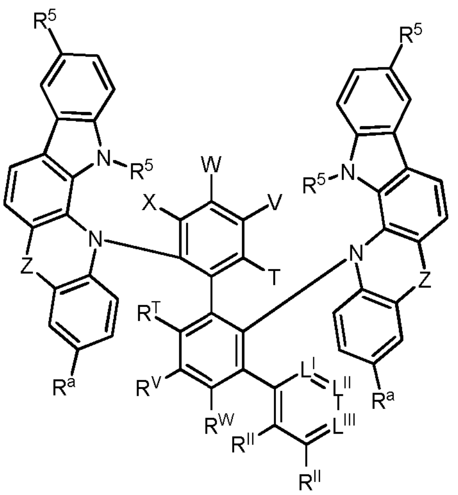

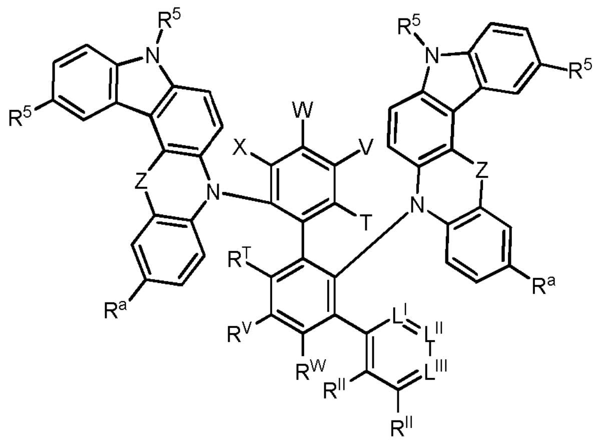

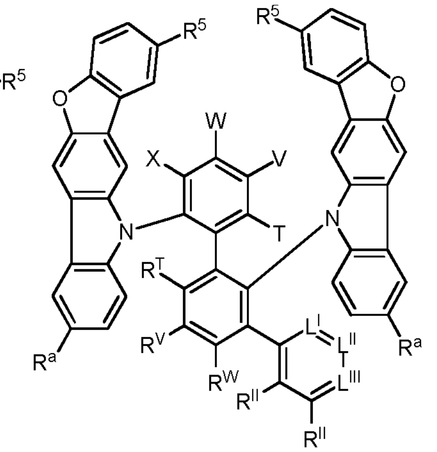

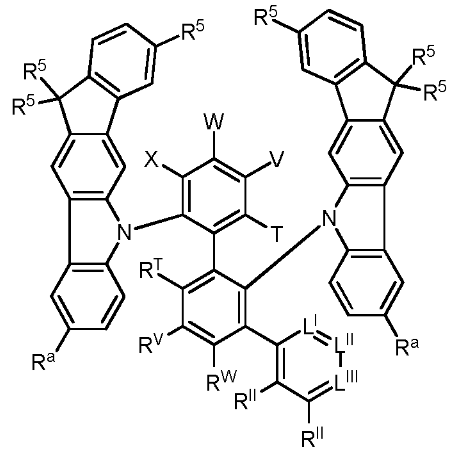

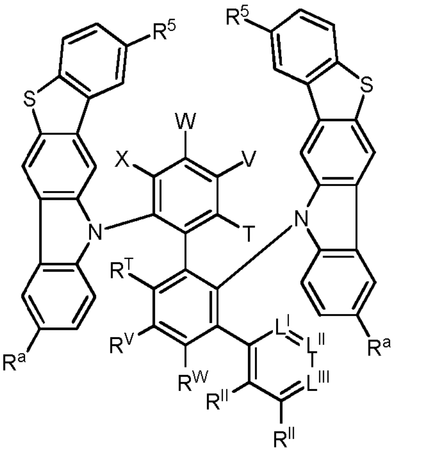

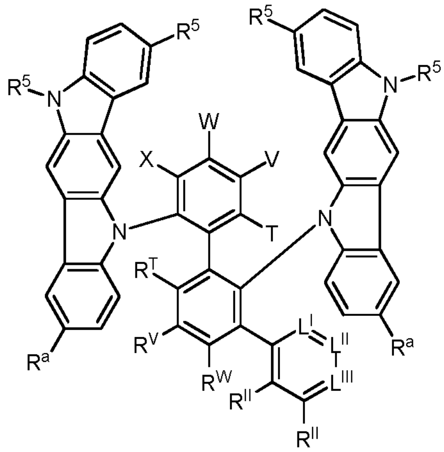

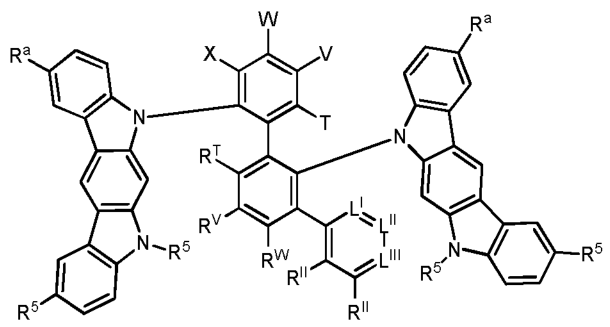

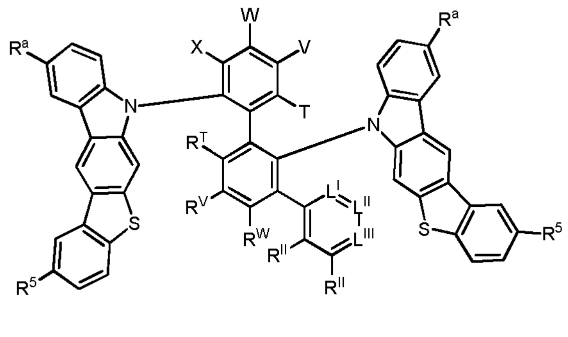

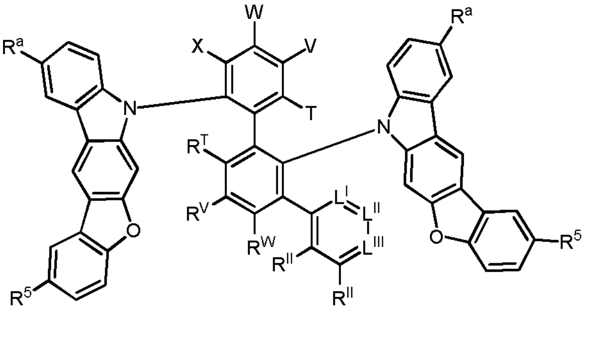

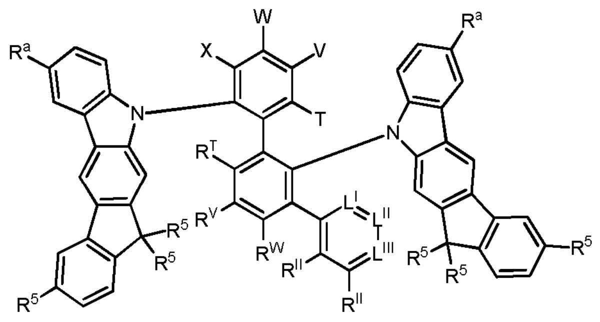

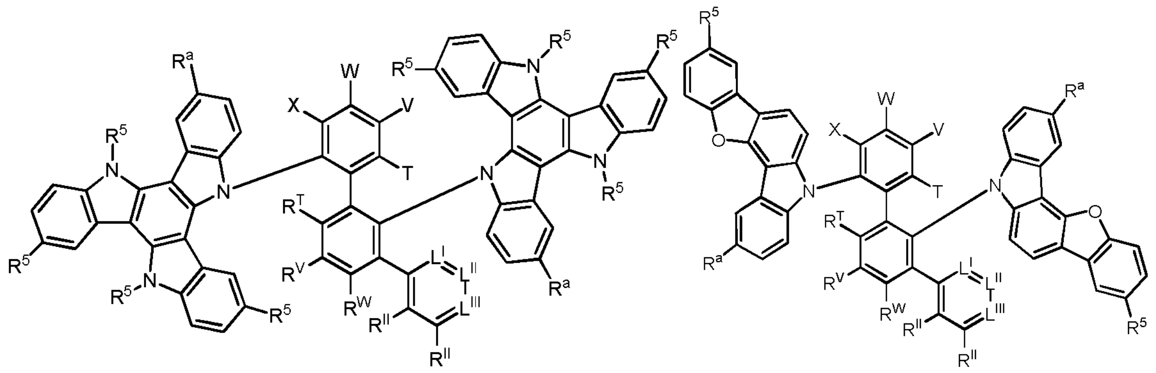

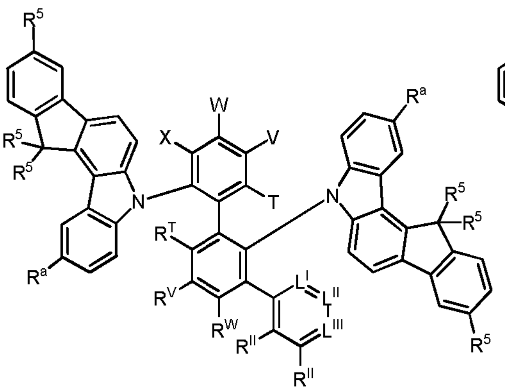

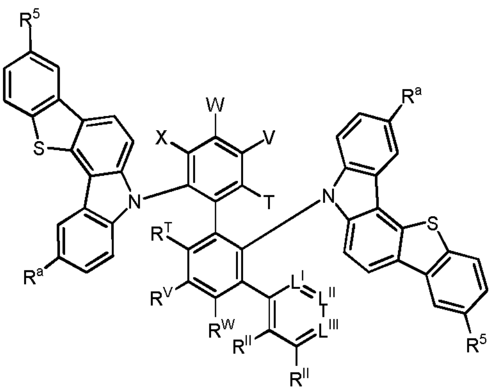

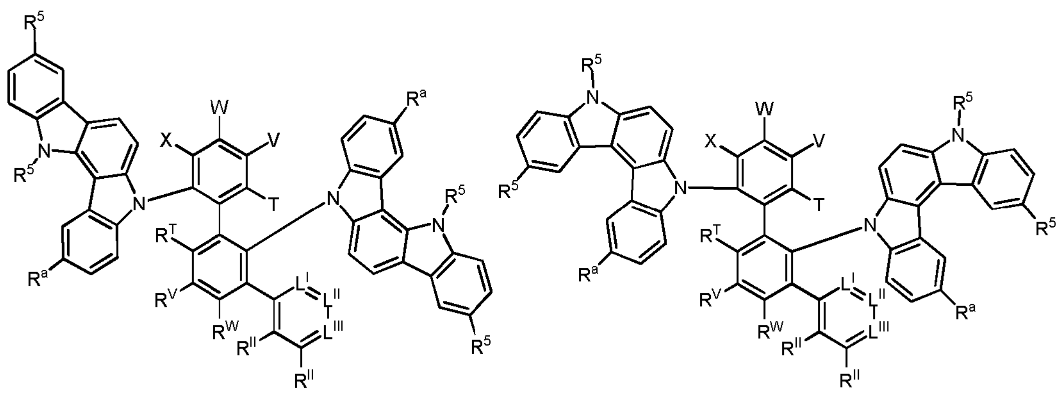

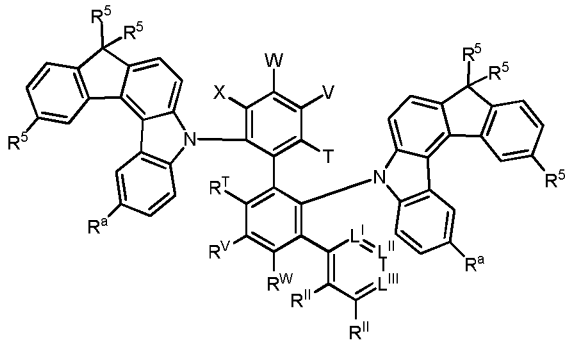

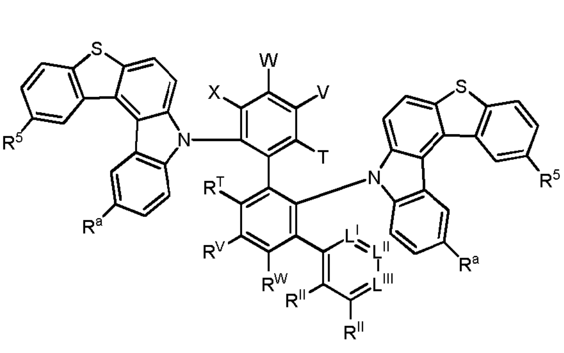

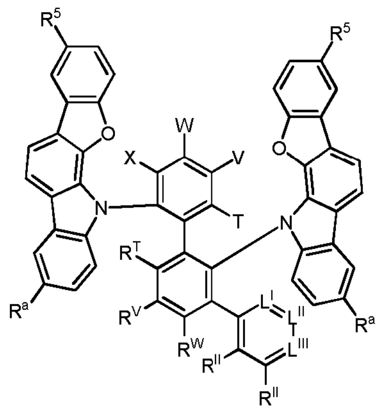

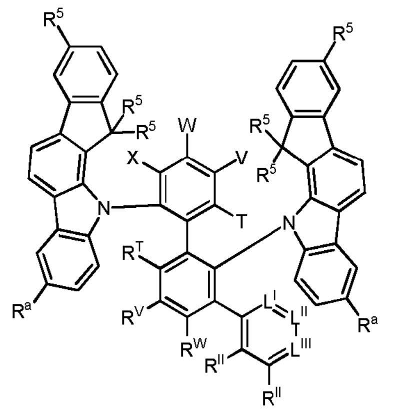

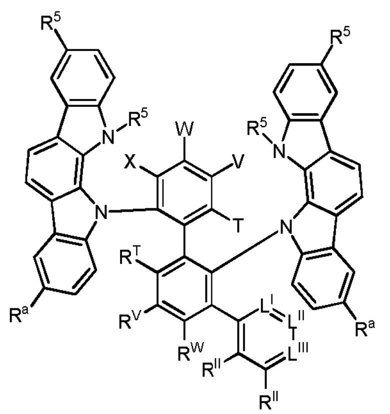

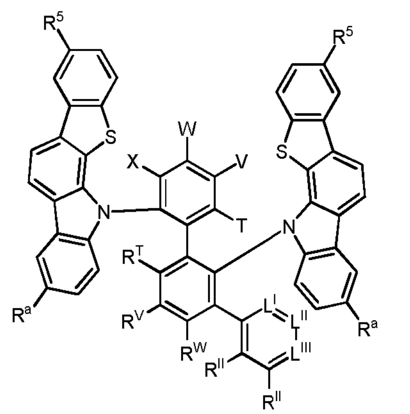

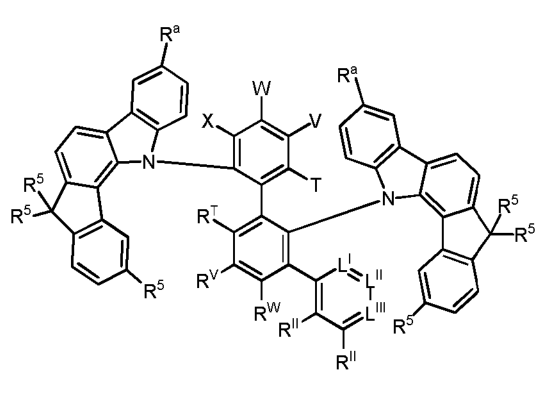

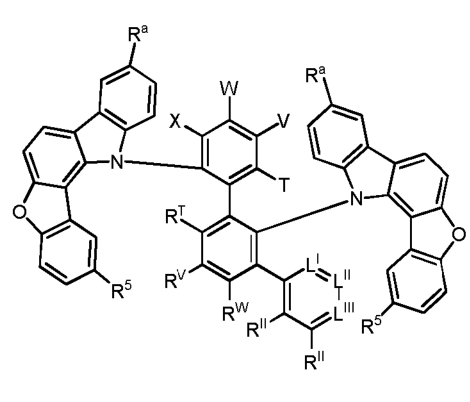

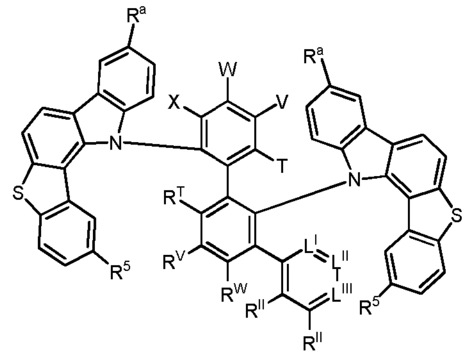

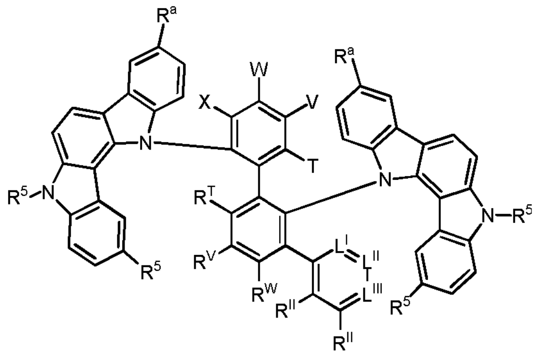

wobei für Z, $R^a$, $R^3$, $R^4$ und $R^5$ die oben genannten Definitionen gelten. In einer Ausführungsform der erfindungsgemäßen organischen Moleküle ist der Rest $R^5$ bei jedem Auftreten gleich oder verschieden ausgewählt aus der Gruppe bestehend aus H, Methyl, Ethyl, Phenyl und Mesityl. In einer Ausführungsform ist $R^a$ bei jedem Auftreten gleich oder verschieden ausgewählt aus der Gruppe bestehend aus H, Methyl (Me), i-Propyl ($CH(CH_3)_2$) ($^i$Pr), t-Butyl ($^t$Bu), Phenyl (Ph), CN, $CF_3$ und Diphenylamin ($NPh_2$).

In einer Ausführungsform weisen die erfindungsgemäßen organischen Moleküle eine Struktur der Formel III auf:

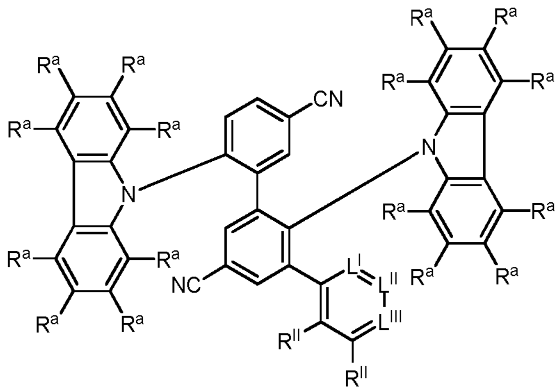

Formel III

wobei die oben genannten Definitionen gelten.

In einer weiteren Ausführungsform weisen die erfindungsgemäßen organischen Moleküle eine Struktur der Formel IIIa, Formel IIIb oder Formel IIIc auf:

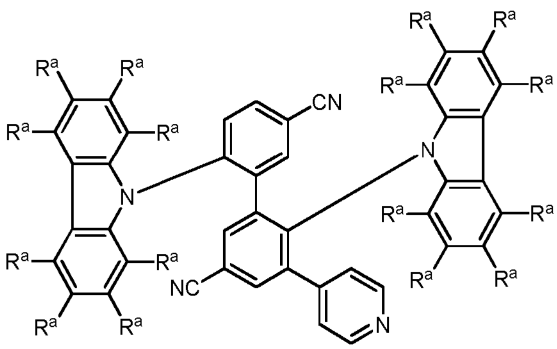

Formel IIIa

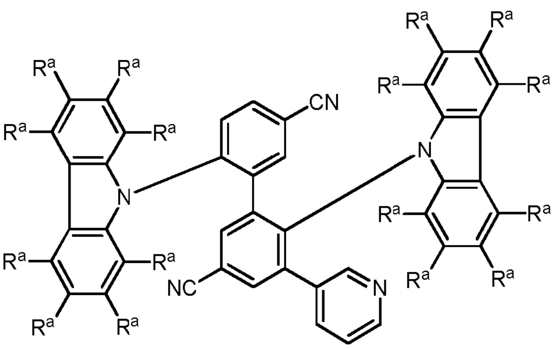

Formel IIIb

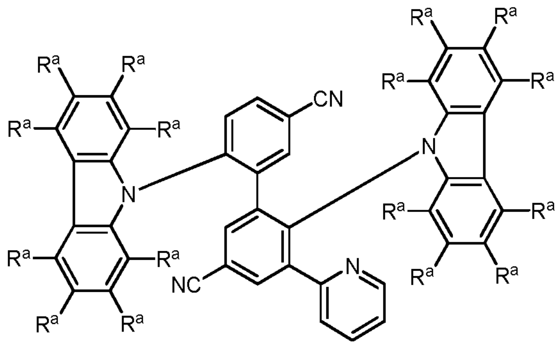

Formel IIIc

wobei die oben genannten Definitionen gelten.

In einer weiteren Ausführungsform weisen die erfindungsgemäßen organischen Moleküle eine Struktur der Formel IIIa-1, Formel IIIb-1 oder Formel IIIc-1 auf:

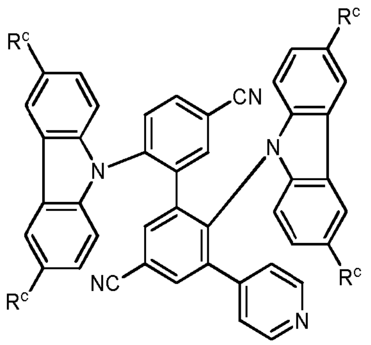

Formel IIIa-1

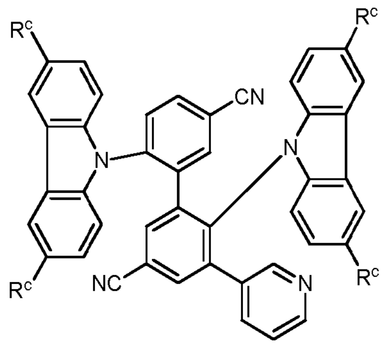

Formel IIIb-1

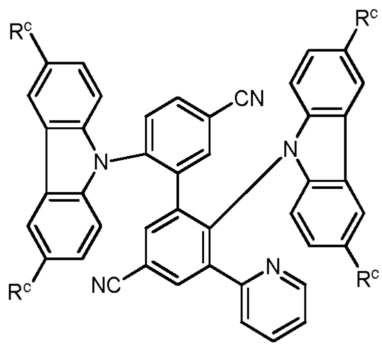

Formel IIIc-1

wobei

$R^c$ bei jedem Auftreten unabhängig voneinander ausgewählt aus der Gruppe bestehend aus Me, $^i$Pr, $^t$Bu, CN, $CF_3$, Ph, das jeweils durch einen oder mehrere Reste ausgewählt aus Me, $^i$Pr, $^t$Bu, CN, $CF_3$ oder Ph substituiert sein kann, Pyridinyl, das jeweils durch einen oder mehrere Reste ausgewählt aus Me, $^i$Pr, $^t$Bu, CN, $CF_3$ oder Ph substituiert sein kann, Pyrimidinyl, das jeweils durch einen oder mehrere Reste ausgewählt aus Me, $^i$Pr, $^t$Bu, CN, $CF_3$ oder Ph substituiert sein kann, Carbazolyl, das jeweils durch einen oder mehrere Reste ausgewählt aus Me, $^i$Pr, $^t$Bu, CN, $CF_3$ oder Ph substituiert sein kann, Triazinyl, das jeweils durch einen oder mehrere Reste ausgewählt aus Me, $^i$Pr, $^t$Bu, CN, $CF_3$ oder Ph substituiert sein kann, und $N(Ph)_2$ ist.

In einer weiteren Ausführungsform weisen die erfindungsgemäßen organischen Moleküle eine Struktur der Formel IIIa-2, Formel IIIb-2 oder Formel IIIc-2 auf:

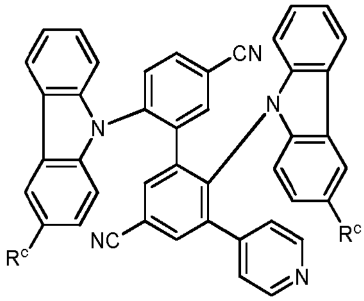

Formel IIIa-2

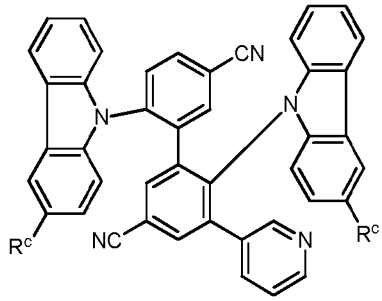

Formel IIIb-2

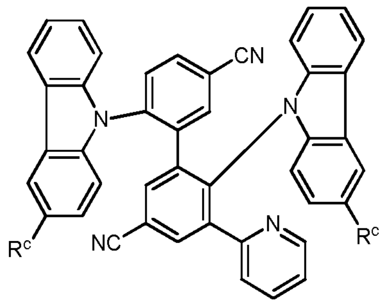

Formel IIIc-2

wobei die oben genannten Definitionen gelten.

In einer weiteren Ausführungsform weisen die erfindungsgemäßen organischen Moleküle eine Struktur der Formel IV auf:

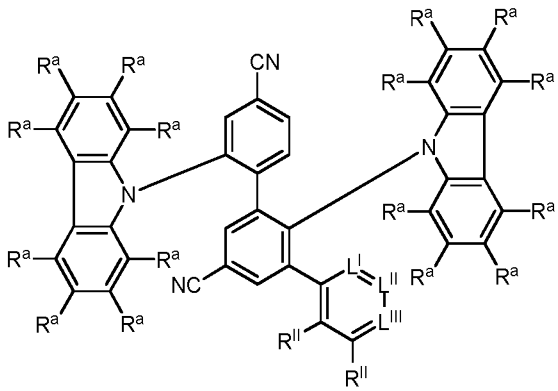

Formel IV

wobei die oben genannten Definitionen gelten.

In einer weiteren Ausführungsform weisen die erfindungsgemäßen organischen Moleküle eine Struktur der Formel IVa, Formel IVb oder Formel IVc auf:

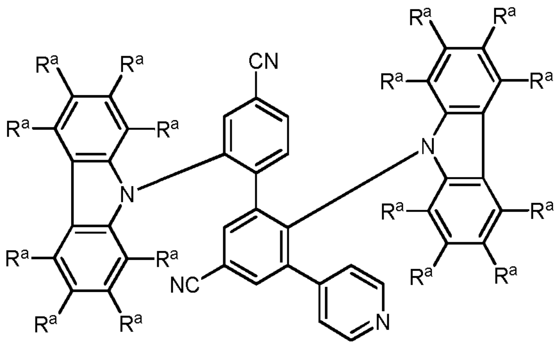

Formel IVa

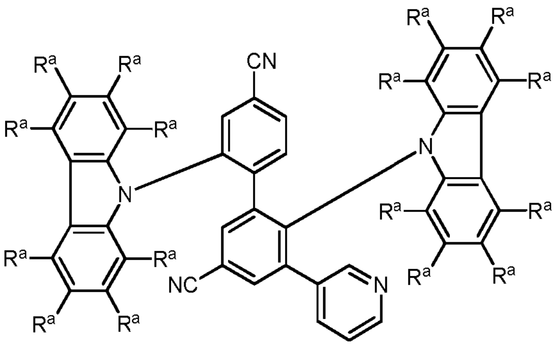

Formel IVb

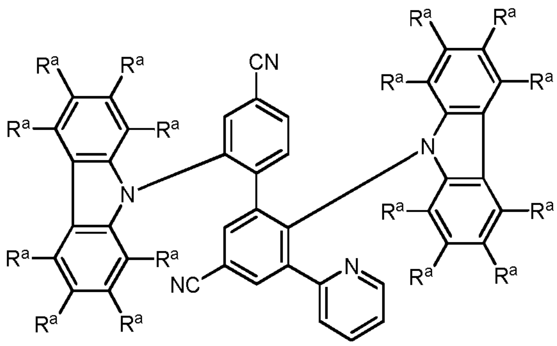

Formel IVc

wobei die oben genannten Definitionen gelten.

In einer weiteren Ausführungsform weisen die erfindungsgemäßen organischen Moleküle eine Struktur der Formel IVa-1, Formel IVb-1 oder Formel IVc-1 auf:

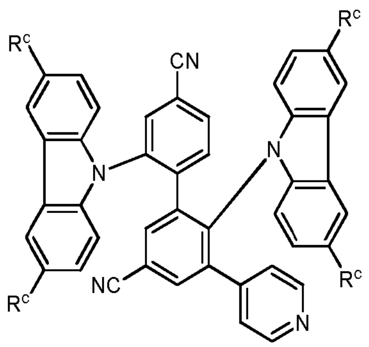

Formel IVa-1

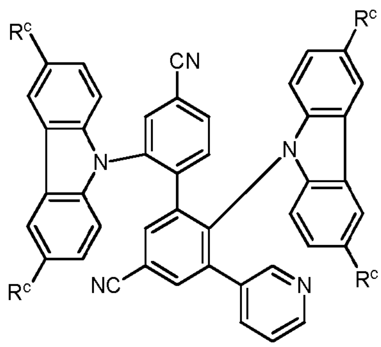

Formel IVb-1

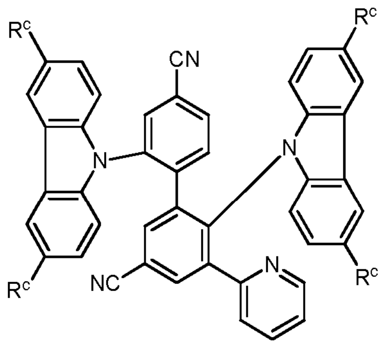

Formel IVc-1

wobei die oben genannten Definitionen gelten.

In einer weiteren Ausführungsform weisen die erfindungsgemäßen organischen Moleküle eine Struktur der Formel IVa-2, Formel IVb-2 oder Formel IVc-2 auf:

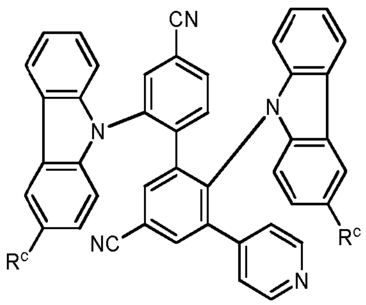

Formel IVa-2

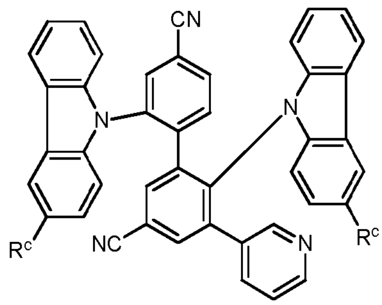

Formel IVb-2

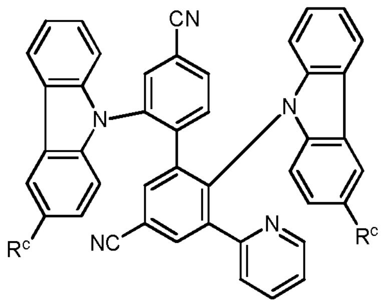

Formel IVc-2

wobei die oben genannten Definitionen gelten.

In einer weiteren Ausführungsform weisen die erfindungsgemäßen organischen Moleküle eine Struktur der Formel V auf:

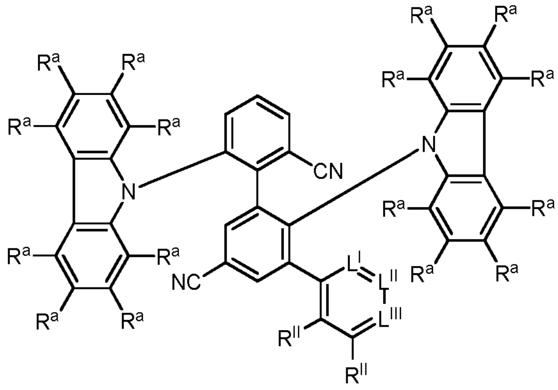

Formel V

wobei die oben genannten Definitionen gelten.

In einer weiteren Ausführungsform weisen die erfindungsgemäßen organischen Moleküle eine Struktur der Formel Va, Formel Vb oder Formel Vc auf:

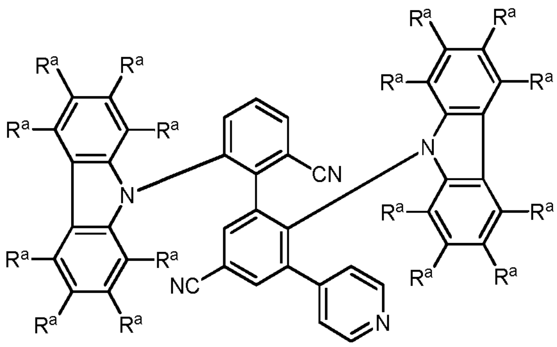

Formel Va

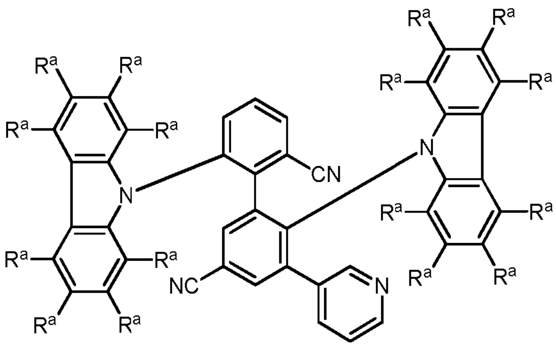

Formel Vb

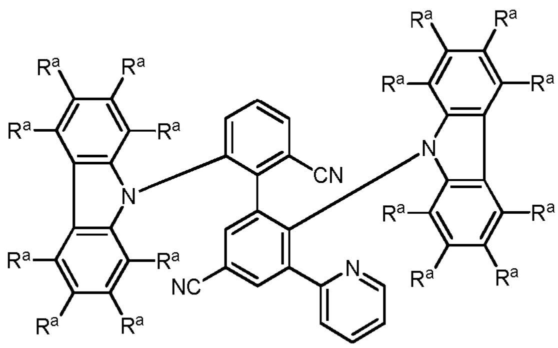

Formel Vc

wobei die oben genannten Definitionen gelten.

In einer weiteren Ausführungsform weisen die erfindungsgemäßen organischen Moleküle eine Struktur der Formel Va-1, Formel Vb-1 oder Formel Vc-1 auf:

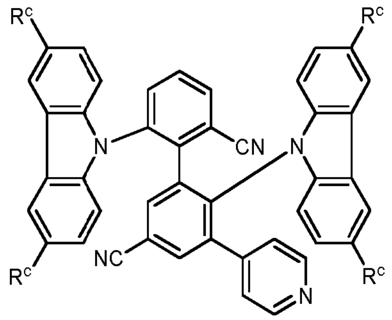

Formel Va-1

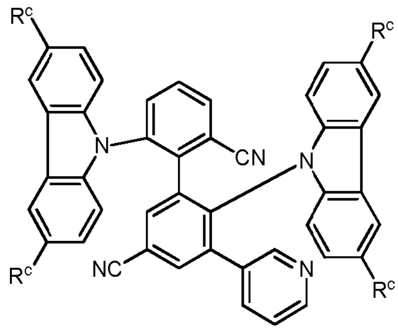

Formel Vb-1

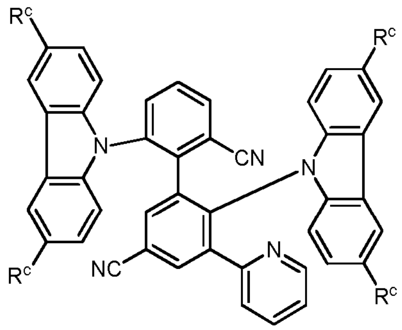

Formel Vc-1

wobei die oben genannten Definitionen gelten.

In einer weiteren Ausführungsform weisen die erfindungsgemäßen organischen Moleküle eine Struktur der Formel Va-2, Formel Vb-2 oder Formel Vc-2 auf:

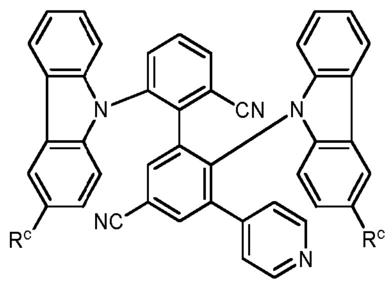

Formel Va-2

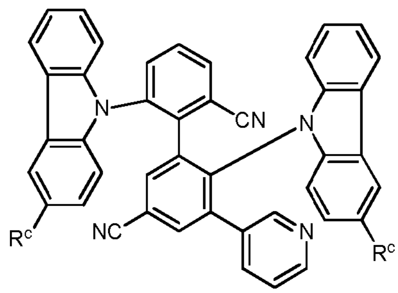

Formel Vb-2

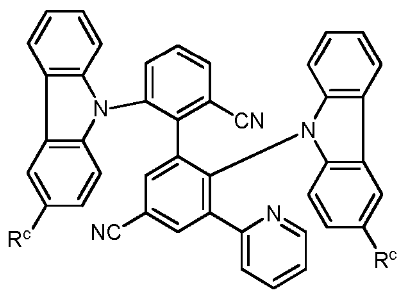

Formel Vc-2

wobei die oben genannten Definitionen gelten.

In einer weiteren Ausführungsform weisen die erfindungsgemäßen organischen Moleküle eine Struktur der

Formel VI auf:

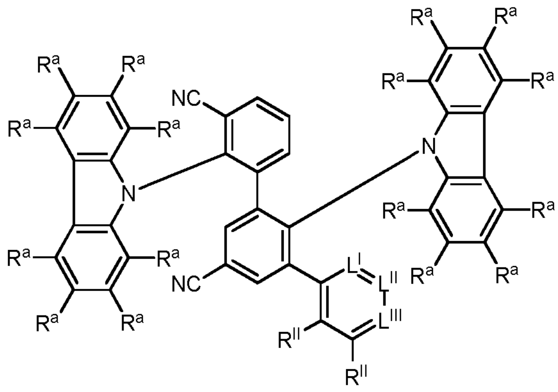

Formel VI

wobei die oben genannten Definitionen gelten.

In einer weiteren Ausführungsform weisen die erfindungsgemäßen organischen Moleküle eine Struktur der Formel VIa, Formel VIb oder Formel VIc auf:

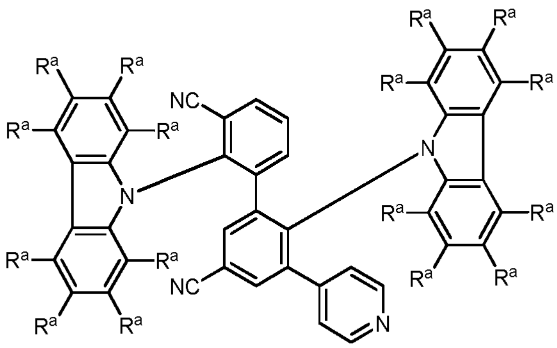

Formel VIa

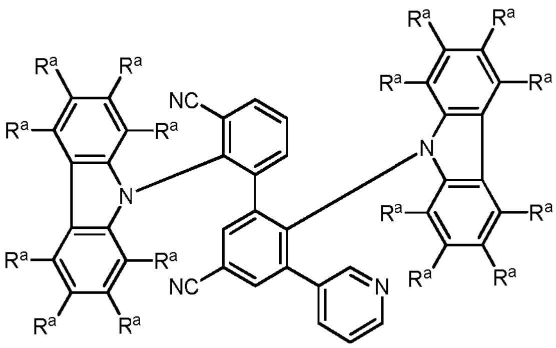

Formel VIb

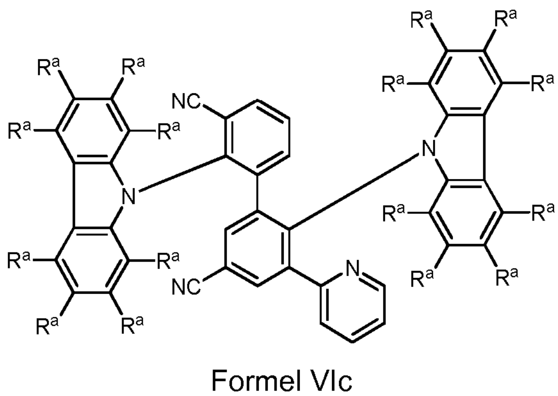

Formel VIc

wobei die oben genannten Definitionen gelten.

In einer weiteren Ausführungsform weisen die erfindungsgemäßen organischen Moleküle eine Struktur der Formel VIa-1, Formel VIb-1 oder Formel VIc-1 auf:

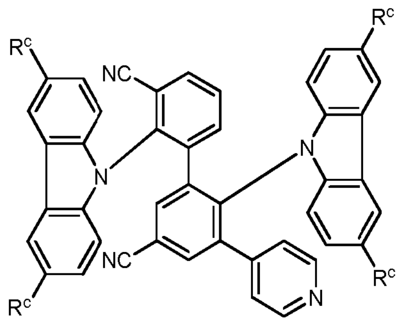

Formel VIa-1

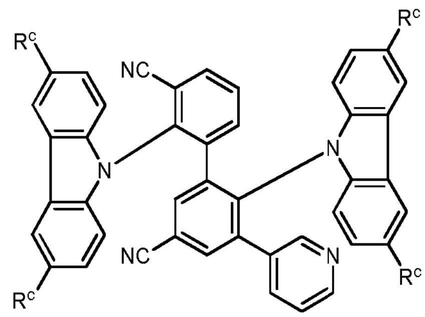

Formel VIb-1

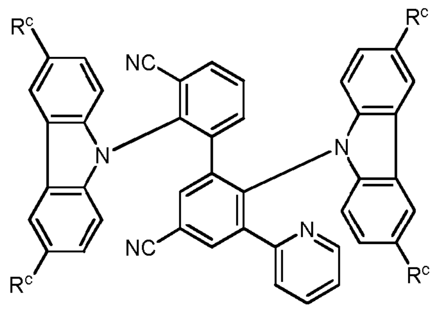

Formel VIc-1

wobei die oben genannten Definitionen gelten.

In einer weiteren Ausführungsform weisen die erfindungsgemäßen organischen Moleküle eine Struktur der Formel VIa-2, Formel VIb-2 oder Formel VIc-2 auf:

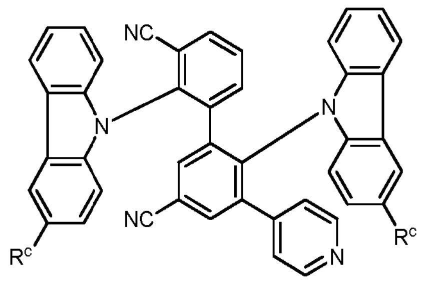

Formel VIa-2

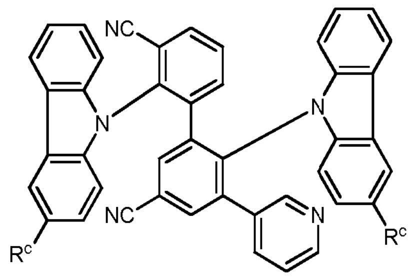

Formel VIb-2

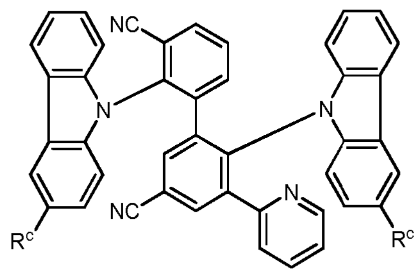

Formel VIc-2

wobei die oben genannten Definitionen gelten.

In einer weiteren Ausführungsform weisen die erfindungsgemäßen organischen Moleküle eine Struktur der Formel VII auf:

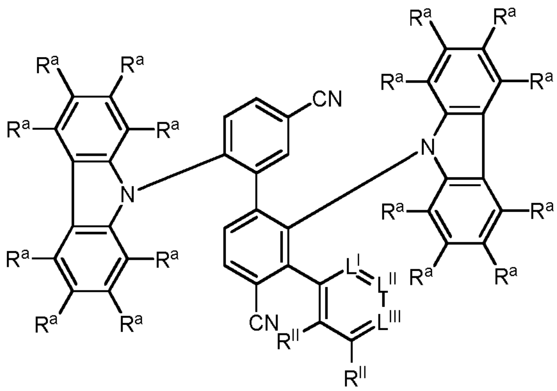

Formel VII

wobei die oben genannten Definitionen gelten.

In einer weiteren Ausführungsform weisen die erfindungsgemäßen organischen Moleküle eine Struktur der Formel VIIa, Formel VIIb oder Formel VIIc auf:

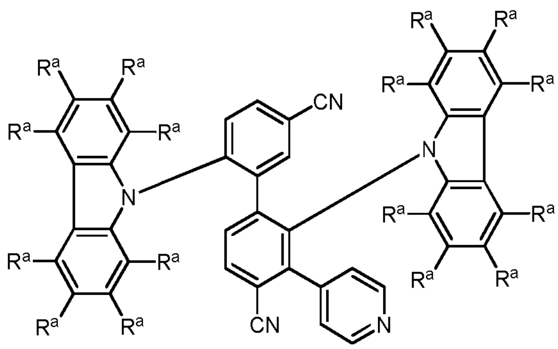

Formel VIIa

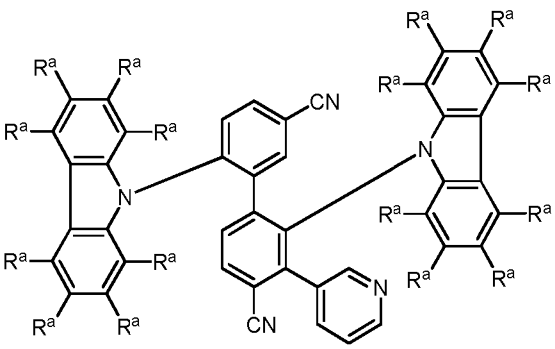

Formel VIIb

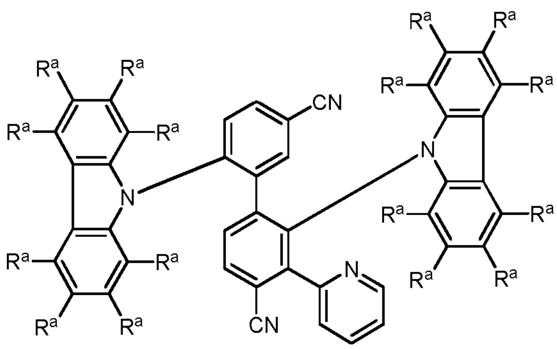

Formel VIIc

wobei die oben genannten Definitionen gelten.

In einer weiteren Ausführungsform weisen die erfindungsgemäßen organischen Moleküle eine Struktur der Formel VIIa-1, Formel VIIb-1 oder Formel VIIc-1 auf:

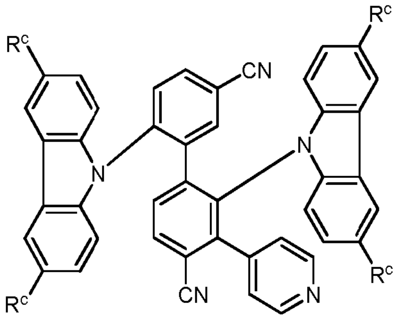

Formel VIIa-1

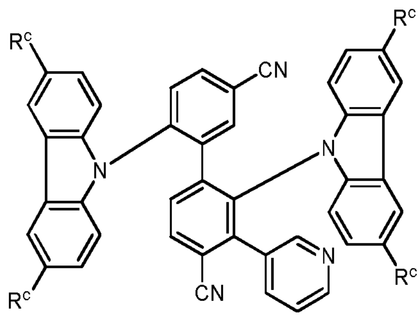

Formel VIIb-1

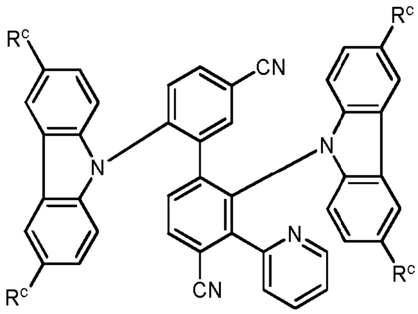

Formel VIIc-1

wobei die oben genannten Definitionen gelten.

In einer weiteren Ausführungsform weisen die erfindungsgemäßen organischen Moleküle eine Struktur der Formel VIIa-2, Formel VIIb-2 oder Formel VIIc-2 auf:

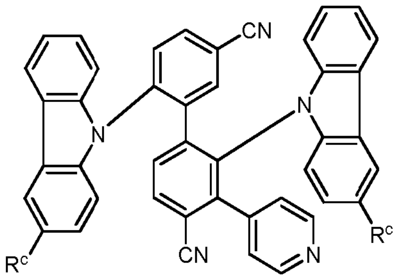

Formel VIIa-2

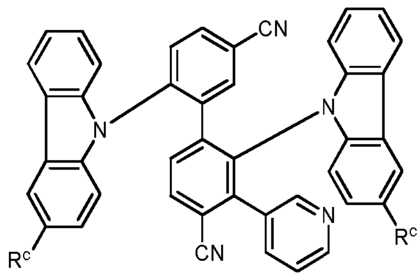

Formel VIIb-2

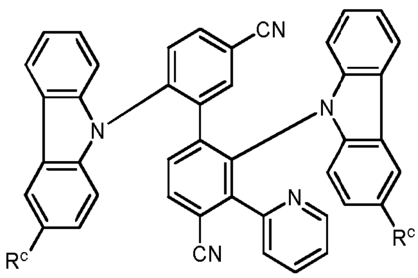

Formel VIIc-2

wobei die oben genannten Definitionen gelten.

In einer weiteren Ausführungsform weisen die erfindungsgemäßen organischen Moleküle eine Struktur der Formel VIII auf:

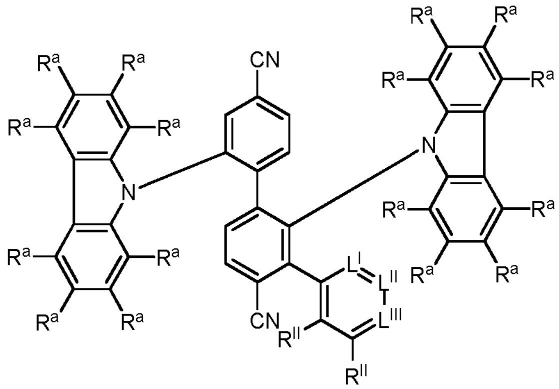

Formel VIII

wobei die oben genannten Definitionen gelten.

In einer weiteren Ausführungsform weisen die erfindungsgemäßen organischen Moleküle eine Struktur der Formel VIIIa, Formel VIIIb oder Formel VIIIc auf:

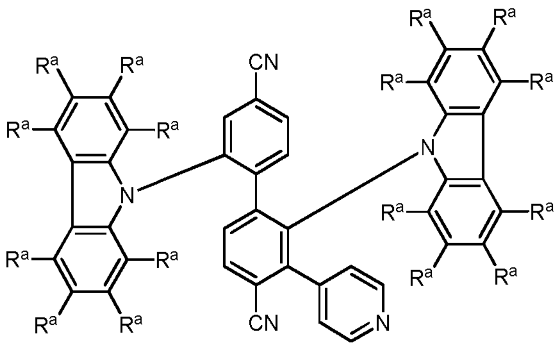

Formel VIIIa

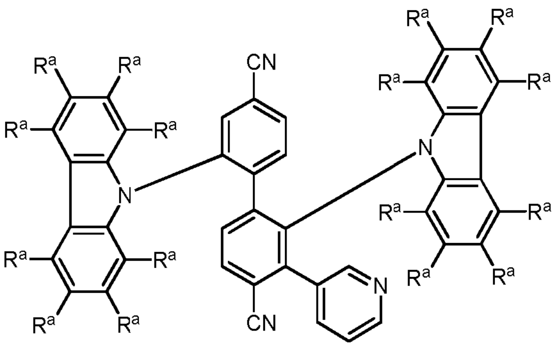

Formel VIIIb

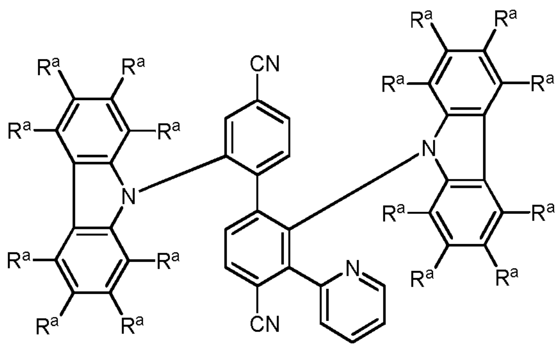

Formel VIIIc

wobei die oben genannten Definitionen gelten.

In einer weiteren Ausführungsform weisen die erfindungsgemäßen organischen Moleküle eine Struktur der Formel VIIIa-1, Formel VIIIb-1 oder Formel VIIIc-1 auf:

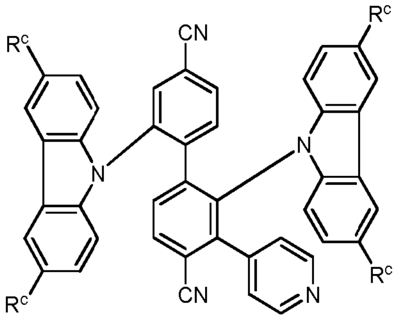

Formel VIIIa-1

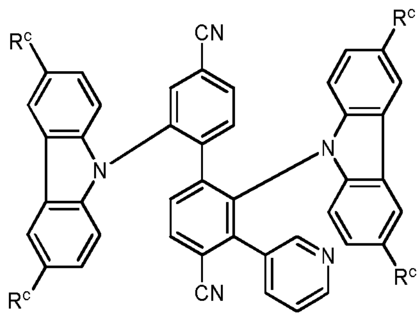

Formel VIIIb-1

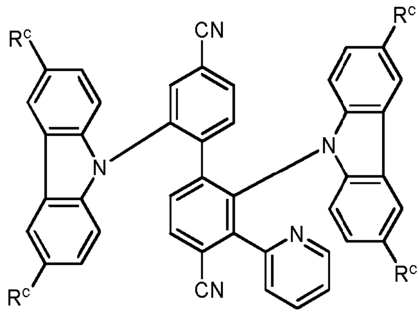

Formel VIIIc-1

wobei die oben genannten Definitionen gelten.

In einer weiteren Ausführungsform weisen die erfindungsgemäßen organischen Moleküle eine Struktur der Formel VIIIa-2, Formel VIIIb-2 oder Formel VIIIc-2 auf:

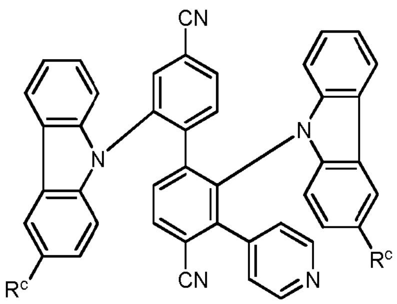

Formel VIIIa-2

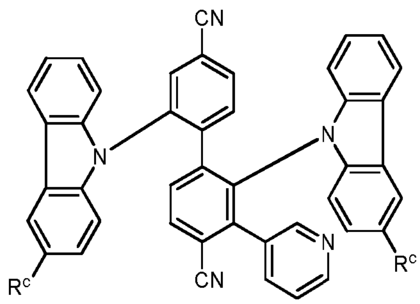

Formel VIIIb-2

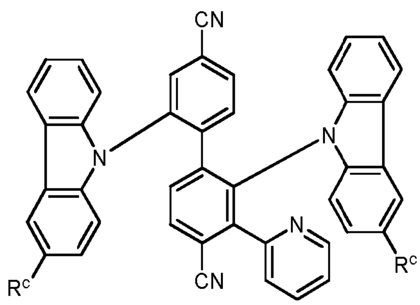

Formel VIIIc-2

wobei die oben genannten Definitionen gelten.

In einer weiteren Ausführungsform weisen die erfindungsgemäßen organischen Moleküle eine Struktur der

Formel IX auf:

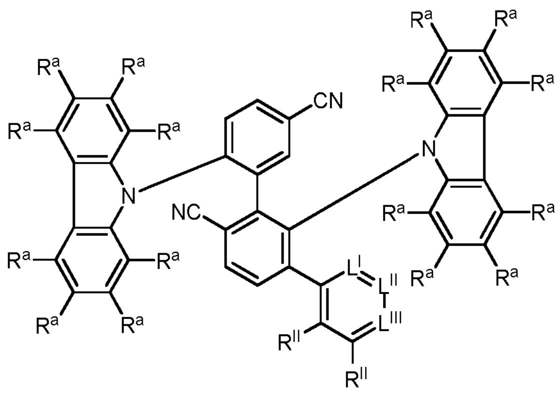

Formel IX

wobei die oben genannten Definitionen gelten.

In einer weiteren Ausführungsform weisen die erfindungsgemäßen organischen Moleküle eine Struktur der Formel IXa, Formel IXb oder Formel IXc auf:

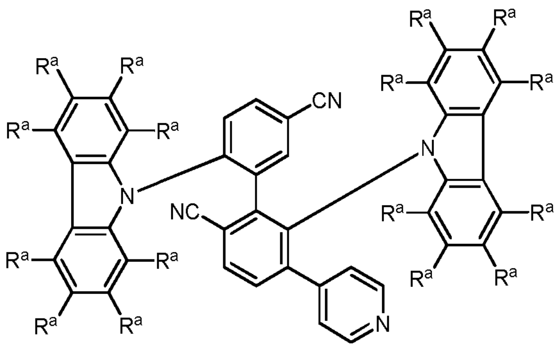

Formel IXa

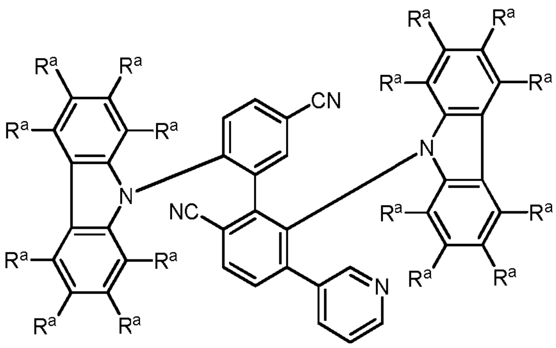

Formel IXb

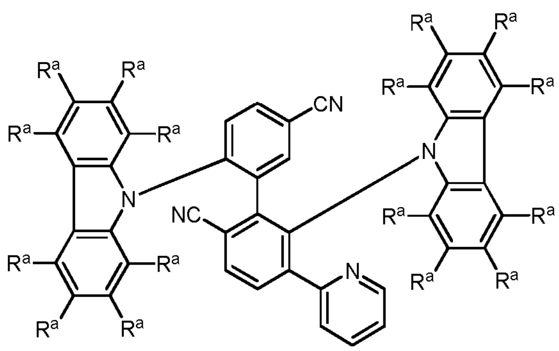

Formel IXc

wobei die oben genannten Definitionen gelten.

In einer weiteren Ausführungsform weisen die erfindungsgemäßen organischen Moleküle eine Struktur der Formel IXa-1, Formel IXb-1 oder Formel IXc-1 auf:

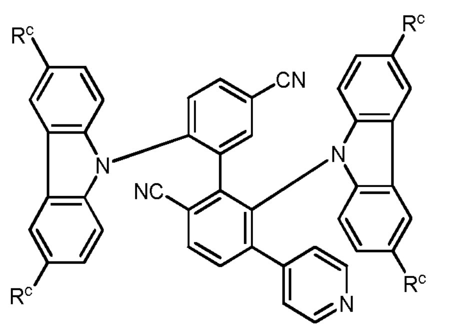

Formel IXa-1

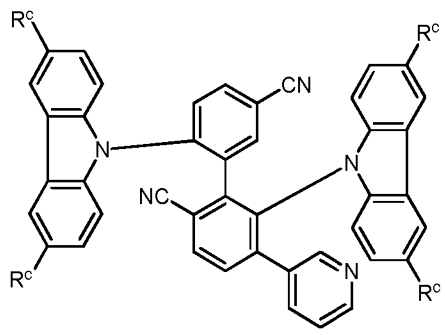

Formel IXb-1

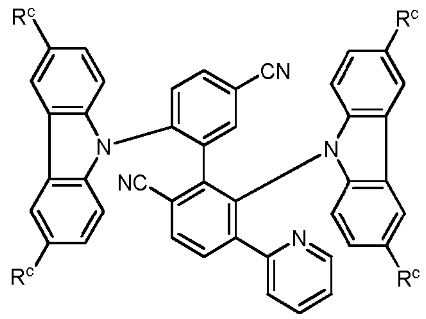

Formel IXc-1

wobei die oben genannten Definitionen gelten.

In einer weiteren Ausführungsform weisen die erfindungsgemäßen organischen Moleküle eine Struktur der Formel IXa-2, Formel IXb-2 oder Formel IXc-2 auf:

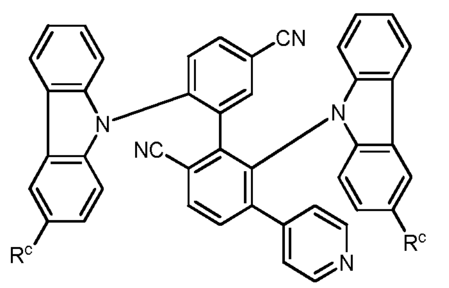

Formel IXa-2

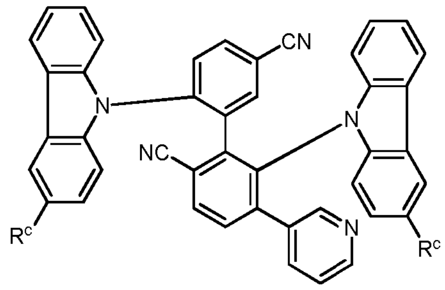

Formel IXb-2

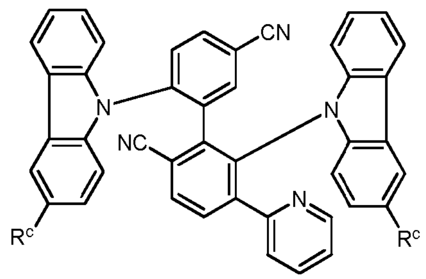

Formel IXc-2

wobei die oben genannten Definitionen gelten.

In einer weiteren Ausführungsform weisen die erfindungsgemäßen organischen Moleküle eine Struktur der Formel X auf:

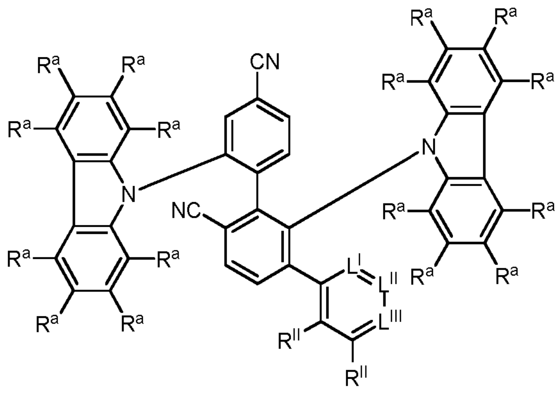

Formel X

wobei die oben genannten Definitionen gelten.

In einer weiteren Ausführungsform weisen die erfindungsgemäßen organischen Moleküle eine Struktur der Formel Xa, Formel Xb oder Formel Xc auf:

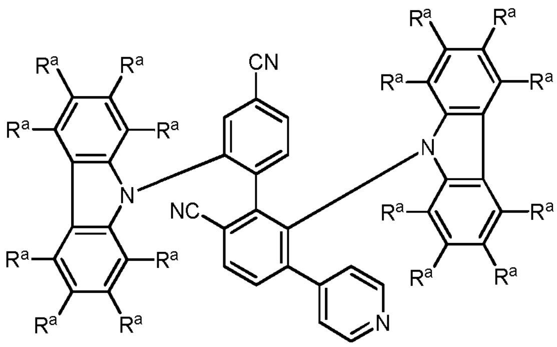

Formel Xa

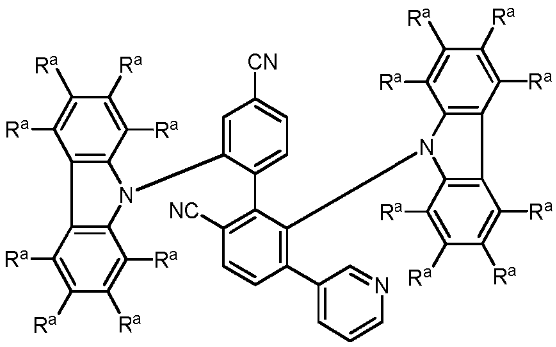

Formel Xb

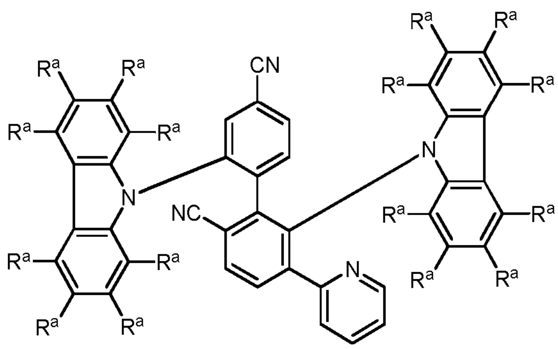

Formel Xc

wobei die oben genannten Definitionen gelten.

In einer weiteren Ausführungsform weisen die erfindungsgemäßen organischen Moleküle eine Struktur der Formel Xa-1, Formel Xb-1 oder Formel Xc-1 auf:

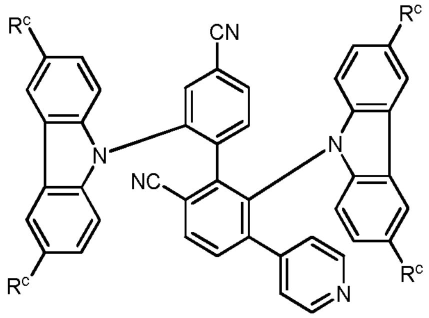

Formel Xa-1

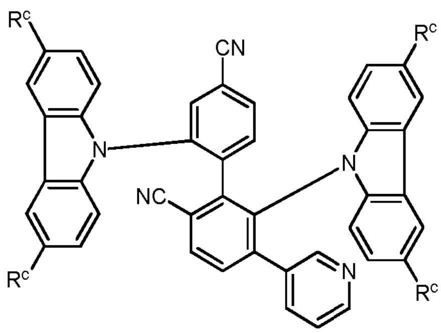

Formel Xb-1

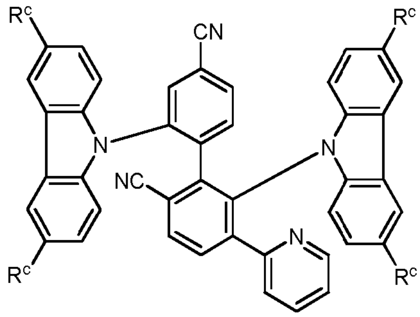

Formel Xc-1

wobei die oben genannten Definitionen gelten.

In einer weiteren Ausführungsform weisen die erfindungsgemäßen organischen Moleküle eine Struktur der Formel Xa-2, Formel Xb-2 oder Formel Xc-2 auf:

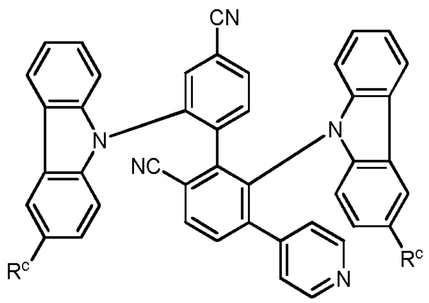

Formel Xa-2

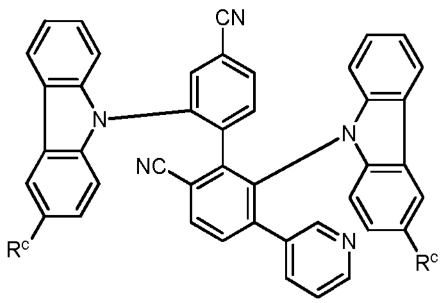

Formel Xb-2

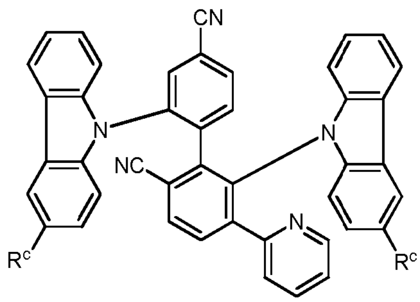

Formel Xc-2

wobei die oben genannten Definitionen gelten.

In einer Ausführungsform der organischen Moleküle ist $R^c$ ist bei jedem Auftreten unabhängig voneinander ausgewählt aus der Gruppe bestehend aus CN, $CF_3$, Me, $^iPr$, $^tBu$, Ph, das jeweils durch einen oder mehrere Reste ausgewählt aus CN, $CF_3$, Me, $^iPr$, $^tBu$, CN, $CF_3$ oder Ph substituiert sein kann, und Carbazolyl, das jeweils durch einen oder mehrere Reste ausgewählt aus CN, $CF_3$, Me, $^iPr$, $^tBu$, oder Ph substituiert sein kann.

Im Sinne dieser Erfindung enthält eine Arylgruppe 6 bis 60 aromatische Ringatome; eine Heteroarylgruppe enthält 5 bis 60 aromatische Ringatome, von denen mindestens eines ein Heteroatom darstellt. Die Heteroatome sind insbesondere N, O und/oder S. Werden in der Beschreibung bestimmter Ausführungsformen der Erfindung andere, von der genannten Definition abweichende Definitionen angegeben, beispielsweise bezüglich der Zahl der aromatischen Ringatome oder der enthaltenen Heteroatome, so gelten diese.

Unter einer Arylgruppe bzw. Heteroarylgruppe wird ein einfacher aromatischer Cyclus, also Benzol, oder ein einfacher heteroaromatischer Cyclus, beispielsweise Pyridin, Pyrimidin oder Thiophen, oder ein heteroaromatischer Polycyclus, beispielsweise Phenanthren, Chinolin oder Carbazol verstanden. Ein kondensierter (annelierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen; Pyrrol, Indol, Isoindol, Carbazol, Pyridin, Chinolin, Isochinolin, Acridin, Phenanthridin, Benzo-5,6-chinolin, Isochinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Napthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benztriazol, 1,2,3-Oxadiazol, 1,2,4-Oxadiazol, 1,2,5-Oxadiazol, 1,2,3,4-Tetrazin, Purin, Pteridin, Indolizin und Benzothiadiazol oder Kombinationen der genannten Gruppen.

Unter einer cyclischen Alkyl-, Alkoxy- oder Thioalkoxygruppe wird hier eine monocyclische, eine bicyclische oder eine polycyclische Gruppe verstanden.

Im Rahmen der vorliegenden Erfindung werden unter einer $C_1$- bis $C_{40}$-Alkylgruppe, in der auch einzelne H-Atome oder $CH_2$-Gruppen durch die oben genannten Gruppen substituiert sein können, beispielsweise die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, t-Pentyl, 2-Pentyl, neoPentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Adamantyl, Trifluor-methyl, Pentafluorethyl, 2,2,2-Trifluorethyl, 1,1-Dimethyl-n-hex-1-yl-, 1,1-Dimethyl-n-hept-1-yl-, 1,1-Dimethyl-n-oct-1-yl-, 1,1-Dimethyl-n-dec-1-yl-, 1,1-Dimethyl-n-dodec-1-yl-, 1,1-Dimethyl-n-tetradec-1-yl-, 1,1-Dimethyl-n-hexadec-1-yl-, 1,1-Dimethyl-n-octadec-1-yl-, 1,1-Diethyl-n-hex-1-yl-, 1,1 -Diethyl-n-hept-1-yl-, 1,1-Diethyl-n-oct-1-yl-, 1,1-Diethyl-n-dec-1-yl-, 1,1-Diethyl-n-dodec-1-yl-, 1,1-Diethyl-n-tetradec-1-yl-, 1,1-Diethyln-n-hexadec-1-yl-, 1,1-Diethyl-n-octadec-1-yl-, 1-(n-Propyl)-cyclohex-1-yl-, 1-(n-Butyl)-cyclohex-1-yl-, 1-(n-Hexyl)-cyclohex-1-yl-, 1-(n-0ctyl)-cyclohex-1-yl- und 1-(n-Decyl)-cyclohex-1-yl- verstanden. Unter einer Alkenylgruppe werden beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl oder Cyclooctadienyl verstanden. Unter einer Alkinylgruppe werden beispielsweise Ethinyl, Propinyl, Butinyl, Pentinyl, Hexinyl, Heptinyl oder Octinyl verstanden. Unter einer $C_1$- bis $C_{40}$-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden.

Eine Ausführungsform der Erfindung betrifft organische Moleküle, welche einen $\Delta E(S_1-T_1)$-Wert zwischen dem untersten angeregten Singulett ($S_1$)- und dem darunter liegenden Triplett ($T_1$)-Zustand von nicht höher als 5000 $cm^{-1}$, insbesondere nicht höher als 3000 $cm^{-1}$, oder nicht höher als 1500 $cm^{-1}$ oder 1000 $cm^{-1}$ aufweisen und/oder eine Emissionslebensdauer von höchstens 150 $\mu$s, insbesondere von höchstens 100 $\mu$s, von höchsten 50 $\mu$s, oder von höchstens 10 $\mu$s aufweisen und/oder eine Hauptemissionsbande mit einer Halbwertsbreite kleiner als 0,50 eV, insbesondere kleiner als 0,58 eV, kleiner als 0,45 eV, oder kleiner als 0,42 eV aufweisen.

Die erfindungsgemäßen organischen Moleküle zeigen insbesondere ein Emissionsmaximum bei zwischen 420 und 500 nm, zwischen 430 und 480 nm, oder zwischen 450 und 470 nm.

In einer Ausführungsform weisen die Moleküle einen "blue material index" (BMI), den Quotienten aus der PLQY (in %) und ihrer $CIE_y$-Farbkoordinate des von dem erfindungsgemäßen Molekül emittierten Lichts, von größer 150, insbesondere von größer 200, von größer 250 oder von größer 300 auf.

In einem weiteren Aspekt betrifft die Erfindung ein Verfahren zur Herstellung eines erfindungsgemäßen organischen Moleküls der hier beschriebenen Art (optional mit einer Folgeumsetzung), wobei ein Dichlor-Fluorbenzonitril, das mit zwei Resten $R^2$ substituiert ist, als Edukt eingesetzt wird:

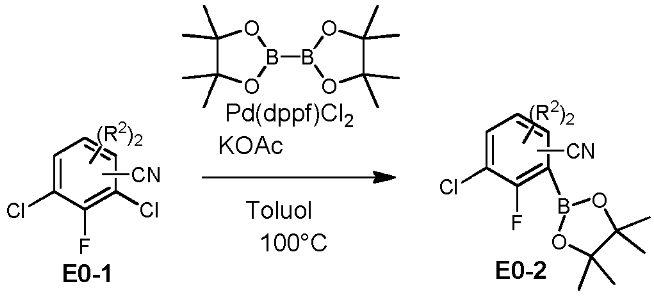


E0-2

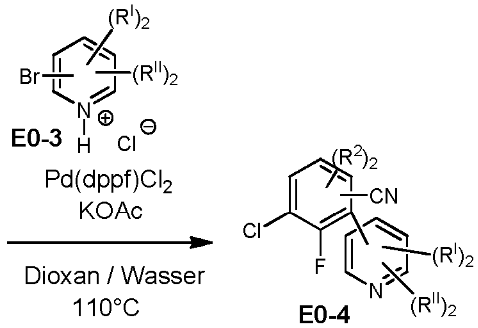


E0-4

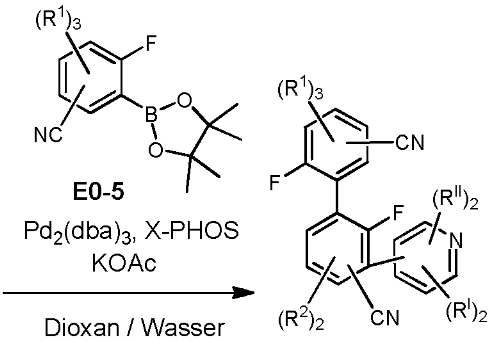


Z

Z

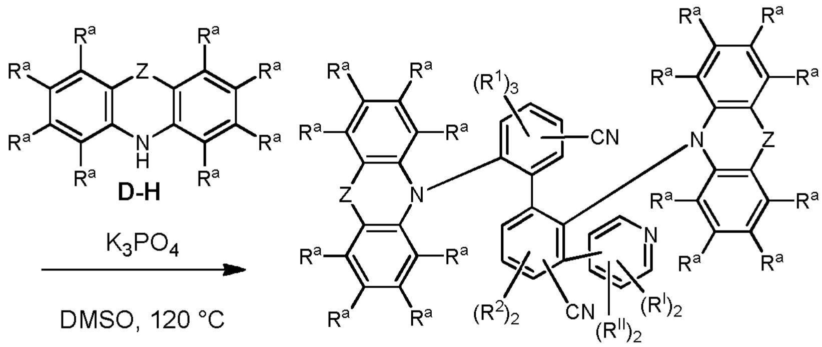


Erfindungsgemäße Dichlor-Fluorbenzonitrile **E0-1** sind beispielhaft 3,5-Dichlor-4-Fluorbenzonitril und 2,4-Dichlor-3-Fluorbenzonitril.

Erfindungsgemäße Brompyridin Hydrochloride **E0-3** sind beispielhaft 4-Brompyridin Hydrochlorid, 3-Brompyridin Hydrochlorid und 2-Brompyridin Hydrochlorid. Alternativ können anstelle von Brompyridin Hydrochloriden auch die Brompyridine 4-Brompyridin, 3-Brompyridin und 2-Brompyridin verwendet werden.

Erfindungsgemäße Fluorcyanophenylboronsäurepinakolester **E0-5** sind beispielhaft 2-Fluor-3-Cyanophenylboronsäurepinakolester, 2-Fluor-4-Cyanophenylboronsäurepinakolester, 2-Fluor-5-Cyanophenylboronsäurepinakolester und 2-Fluor-6-Cyanophenylboronsäurepinakolester.

Das Produkt wird durch Deprotonierung des entsprechenden Amins und anschließender nukleophiler Substitution der zwei Fluorgruppen erhalten. Hierbei werden zwei Stickstoffheterozyklen **D-H** im Sinne einer nukleophilen aromatischen Substitution mit einem Edukt **Z** umgesetzt. Typische Bedingungen beinhalten die Verwendung einer Base wie beispielweise tribasisches Kaliumphosphat oder Natriumhydrid in einem aprotischen polaren Lösungsmittel wie beispielweise Dimetylsulfoxid (DMSO) oder N,N-Dimethylformamid (DMF).

Ein alternativer Syntheseweg zur Herstellung eines erfindungsgemäßen organischen Moleküls der hier beschriebenen Art (optional mit einer Folgeumsetzung), wobei ein Dichlor-Fluorbenzonitril, das mit zwei Resten $R^2$ substituiert ist, als Edukt eingesetzt wird, beinhaltet folgende Synthese von **E0-1** zu **E0-4:**

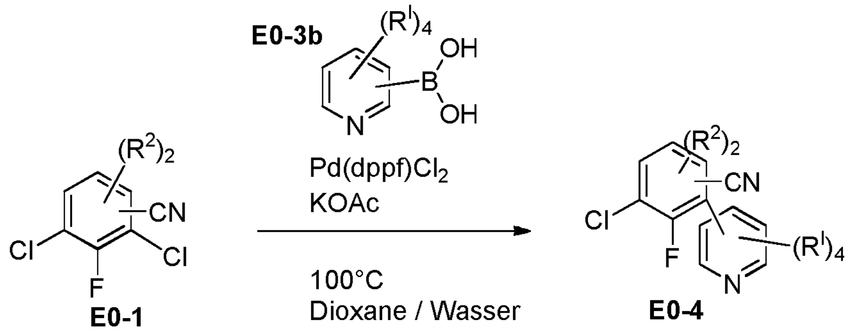


Erfindungsgemäße Pyridinboronsäuren **E0-3b** sind beispielhaft 4-Pyridinboronsäure, 3-Pyridinboronsäure und 2-Pyridinboronsäure. Alternativ können anstelle von Pyridinborsäuren auch die entsprechenden Pyridinboronsäureester verwendet werden, zum Beispiel 4-Pyridinboronsäure Pinakolester, 3-Pyridinboronsäure Pinakolester und 2-Pyridinboronsäure Pinakolester.

In einem weiteren Aspekt betrifft die Erfindung die Verwendung der organischen Moleküle als lumineszierender Emitter oder als Hostmaterial in einer optoelektronischen Vorrichtung, insbesondere wobei die optoelektronische Vorrichtung ausgewählt ist aus der Gruppe bestehend aus:

- organischen lichtemittierenden Dioden (OLEDs),
- lichtemittierenden elektrochemischen Zellen,
- OLED-Sensoren, insbesondere in nicht hermetisch nach außen abgeschirmten Gas- und Dampf-Sensoren,
- organischen Dioden,
- organischen Solarzellen,
- organischen Transistoren,
- organischen Feldeffekttransistoren,
- organischen Lasern und
- Down-Konversions-Elementen.

In einem weiteren Aspekt betrifft die Erfindung eine Zusammensetzung aufweisend oder bestehend aus:

(a) mindestens einem erfindungsgemäßen organischen Molekül, insbesondere als Emitter und/oder Host, und
(b) mindestens einem, d. h. einem oder mehreren (wie 2, 3, 4, ...) Emitter- und/oder Hostmaterialien, die von dem erfindungsgemäßen organischen Molekül verschiedenen ist bzw. sind und
(c) optional einem oder mehreren Farbstoffen und/ oder einem oder mehreren organischen Lösungsmitteln.

In einer Ausführungsform besteht die erfindungsgemäße Zusammensetzung aus einem erfindungsgemäßen organischen Molekül und einem oder mehreren Hostmaterialien. Das oder die Hostmaterialen weisen insbesondere Triplett ($T_1$)- und Singulett ($S_1$)- Energieniveaus auf, die energetisch höher liegen als die Triplett ($T_1$)- und Singulett ($S_1$)-Energieniveaus des erfindungsgemäßen organischen Moleküls. In einer Ausführungsform weist die Zusammensetzung neben dem erfindungsgemäßen organischen Molekül ein elektronendominantes und ein lochdominantes Hostmaterial auf. Das höchste besetzte Orbital (HOMO) und das niedrigste unbesetzte Orbital (LUMO) des lochdominanten Hostmaterials liegen energetisch insbesondere höher als das des elektronendominanten Hostmaterials. Das HOMO des lochdominanten Hostmaterials liegt energetisch unter dem HOMO des erfindungsgemäßen organischen Moleküls, während das LUMO des elektronendominanten Hostmaterials energetisch über dem LUMO des erfindungsgemäßen organischen Moleküls liegt. Um Exciplex-Formation zwischen Emitter und Hostmaterial oder Hostmaterialien zu vermeiden, sollten die Materialien so gewählt sein, dass die Energieabstände zwischen den jeweiligen Orbitalen gering sind. Der Abstand zwischen dem LUMO des elektronendominanten Hostmaterials und dem LUMO des erfindungsgemäßen organischen Moleküls beträgt insbesondere weniger als 0,5 eV, bevorzugt weniger als 0,3 eV, noch bevorzugter weniger als 0,2 eV. Der Abstand zwischen dem HOMO des lochdominanten Hostmaterials und dem HOMO des erfindungsgemäßen organischen Moleküls beträgt insbesondere weniger als 0,5 eV, bevorzugt weniger als 0,3 eV, noch bevorzugter weniger als 0,2 eV.

In einem weiteren Aspekt betrifft die Erfindung eine optoelektronische Vorrichtung, die ein erfindungsgemäßes

organisches Molekül oder eine erfindungsgemäße Zusammensetzung aufweist. Die optoelektronische Vorrichtung ist insbesondere ausgeformt als eine Vorrichtung ausgewählt aus der Gruppe bestehend aus organischer lichtemittierender Diode (OLED); lichtemittierender elektrochemischer Zelle; OLED-Sensor, insbesondere nicht hermetisch nach außen abgeschirmten Gas- und Dampf-Sensoren; organischer Diode; organischer Solarzelle; organischem Transistor; organischem Feldeffekttransistor; organischem Laser und Down-Konversion-Element.

Eine optoelektronische Vorrichtung weist in einer Ausführungsform auf:

- ein Substrat,
- eine Anode und
- eine Kathode, wobei die Anode oder die Kathode auf das Substrat aufgebracht sind, und
- mindestens eine lichtemittierende Schicht, die zwischen Anode und Kathode angeordnet ist und die ein erfindungsgemäßes organisches Molekül aufweist, stellt eine weitere Ausführungsform der Erfindung dar.

In einer Ausführungsform handelt es sich bei der optoelektronischen Vorrichtung um eine organische lichtemittierende Dioden (OLED). Eine typische OLED weist beispielsweise folgenden Schichtaufbau auf:

1. Substrat (Trägermaterial)
2. Anode
3. Lochinjektionsschicht (hole injection layer, HIL)
4. Lochtransportschicht (hole transport layer, HTL)
5. Elektronenblockierschicht (electron blocking layer, EBL)
6. Emitterschicht (emitting layer, EML)
7. Lochblockierschicht (hole blocking layer, HBL)
8. Elektronenleitschicht (electron transport layer, ETL)
9. Elektroneninjektionsschicht (electron injection layer, EIL)
10. Kathode.

Dabei sind einzelne Schichten lediglich in optionaler Weise vorhanden. Weiterhin können mehrere dieser Schichten zusammenfallen. Und es können einzelne Schichten mehrfach im Bauteil vorhanden sein.

Gemäß einer Ausführungsform ist mindestens eine Elektrode des organischen Bauelements transluzent ausgebildet. Hier wird mit "transluzent" eine Schicht bezeichnet, die durchlässig für sichtbares Licht ist. Dabei kann die transluzente Schicht klar durchscheinend, also transparent, oder zumindest teilweise Licht absorbierend und/oder teilweise Licht streuend sein, so dass die transluzente Schicht beispielsweise auch diffus oder milchig durchscheinend sein kann. Insbesondere ist eine hier als transluzent bezeichnete Schicht möglichst transparent ausgebildet, so dass insbesondere die Absorption von Licht so gering wie möglich ist.

Gemäß einer weiteren Ausführungsform weist das organische Bauelement, insbesondere eine OLED, einen invertierten Aufbau auf. Der invertierte Aufbau zeichnet sich dadurch aus, dass sich die Kathode auf dem Substrat befindet und die anderen Schichten entsprechend invertiert aufgebracht werden:

1. Substrat (Trägermaterial)
2. Kathode
3. Elektroneninjektionsschicht (electron injection layer, EIL)
4. Elektronenleitschicht (electron transport layer, ETL)
5. Lochblockierschicht (hole blocking layer, HBL)
6. Emissionsschicht bzw. Emitterschicht (emitting layer, EML)
7. Elektronenblockierschicht (electron blocking layer, EBL)
8. Lochtransportschicht (hole transport layer, HTL)
9. Lochinjektionsschicht (hole injection layer, HIL)
10. Anode

Dabei sind einzelne Schichten lediglich in optionaler Weise vorhanden. Weiterhin können mehrere dieser Schichten zusammenfallen. Und es können auch einzelne Schichten mehrfach im Bauteil vorhanden sein.

In einer Ausführungsform wird bei der invertierten OLED die Anodenschicht des typischen Aufbaus, z. B. eine ITO-Schicht (Indium-Zinn-Oxid), als Kathode geschaltet.

Gemäß einer weiteren Ausführungsform weist das organische Bauelement, insbesondere eine OLED, einen gestapelten Aufbau auf. Hierbei werden die einzelnen OLEDs übereinander und nicht wie üblich nebeneinander angeordnet. Durch einen gestapelten Aufbau kann die Erzeugung von Mischlicht ermöglicht werden. Beispielsweise kann dieser Aufbau bei der Erzeugung von weißem Licht eingesetzt werden, für dessen Erzeugung das gesamte sichtbare

Spektrum typischerweise durch die Kombination des emittierten Lichts von blauen, grünen und roten Emittern abgebildet wird. Weiterhin können bei praktisch gleicher Effizienz und identischer Leuchtdichte signifikant längere Lebensdauern im Vergleich zu üblichen OLEDs erzielt werden. Für den gestapelten Aufbau wird optional eine sogenannte Ladungserzeugungsschicht (charge generation layer, CGL) zwischen zwei OLEDs eingesetzt. Diese besteht aus einer n-dotierten und einem p-dotierten Schicht, wobei die n-dotierte Schicht typischerweise näher an der Anode aufgebracht wird.

In einer Ausführungsform - einer sogenannten Tandem-OLED - treten zwei oder mehr Emissionsschichten zwischen Anode und Kathode auf. In einer Ausführungsform sind drei Emissionsschichten übereinander angeordnet, wobei eine Emissionsschicht rotes Licht emittiert, eine Emissionsschicht grünes Licht emittiert und eine Emissionsschicht blaues Licht emittiert und optional weitere Ladungserzeugungs-, Blockier- oder Transportschichten zwischen den einzelnen Emissionsschichten aufgebracht sind. In einer weiteren Ausführungsform werden die jeweiligen Emissionsschichten direkt angrenzend aufgebracht. In einer weiteren Ausführungsform befindet sich jeweils eine Ladungserzeugungsschicht zwischen den Emissionsschichten. Weiterhin können in einer OLED direkt angrenzende und durch Ladungserzeugungsschichten getrennte Emissionsschichten kombiniert werden.

Über den Elektroden und den organischen Schichten kann weiterhin noch eine Verkapselung angeordnet sein. Die Verkapselung kann beispielsweise in Form eines Glasdeckels oder in Form einer Dünnschichtverkapselung ausgeführt sein.

Als Trägermaterial der optoelektronischen Vorrichtung kann beispielsweise Glas, Quarz, Kunststoff, Metall, ein Siliziumwafer oder jedes andere geeignete feste oder flexible, optional durchsichtige Material dienen. Das Trägermaterial kann beispielsweise ein oder mehrere Materialien in Form einer Schicht, einer Folie, einer Platte oder einem Laminat aufweisen.

Als Anode der optoelektronischen Vorrichtung können beispielsweise transparente leitende Metalloxide wie beispielsweise ITO (Indium-Zinn-Oxid), Zinkoxid, Zinnoxid, Cadmiumoxid, Titanoxid, Indiumoxid oder Aluminiumzinkoxid (AZO), $Zn_2SnO_4$, $CdSnO_3$, $ZnSnO_3$, $MgIn_2O_4$, $GaInO_3$, $Zn_2In_2O_5$ oder $In_4Sn_3O_{12}$ oder Mischungen unterschiedlicher transparenter leitender Oxide dienen.

Als Materialien einer HIL können beispielsweise PEDOT:PSS (Poly-3,4-ethylendioxythiophen:Polystyrolsulfonsäure), PEDOT (Poly-3,4-ethylendioxythiophen), m-MTDATA (4,4',4"-Tris[phenyl(m-tolyl)amino]triphenylamin), Spiro-TAD (2,2',7,7'-Tetrakis(N,N-diphenylamino)-9,9-spirobifluoren), DNTPD (4,4'-Bis[N-[4-{N,N-bis(3-methylphenyl)amino}phenyl]-N-phenylamino]biphenyl), NPB (N,N'-Bis-(1-naphthalenyl)-N,N'-bisphenyl-(1,1'-biphenyl)-4,4'-diamin), NPNPB (N,N'-Diphenyl-N,N'-di-[4-(N,N-diphenylamino)phenyl]benzol), MeO-TPD (N,N,N',N'-Tetrakis(4-methoxyphenyl)benzol), HAT-CN (1,4,5,8,9,11-Hexaazatriphenylen-hexacarbonitril) oder Spiro-NPD (N,N'-diphenyl-N,N'-Bis-(1-naphthyl)-9,9'-spirobifluorene-2,7-diamin) dienen. Beispielhaft ist die Schichtdicke 10-80 nm. Desweiteren können kleine Moleküle verwendet werden (z. B. Kupfer-Phthalocyanin (CuPc z. B. 10 nm dick)) oder Metalloxide wie beispielsweise $MoO_3$, $V_2O_5$.

Als Materialien einer HTL können tertiäre Amine, Carbazolderivate, mit Polystyrolsulfonsäure dotiertes Polyethylendioxythiophen, mit Camphersulfonsäure dotiertes Polyanilin poly-TPD (Poly(4-butylphenyl-diphenyl-amin)), [alpha]-NPD (Poly(4-butylphenyl-diphenyl-amin)), TAPC (4,4'-Cyclohexyliden-bis[N,N-bis(4-methylphenyl)benzenamin]), TCTA (Tris(4-carbazoyl-9-ylphenyl)amin), 2-TNATA (4,4',4"-Tris[2-naphthyl(phenyl)amino]triphenylamin), Spiro-TAD, DNTPD, NPB, NPNPB, MeO-TPD, HAT-CN oder TrisPcz (9,9'-Diphenyl-6-(9-phenyl-9H-carbazol-3-yl)-9H,9'H-3,3'-bicarbazol) dienen. Beispielhaft ist die Schichtdicke 10 nm bis 100 nm.

Die HTL kann eine p-dotierte Schicht aufweisen, die einen anorganischen oder organischen Dotierstoff in einer organischen löcherleitenden Matrix aufweist. Als anorganischer Dotierstoff können beispielsweise Übergangsmetalloxide wie etwa Vanadiumoxid, Molybdänoxid oder Wolframoxid genutzt werden. Als organische Dotierstoffe können beispielsweise Tetrafluorotetracyanoquinodimethan (F4-TCNQ), Kupfer-Pentafluorobenzoat (Cu(I)pFBz) oder Übergangsmetallkomplexe verwendet werden. Beispielhaft ist die Schichtdicke 10 nm bis 100 nm.

Als Materialien einer Elektronenblockierschicht können beispielsweise mCP (1,3-Bis(carbazol-9-yl)benzol), TCTA, 2-TNATA, mCBP (3,3-Di(9H-carbazol-9-yl)biphenyl), tris-Pcz (9,9'-Diphenyl-6-(9-phenyl-9H-carbazol-3-yl)-9H,9'H-3,3'-bicarbazol), CzSi (9-(4-tert-Butylphenyl)-3,6-bis(triphenylsilyl)-9H-carbazol), SimCP ([3,5-Di(9H-carbazol-9-yl)phenyl]-9H-carbazol) oder DCB (N,N'-Dicarbazolyl-1,4-dimethylbenzol) dienen. Beispielhaft ist die Schichtdicke 10 nm bis 50 nm.

Die Emitter-Schicht EML oder Emissionsschicht besteht aus oder enthält Emittermaterial oder eine Mischung aufweisend mindestens zwei Emittermaterialien und optional ein oder mehreren Hostmaterialien. Geeignete Hostmaterialien sind beispielsweise mCP, TCTA, 2-TNATA, mCBP, CBP (4,4'-Bis-(N-carbazolyl)-biphenyl), Sif87 (Dibenzo[b,d]thiophen-2-yltriphenylsilan), Sif88 (Dibenzo[b,d]thiophen-2-yl)diphenylsilan), 9-[3-(Dibenzofuran-2-yl)phenyl]-9H-carbazol, 9-[3-(Dibenzofuran-2-yl)phenyl]-9H-carbazol, 9-[3-(Dibenzothiophen-2-yl)phenyl]-9H-carbazol, 9-[3,5-Bis(2-dibenzofuranyl)phenyl]-9H-carbazol, 9-[3,5-Bis(2-dibenzothiophenyl)phenyl]-9H-carbazol, T2T (2,4,6-Tris(biphenyl-3-yl)-1,3,5-triazin), T3T (2,4,6-Tris(triphenyl-3-yl)-1,3,5-triazin), TST (2,4,6-Tris(9,9'-spirobifluorene-2-yl)-1,3,5-triazin) und/oder DPEPO (Bis[2-((oxo)diphenylphosphino)phenyl]ether). In einer Ausführungsform enthält die EML 50-80 Gewichts-%, bevorzugt 60-75 Gewichts-% eines Hostmaterials ausgewählt aus der Gruppe bestehend aus CBP, mCP,

mCBP, 9-[3-(Dibenzofuran-2-yl)phenyl]-9H-carbazol, 9-[3-(Dibenzofuran-2-yl)phenyl]-9H-carbazol, 9-[3-(Dibenzothiophen-2-yl)phenyl]-9H-carbazol, 9-[3,5-Bis(2-dibenzofuranyl)phenyl]-9H-carbazol und 9-[3,5-bis(2-Dibenzothiophenyl)phenyl]-9H-carbazol; 10-45 Gewichts-%, bevorzugt 15-30 Gewichts-% T2T und 5-40 Gewichts-%, bevorzugt 10-30 Gewichts-% eines erfindungsgemäßen organischen Moleküls als Emitter. Für im Grünen oder im Roten emittierendes Emittermaterial oder einer Mischung aufweisend mindestens zwei Emittermaterialien eignen sich die gängigen Matrixmaterialien wie CBP. Für im Blauen emittierendes Emittermaterial oder einer Mischung aufweisend mindestens zwei Emittermaterialien können UHG-Matrixmaterialien (Ultra-High energy Gap Materialien) (siehe z. B. M.E. Thompson et al., Chem. Mater. 2004, 16, 4743) oder andere sogenannten Wide-Gap-Matrixmaterialien eingesetzt werden. Beispielhaft ist die Schichtdicke 10 nm bis 250 nm.

Die Lochblockierschicht HBL kann beispielsweise BCP (2,9-Dimethyl-4,7-diphenyl-1,10-phenanthrolin = Bathocuproin), Bis-(2-methyl-8-hydroxychinolinato)-(4-phenylphenolato)-aluminium(III) (BAlq), NBphen (2,9-Bis(naphthalen-2-yl)-4,7-diphenyl-1,10-phenanthrolin), Alq3 (Aluminium-tris(8-hydroxychinolin)), T2T, TSPO1 (Diphenyl-4-triphenylsilylphenylphosphinoxid) oder TCB/TCP (1,3,5-Tris(N-carbazolyl)benzol/ 1,3,5-tris(carbazol)-9-yl) benzol) aufweisen. Beispielhaft ist die Schichtdicke 10 nm bis 50 nm.

Die Elektronentransportschicht ETL kann beispielsweise Materialien auf Basis von $AlQ_3$, TSPO1, NBphen, BPyTP2 (2,7-Di(2,2'-bipyridin-5-yl)triphenyl), Sif87, Sif88, BmPyPhB (1,3-Bis[3,5-di(pyridin-3-yl)phenyl]benzol) oder BTB (4,4'-Bis-[2-(4,6-diphenyl-1,3,5-triazinyl)]-1,1'-biphenyl) aufweisen. Beispielhaft ist die Schichtdicke 10 nm bis 200 nm.

Als Materialien einer dünnen Elektroneninjektionsschicht EIL können beispielsweise CsF, LiF, 8-Hydroxyquinolinolatolithium (Liq), $Li_20$, $BaF_2$, MgO oder NaF verwendet werden.

Als Materialien der Kathodenschicht können Metalle oder Legierungen dienen, beispielsweise Al, Al > AlF, Ag, Pt, Au, Mg, Ag:Mg. Typische Schichtdicken betragen 100 nm bis 200 nm. Insbesondere werden ein oder mehrere Metalle verwendet, die stabil an der Luft sind und/oder die selbstpassivierend, beispielsweise durch Ausbildung einer dünnen schützenden Oxidschicht, sind.

Als Materialien zur Verkapselung sind beispielsweise Aluminiumoxid, Vanadiumoxid, Zinkoxid, Zirkoniumoxid, Titanoxid, Hafniumoxid, Lanthanoxid, Tantaloxid geeignet.

In einer Ausführungsform der erfindungsgemäßen optoelektronischen Vorrichtung ist das erfindungsgemäße organische Molekül als Emissionsmaterial in einer lichtemittierenden Schicht EML eingesetzt, wobei es entweder als Reinschicht oder in Kombination mit einem oder mehreren Hostmaterialien eingesetzt ist.

Eine Ausführungsform der Erfindung betrifft optoelektronische Vorrichtungen, welche eine externe Quanteneffizienz (EQE) bei 1000 cd/m$^2$ von größer 5 %, insbesondere von größer 8 %, insbesondere von größer 10 %, oder von größer 13 %, oder von größer 16 % und insbesondere von größer 20 % und/oder ein Emissionsmaximum bei einer Wellenlänge zwischen 420 nm und 500 nm, insbesondere zwischen 430 nm und 490 nm, oder zwischen 440 nm und 480 nm und insbesondere zwischen 450 nm und 470 nm und/oder einen LT80 Wert bei 500 cd/m$^2$ von größer 30 h, insbesondere von größer 70 h, oder von größer 100 h, oder von größer 150 h und insbesondere von größer 200 h aufweisen.

Der Massenanteil des erfindungsgemäßen organischen Moleküls an der Emitter-Schicht EML beträgt in einer weiteren Ausführungsform in einer lichtemittierenden Schicht in optischen Licht emittierenden Vorrichtungen, insbesondere in OLEDs, zwischen 1 % und 80 %. In einer Ausführungsform der erfindungsgemäßen optoelektronischen Vorrichtung ist die lichtemittierende Schicht auf ein Substrat aufgebracht, wobei bevorzugt eine Anode und eine Kathode auf das Substrat aufgebracht sind und die lichtemittierende Schicht zwischen Anode und Kathode aufgebracht ist.

Die lichtemittierende Schicht kann in einer Ausführungsform ausschließlich ein erfindungsgemäßes organisches Molekül in 100 % Konzentration aufweisen, wobei die Anode und die Kathode auf das Substrat aufgebracht sind, und die lichtemittierende Schicht zwischen Anode und Kathode aufgebracht ist.

In einer Ausführungsform der erfindungsgemäßen optoelektronischen Vorrichtung sind eine löcher- und elektroneninjizierende Schicht zwischen Anode und Kathode, und eine löcher- und elektronentransportierende Schicht zwischen löcher- und elektroneninjizierender Schicht, und die lichtemittierende Schicht zwischen löcher- und elektronentransportierender Schicht aufgebracht.

Die optoelektronische Vorrichtung weist in einer weiteren Ausführungsform der Erfindung auf: ein Substrat, eine Anode, eine Kathode und mindestens je eine löcher- und elektroneninjizierende Schicht, und mindestens je eine löcher- und elektronentransportierende Schicht, und mindestens eine lichtemittierende Schicht, die erfindungsgemäßes organisches Molekül und ein oder mehrere Hostmaterialen aufweist, deren Triplett ($T_1$)- und Singulett ($S_1$)-Energieniveaus energetisch höher liegen als die Triplett ($T_1$)- und Singulett ($S_1$)-Energieniveaus des organischen Moleküls, wobei die Anode und die Kathode auf das Substrat aufgebracht ist, und die löcher- und elektroneninjizierende Schicht zwischen Anode und Kathode aufgebracht ist, und die löcher- und elektronentransportierende Schicht zwischen löcher- und elektroneninjizierender Schicht aufgebracht ist, und die lichtemittierende Schicht zwischen löcher- und elektronentransportierender Schicht aufgebracht ist.

Ein weiterer Aspekt der vorliegenden Erfindung betrifft eine OLED, die eine externe Quanteneffizienz bei 1000

$cd/m^2$ von mehr als 8 %, weiter bevorzugt von mehr als 10 %, weiter bevorzugt von mehr als 13 %, noch weiter bevorzugt von mehr als 15 % oder sogar mehr als 20 % aufweist und/oder ein Emissionsmaximum zwischen 420 nm und 500 nm, vorzugsweise zwischen 430 nm und 490 nm, weiter bevorzugt zwischen 440 nm und 480 nm, noch weiter bevorzugt zwischen 450 nm und 470 nm aufweist und/oder einen LT80-Wert bei 500 $cd/m^2$ von mehr als 100 h, vorzugsweise mehr als 200 h, weiter bevorzugt mehr als 400 h, noch weiter bevorzugt mehr als 750 h oder sogar mehr als 1000 h aufweist. Dementsprechend betrifft ein weiterer Aspekt der vorliegenden Erfindung eine OLED, deren Emission eine CIEy-Farbkoordinate von weniger als 0,45, vorzugsweise weniger als 0,30, weiter bevorzugt weniger als 0,20 oder noch weiter bevorzugt weniger als 0,15 oder sogar weniger als 0,10 aufweist.

Ein weiterer Aspekt der vorliegenden Erfindung betrifft eine OLED, die Licht bei einem unterschiedlichen Farbpunkt emittiert. Gemäß der vorliegenden Erfindung emittiert die OLED Licht mit einem schmalen Emissionsbereich (kleine Halbwertsbreite (FWHM)). In einem Aspekt emittiert die OLED gemäß der Erfindung Licht mit einer FWHM der Hauptemissionsspitze von weniger als 0,50 eV, vorzugsweise weniger als 0,48 eV, weiter bevorzugt weniger als 0,45 eV, noch weiter bevorzugt weniger als 0,43 eV oder weniger als 0,40 eV.

Ein weiterer Aspekt der vorliegenden Erfindung betrifft eine OLED, die Licht mit CIEx- und CIEy-Farbkoordinaten nahe den CIEx- (= 0,131) und CIEy- (= 0,046) -Farbkoordinaten der primären Farbe blau (CIEx = 0,131 und CIEy = 0,046) emittiert, wie durch ITU-R Recommendation BT.2020 (Rec. 2020) definiert, und ist somit zur Verwendung bei Ultra-High-Definition(UHD)-Displays, z. B. UHD-TVs, geeignet. In herkömmlichen Anwendungen werden typischerweise nach oben emittierende (obere Elektrode ist transparent) Vorrichtungen verwendet, wobei Testvorrichtung, wie sie in der gesamten vorliegenden Anmeldung verwendet werden, nach unten emittierende Vorrichtungen darstellen (untere Elektrode und Substrat sind transparent). Die CIEy-Farbkoordinate einer blauen Vorrichtung kann durch bis zu einem Faktor von zwei reduziert werden, wenn der Wechsel von einer nach unten zu einer nach oben emittierenden Vorrichtung erfolgt, während die CIEx nahezu unverändert bleibt (Okinaka et al. (2015), 22.1: Invited Paper: New Fluorescent Blue Host Materials for Achieving Low Voltage in OLEDs, SID Symposium Digest of Technical Papers, 46; doi:10.1002/sdtp.10480). Dementsprechend betrifft ein weiterer Aspekt der vorliegenden Erfindung eine OLED, deren Emission eine CIEx-Farbkoordinate von zwischen 0,02 und 0,30, vorzugsweise zwischen 0,03 und 0,25, weiter bevorzugt zwischen 0,05 und 0,20 oder weiter bevorzugt zwischen 0,08 und 0,18 oder sogar zwischen 0,10 und 0,15 und/oder eine CIEy-Farbkoordinate von zwischen 0,00 und 0,45, vorzugsweise zwischen 0,01 und 0,30, weiter bevorzugt zwischen 0,02 und 0,20 oder noch weiter bevorzugt zwischen 0,03 und 0,15 oder sogar zwischen 0,04 und 0,10 aufweist.

In einem weiteren Aspekt betrifft die Erfindung ein Verfahren zur Herstellung eines optoelektronischen Bauelements. Dabei wird ein erfindungsgemäßes organisches Molekül verwendet.

In einer Ausführungsform umfasst das Herstellungsverfahren die Verarbeitung des erfindungsgemäßen organischen Moleküls mittels eines Vakuumverdampfungsverfahrens oder aus einer Lösung.

Erfindungsgemäß ist auch ein Verfahren zur Herstellung einer erfindungsgemäßen optoelektronischen Vorrichtung, bei dem mindestens eine Schicht der optoelektronischen Vorrichtung

- mit einem Sublimationsverfahren beschichtet wird,
- mit einem OVPD (Organic Vapor Phase Deposition) Verfahren beschichtet wird,
- mit einer Trägergassublimation beschichtet wird, und/oder
- aus Lösung oder mit einem Druckverfahren hergestellt wird.

Bei der Herstellung der erfindungsgemäßen optoelektronischen Vorrichtung werden bekannte Verfahren eingesetzt. Generell werden die Schichten einzeln auf ein geeignetes Substrat in aufeinanderfolgenden Abscheideverfahrensschritten aufgebracht. Bei der Gasphasenabscheidung können die gebräuchlichen Methoden, wie thermische Verdampfung, chemische Gasphasenabscheidung (CVD), physikalische Gasphasenabscheidung (PVD) angewendet werden. Für active matrix OLED (AMOLED) Displays erfolgt die Abscheidung auf eine AMOLED Backplane als Substrat.

Alternativ können Schichten aus Lösungen oder Dispersionen in geeigneten Lösungsmitteln aufgebracht werden. Beispielhafte geeignete Beschichtungsverfahren sind Rotationsbeschichtung (spin-coating), Tauchbeschichtung (dip-coating) und Strahldruckmethoden. Die einzelnen Schichten können erfindungsgemäß sowohl über dasselbe als auch über jeweils verschiedene Beschichtungsmethoden hergestellt werden.

## Beispiele

Allgemeines Syntheseschema I

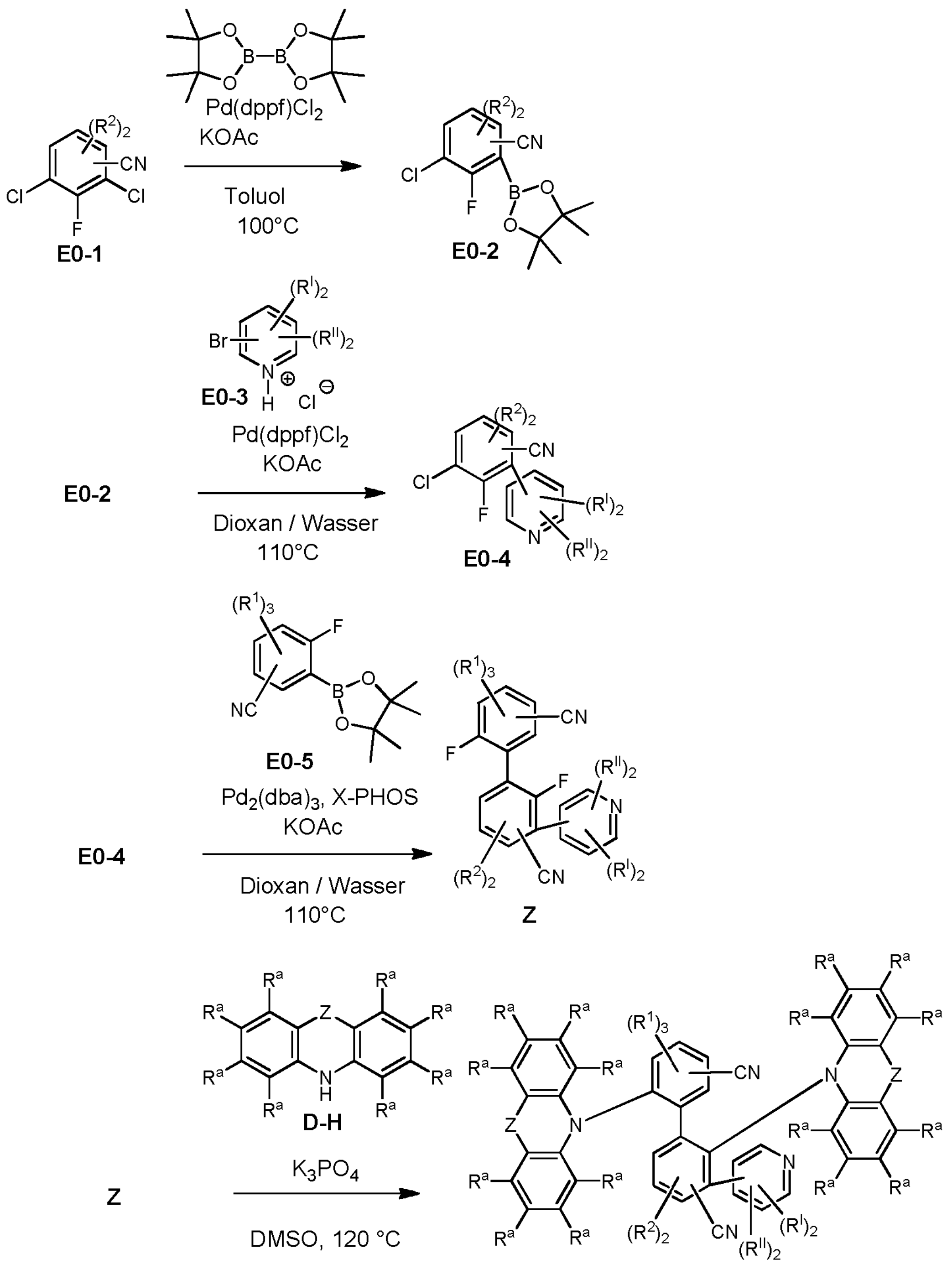


*Allgemeine Synthesevorschrift* ***AAV1:***

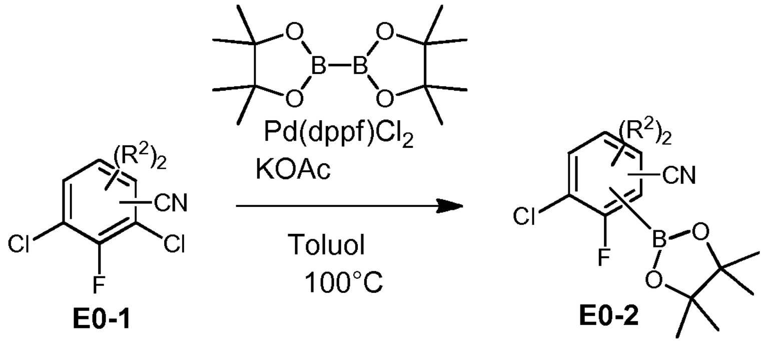


E0-1 E0-2

3,5-Dichlor-4-fluorbenzonitril **E0-1** (1.00 Äquivalent), Bis(pinacolato)diboron (1,00 Äquivalente), $Pd(dppf)Cl_2$ (0,03 Äquivalente) und Kaliumacetat (2,50 Äquivalente) werden in einer Stickstoffschutzgasatmosphäre in Toluol bei 100°C für 22 h gerührt. Aktivkohle und Celite® (ein Kieselguhr) werden zur Reaktionsmischung hinzugegeben und weitere 10 Minuten bei 100°C gerührt. Anschließend wird die Reaktionsmischung heiß filtriert und das Lösungsmittel unter reduziertem Druck abgezogen. Der Rückstand wird in Dichlormethan gelöst, mit gesättigter NaCl-Lösung gewaschen und über Magnesiumsulfat getrocknet. Nach erneuter Konzentrierung der organischen Phase unter reduziertem Druck wird das Rohprodukt über eine Filtersäule aufgereinigt, wobei als Laufmittel eine Cyclohexan/Dichlormethan-Mischung mit steigendem Gradienten (von 25% bis 50%) verwendet wird. Das Produkt **E0-2** wird als weißer Feststoff erhalten.

*Allgemeine Synthesevorschrift **AAV2:***

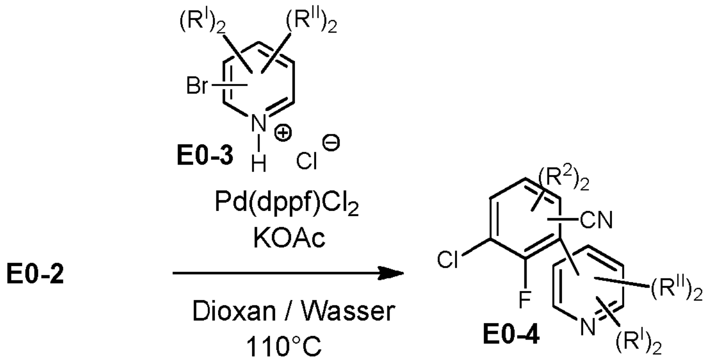


E0-3 E0-2 E0-4

**E0-2** (1,00 Äquivalent), das Brompyridin Hydrochlorid **E0-3** (1,10 Äquivalente), $Pd(dppf)Cl_2$ 0,03 Äquivalente) and Kaliumacetat (4,00 Äquivalente) werden in einer Stickstoffschutzgasatmosphäre in Dioxan/Wasser (10:1 Verhältnis) bei 110°C für 2 h gerührt. Aktivkohle und Celite® werden zur Reaktionsmischung hinzugegeben und weitere 10 Minuten bei 100°C gerührt. Anschließend wird die Reaktionsmischung heiß filtriert und das Lösungsmittel unter reduziertem Druck abgezogen. Der Rückstand wird in Dichlormethan gelöst, mit gesättigter NaCl-Lösung gewaschen und über Magnesiumsulfat getrocknet. Nach erneuter Konzentrierung der organischen Phase unter reduziertem Druck wird das Rohprodukt filtriert und mit kaltem n-Hexan gewaschen. Das Produkt **E0-4** wird als Feststoff gewonnen.

*Allgemeine Synthesevorschrift **AAV2b:***

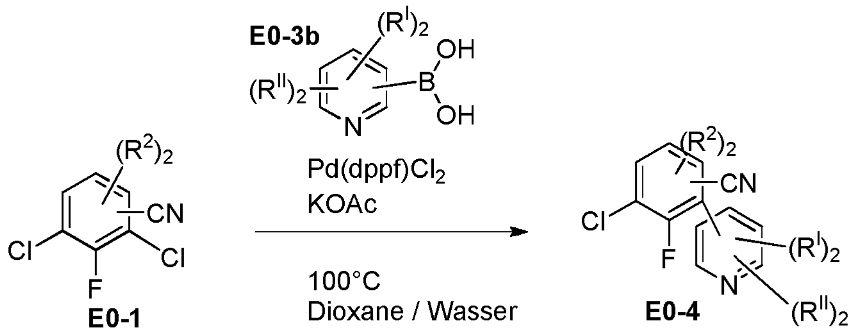


**E0-1** (1,00 Äquivalente), Pyridin-boronsäure (1,00 Äquivalente, **E0-3b),** Pd(dppf)$Cl_2$ (0,05 Äquivalente) und Kaliumacetat (2,50 Äquivalente) werden in einer Stickstoffschutzgasatmosphäre in Dioxan / Wasser (10:1 Verhältnis) bei 100°C für 7 Tage gerührt. Aktivkohle und Celite® werden zur Reaktionsmischung hinzugegeben und weitere 10 Minuten bei 90°C gerührt. Die Reaktionsmischung wird heiß filtriert und das Lösungsmittel unter reduziertem Druck entfernt. Der Rückstand wird in Dichlormethan gelöst, mit einer gesättigten NaCl-Lösung gewaschen, über Magnesiumsulfat getrocknet und das Lösemittel unter reduziertem Druck entfernt. Der Rückstand wird mittels Flashchromatographie aufgereinigt, wobei als Laufmittel eine Cyclohexan/Etylacetat-Mischung mit steigendem Gradienten (0 % - 80 %) verwendet wird. Das Eluat wurde unter reduziertem Druck konzentriert und das Produkt **E0-4** abfiltriert.

*Allgemeine Synthesevorschrift **AAV3:***

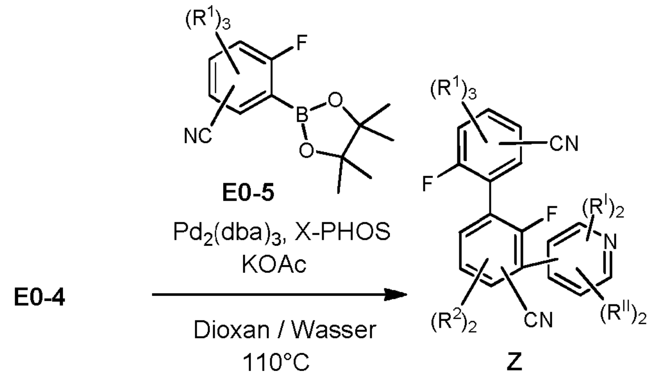


**E0-4** (1,00 Äquivalente), die Cyano-2-fluorphenylboronsäurepinakolester **E0-5** (1,10 Äquivalente), Pd$(dba)_3$ (0,03 Äquivalente), X-PHOS (0,08 Äquivalente) and Kaliumacetat (3,00 Äquivalente) werden in einer Stickstoffschutzgasatmosphäre in Dioxan/Wasser (10:1 Verhältnis) bei 110°C für 20 h gerührt. Aktivkohle und Celite® werden zur Reaktionsmischung hinzugegeben und weitere 10 Minuten bei 100°C gerührt. Die Reaktionsmischung wird heiß filtriert und das Lösungsmittel unter reduziertem Druck entfernt. Der Rückstand wird in Dichlormethan gelöst, mit gesättigter NaCl-Lösung gewaschen und über Magnesiumsulfat getrocknet. Das organische Lösungsmittel wird unter Vakuum reduziert, das Rohprodukt filtriert und kann mit heißem Ethanol gewaschen werden. Das Produkt **Z** wird als Feststoff erhalten.

*Allgemeine Synthesevorschrift **AAV4:***

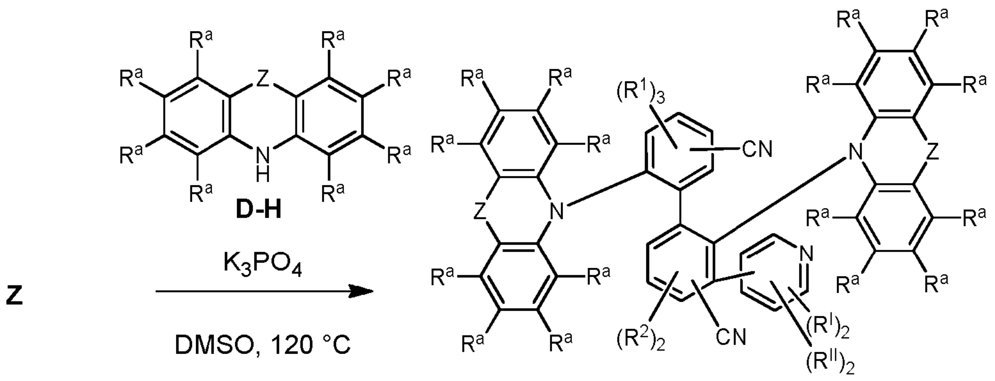


**Z** (jeweils 1,00 Äquivalent), das entsprechende Donor-Molekül **D-H** (2,50 Äquivalente) und tribasisches Kaliumphosphat (5,00 Äquivalente) werden unter Stickstoff in DMSO suspendiert und bei 120 °C gerührt (16 h). Anschließend wird die Reaktionsmischung in gesättigte Natriumchlorid-Lösung gegeben und dreimal mit Dichlormethan extrahiert. Die vereinigten organischen Phasen werden zweimal mit gesättigter Natriumchlorid-Lösung gewaschen, getrocknet über Magnesiumsulfat und das Lösemittel anschließend entfernt. Das Rohprodukt wurde schließlich durch Umkristallisation aus Toluol oder durch Flashchromatographie oder durch waschen in heißen Ethanol gereinigt. Das Produkt wird als Feststoff erhalten.

Im speziellen entspricht **D-H** einem 3,6-substituierten Carbazol (z. B. 3,6-Dimethylcarbazol, 3,6-Diphenylcarbazol, 3,6-Di-tert-butylcarbazol), einem 2,7-substituierten Carbazol (z. B. 2,7-Dimethylcarbazol, 2,7-Diphenylcarbazol, 2,7-Di-tert-butylcarbazol), einem 1,8-substituierten Carbazol (z. B. 1,8-Dimethylcarbazol, 1,8-Diphenylcarbazol, 1,8-Di-tert-butylcarbazol), einem 1-substituierten Carbazol (z. B. 1-Methylcarbazol, 1-Phenylcarbazol, 1-tert-Butylcarbazol), einem 2-substituierten Carbazol (z. B. 2-Methylcarbazol, 2-Phenylcarbazol, 2-tert-Butylcarbazol) oder einem 3-substituierten Carbazol (z. B. 3-Methylcarbazol, 3-Phenylcarbazol, 3-tert-Butylcarbazol). Insbesondere kann ein Halogencarbazol, insbesondere 3-Bromcarbazol oder 3,6-Dibromcarbazol, als **D-H** eingesetzt werden, welches in einer Folgereaktion beispielsweise in eine entsprechende Boronsäure, beispielsweise (Carbazol-3-yl)boronsäure, oder in einen entsprechenden Boronsäureester, beispielsweise (Carbazol-3-yl)boronsäureester, beispielhaft durch Reaktion mit Bis(pinacol)boronsäureester (CAS-Nr. 73183-34-3), umgesetzt wird. In einer Folgereaktion können ein oder mehrere Reste $R^a$, welche als halogeniertes Edukt $R^a$-Hal, bevorzugt $R^a$-Cl und $R^a$-Br, eingesetzt werden, über eine Kupplungsreaktion an Stelle der Boronsäuregruppe oder der Boronsäureestergruppe eingeführt werden. Alternativ können ein oder mehrere Reste $R^a$ durch Reaktion des zuvor eingeführten Halogencarbazols mit Boronsäuren des Restes $R^a$ ($R^a$-$B(OH)_2$) oder entsprechenden Boronsäureestern eingeführt werden.

## Photophysikalische Messungen

*Vorbehandlung von optischen Gläsern*

Alle Gläser (Küvetten und Substrate aus Quarzglas, Durchmesser: 1 cm) wurden nach jeder Benutzung gereinigt: Je dreimaliges Spülen mit Dichlormethan, Aceton, Ethanol, demineralisiertem Wasser, Einlegen in 5 % Hellmanex-Lösung für 24 h, gründliches Ausspülen mit demineralisiertem Wasser. Zum Trocknen wurden die optischen Gläser mit Stickstoff abgeblasen.

*Probenvorbereitung, Film: Spin-Coating*

Gerät: Spin150, SPS euro.

Die Probenkonzentration entsprach 1 mg/ml, angesetzt in Chloroform oder Dichlormethan. Zu 1 ml der entsprechenden Lösung werden 9 mg PMMA hinzugefügt. Mit 50 μl der resultierenden Lösung wird der entsprechende Dünnfilm hergestellt.

Programm: 1) 3 s bei 400 U/min; 2) 20 s bei 1000 U/min bei 1000 Upm/ s. 3) 10 s bei 4000 U/min bei 1000 Upm/s. Die Filme wurden nach dem Beschichten für 1 min bei 70 °C an Luft auf einer Präzisionsheizplatte von LHG getrocknet.

Photolumineszenzspektroskopie und TCSPC

Steady-state Emissionsspektroskopie wurde mit einem Fluoreszenzspektrometer der Horiba Scientific, Modell FluoroMax-4 durchgeführt, ausgestattet mit einer 150 W Xenon-Arc Lampe, Anregungs- und Emissionsmonochromatoren und einer Hamamatsu R928 Photomultiplier-Röhre, sowie einer "zeit-korrelierten Einphotonzähl" *(Time-correlated single-photon counting,* TCSPC)-Option. Hierbei wurde die Probenkammer kontinuierlich mit Stickstoff gespült (Durchflussrate > 800 ml/ min). Emissions- und Anregungsspektren wurden korrigiert durch Standardkorrekturkurven.

Emissionsabklingzeiten wurden ebenfalls auf diesem System gemessen unter Verwendung der TCSPC-Methode mit dem FM-2013 Zubehör und einem TCSPC-Hub von Horiba Yvon Jobin. Anregungsquellen:

NanoLED 370 (Wellenlänge: 371 nm, Pulsdauer: 1,1 ns)
NanoLED 290 (Wellenlänge: 294 nm, Pulsdauer: <1 ns)
SpectraLED 310 (Wellenlänge: 314 nm)
SpectraLED 355 (Wellenlänge: 355 nm).

Die Auswertung (exponentielles Fitten) erfolgte mit dem Softwarepaket DataStation und der DAS 6 Auswertungssoftware. Der Fit wurde über die Chi-Quadrat-Methode angegeben

$$c^2 = \sum_{k=1}^{i} \frac{(e_i - o_i)^2}{e_i}$$

mit $e_i$, den durch den Fit vorhergesagte Größe
und $o_i$, der gemessenen Größe.

Zeitaufgelöste Transienten wurden darüber hinaus an einem FS5 Fluoreszenzspektrometer von Edinburgh Instruments vermessen. Hierbei wird die zu untersuchende Probe über eine breitbandige Xenon-Lampe angeregt (150 W xenon arc lamp). Das FS5 nutzt Czerny-Turner Monochromatoren sowohl für selektive Anregungs- als auch Emissionswellenlängen. Die Photolumineszenz der Probe wird über eine R928P Photomultiplier-Röhre detektiert, wobei die Photokathode des Detektors die zeitaufgelöste Messung im spektralen Bereich von 200 nm bis 870 nm ermöglicht. Die temperaturstabilisierte Detektoreinheit gewährleistet darüber hinaus eine Dunkelzählrate unter 300 Ereignissen pro Sekunde. Zur Bestimmung der Abklingzeit des PL Transienten wird anschließend der μs- Bereich mit Hilfe von drei Exponentialfunktionen angenähert. Über Amplitudengewichtung ergibt sich eine gemittelte Lebensdauer $\tau_{DF}$ für die verzögerte Fluoreszenz von

$$\tau_{\mathrm{DF}} = \sum_{i=1}^{3} \frac{A_i \tau_i}{A_i},$$

mit den jeweiligen monoexponentiellen Abklingzeiten $\tau_i$ und zugehörigen Amplituden $A_i$.

Quanteneffizienzbestimmung

Die Messung der Photolumineszenzquantenausbeute (PLQY) erfolgte mittels eines *Absolute PL Quantum Yield Measurement* C9920-03G-Systems der *Hamamatsu Photonics.* Dieses besteht aus einer 150 W Xenon-Gasentladungslampe, automatisch justierbaren Czerny-Turner Monochromatoren (250 - 950 nm) und einer Ulbricht-Kugel mit hochreflektierender Spektralon-Beschichtung (einem Teflon-Derivat), die über ein Glasfaserkabel mit einem PMA-12 Vielkanaldetektor mit BT- *(back thinned-)* CCD-Chip mit 1024 x 122 Pixeln (Größe 24 x 24 μm) verbunden ist. Die Auswertung der Quanteneffizienz und der CIE-Koordinaten erfolgte mit Hilfe der Software U6039-05 Version 3.6.0.

Das Emissionsmaximum wird in nm, die Quantenausbeute Φ in % und die CIE-Farbkoordinaten als x,y-Werte angegeben.

Die Photolumineszenzquantenausbeute wurde nach folgendem Protokoll bestimmt:

1) Durchführung der Qualitätssicherung: Als Referenzmaterial dient Anthracene in Ethanol mit bekannter Konzentration.
2) Ermitteln der Anregungswellenlänge: Es wurde zuerst das Absorbtionsmaximum des organischen Moleküls bestimmt und mit diesem angeregt.
3) Durchführung der Probenmessung:

Es wurde von entgasten Lösungen und Filmen unter Stickstoff-Atmosphäre die absolute Quantenausbeute bestimmt.

Die Berechnung erfolgte systemintern nach folgender Gleichung:

$$\Phi_{PL} = \frac{n_{photon}, emittiert}{n_{photon}, absorbiert} = \frac{\int \frac{\lambda}{hc}\left[Int_{em}^{Probe}(\lambda) - Int_{em}^{Referenz}(\lambda)\right]d\lambda}{\int \frac{\lambda}{hc}\left[Int_{ex}^{Referenz}(\lambda) - Int_{ex}^{Probe}(\lambda)\right]d\lambda}$$

mit der Photonenzahl $n_{photon}$, der Intensität Int, wobei $Int_{em}^{Probe}$ und $Int_{em}^{Referenz}$ die Photolumineszenzintensitäten mit *(Probe)* und ohne Probe *(Referenz)* sind und $Int_{ex}^{Probe}$ und $Int_{ex}^{Referenz}$ die integrierten Intensitäten des Anregungslichts mit *(Probe)* und ohne Probe *(Referenz)* sind.

Herstellung und Charakterisierung von organischen Elektrolumineszenzvorrichtungen aus der Gasphase

Mit den erfindungsgemäßen organischen Molekülen können OLED-Devices mittels Vakuum-Sublimationstechnik erstellt werden.

Diese noch nicht optimierten OLEDs können standardmäßig charakterisiert werden; hierfür werden die Elektrolumineszenzspektren, die externe Quanteneffizienz (gemessen in %) in Abhängigkeit von der Helligkeit, berechnet aus dem von der Fotodiode detektiertem Licht, den Elektrolumineszenzspektren und dem Strom aufgenommen.

HPLC-MS:
HPLC-MS Spektroskopie wurde mit einer HPLC-Anlage der Firma Agilent (1100er Serie) mit einem angeschlossenen MS-Detektor (Thermo LTQ XL) gemessen.

Beispielsweise wurde für die HPLC eine Eclipse Plus C18 Säule von Agilent mit einer Partikelgröße von 3,5 µm, einer Länge von 150 mm und einem Innendurchmesser von 4,6 mm eingesetzt. Es wurde ohne Vorsäule und bei Raumtemperatur mit den Lösemitteln Acetonitril, Wasser und Tetrahydrofuran in diesen Konzentrationen gearbeitet:

| | | |
|---|---|---|
| Lösemittel A: | $H_2O$ (90%) | MeCN (10%) |
| Lösemittel B: | $H_2O$ (10%) | MeCN (90%) |
| Lösemittel C: | THF (50%) | MeCN (50%) |

Typischerweise wurde mit einem Einspritzvolumen von 15 µL und einer Konzentration von 0.5 mg/mL gemessen.

Die Ionisation der Probe erfolgt durch APCI (atmospheric pressure chemical ionization).

**Beispiel 1**

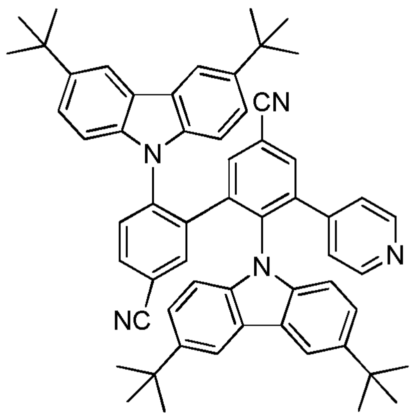

Beispiel **1** wurde nach

***AAV1*** (Ausbeute 72 %), wobei 3,5-Dichlor-4-fluorbenzonitril als **E0-1** verwendet wurde,
**AAV2** (Ausbeute 78 %), wobei 4-Brompyridin Hydrochlorid als **E0-3** verwendet wurde,

**AAV3** (Ausbeute 57 %), wobei 2-Fluor-5-Cyanophenylboronsäurepinakolester als **E0-5** verwendet wurde und ***AAV4*** (Ausbeute 39 %).

MS (HPLC-MS):

| Molekülformel | m/z berechnet | m/z bestimmt |
|---|---|---|
| $C_{60}H_{37}N_5$ | 836,14 | 835,47 |

Figur 1 zeigt das Emissionsspektrum von Beispiel 1 (10 % in PMMA). Das Emissionsmaximum liegt bei 454 nm. Die Photolumineszenzquantenausbeute (PLQY) beträgt 60 % und die Halbwertsbreite beträgt 0,40 eV. Die Emissionsabklingzeit beträgt 31 µs.

**Beispiel 2**

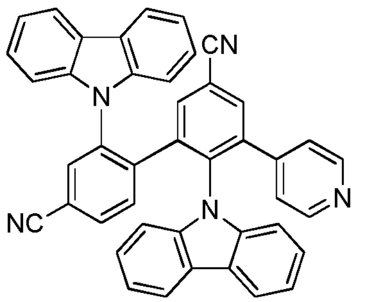

Beispiel **2** wurde nach

***AAV2b*** (Ausbeute 33 %), wobei 3,5-Dichlor-4-fluorbenzonitril als **E0-1** verwendet wurde und 4-Pyridyl-boronsäure als **E0-3b** verwendet wurde;
**AAV3** (Ausbeute 75 %), wobei 2-Fluor-4-Cyanophenylboronsäurepinakolester als **E0-5** verwendet wurde und ***AAV4*** (Ausbeute 74 %).

MS (HPLC-MS):

| Molekülformel | m/z berechnet | m/z bestimmt |
|---|---|---|
| $C_{60}H_{37}N_5$ | 611,71 | 611,25 |

Figur 2 zeigt das Emissionsspektrum von Beispiel **2** (10 % in PMMA). Das Emissionsmaximum liegt bei 462 nm. Die Photolumineszenzquantenausbeute (PLQY) beträgt 53 % und die Halbwertsbreite beträgt 0,41 eV. Die Emissionsabklingzeit beträgt 15 µs.

**Beispiel 3**

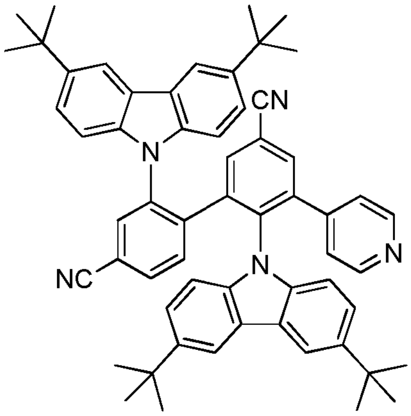

Beispiel **3** wurde gemäß folgender Synthesevorschriften hergestellt.

***AAV2b*** (Ausbeute 33 %), wobei 3,5-Dichlor-4-fluorbenzonitril als **E0-1** verwendet wurde und 4-Pyridyl-boronsäure als **E0-3b** verwendet wurde;

**AAV3** (Ausbeute 75 %), wobei 2-Fluor-4-Cyanophenylboronsäurepinakolester als **E0-5** verwendet wurde und

***AAV4*** (Ausbeute 74 %).

MS (HPLC-MS):

| Molekülformel | m/z berechnet | m/z bestimmt |
|---|---|---|
| $C_{60}H_{37}N_5$ | 836,14 | 835,47 |

Figur 3 zeigt das Emissionsspektrum von Beispiel **3** (10 % in PMMA). Das Emissionsmaximum liegt bei 478 nm. Die Photolumineszenzquantenausbeute (PLQY) beträgt 75 % und die Halbwertsbreite beträgt 0,40 eV. Die Emissionsabklingzeit beträgt 9 µs.

**Weitere Beispiele erfindungsgemäßer Moleküle**

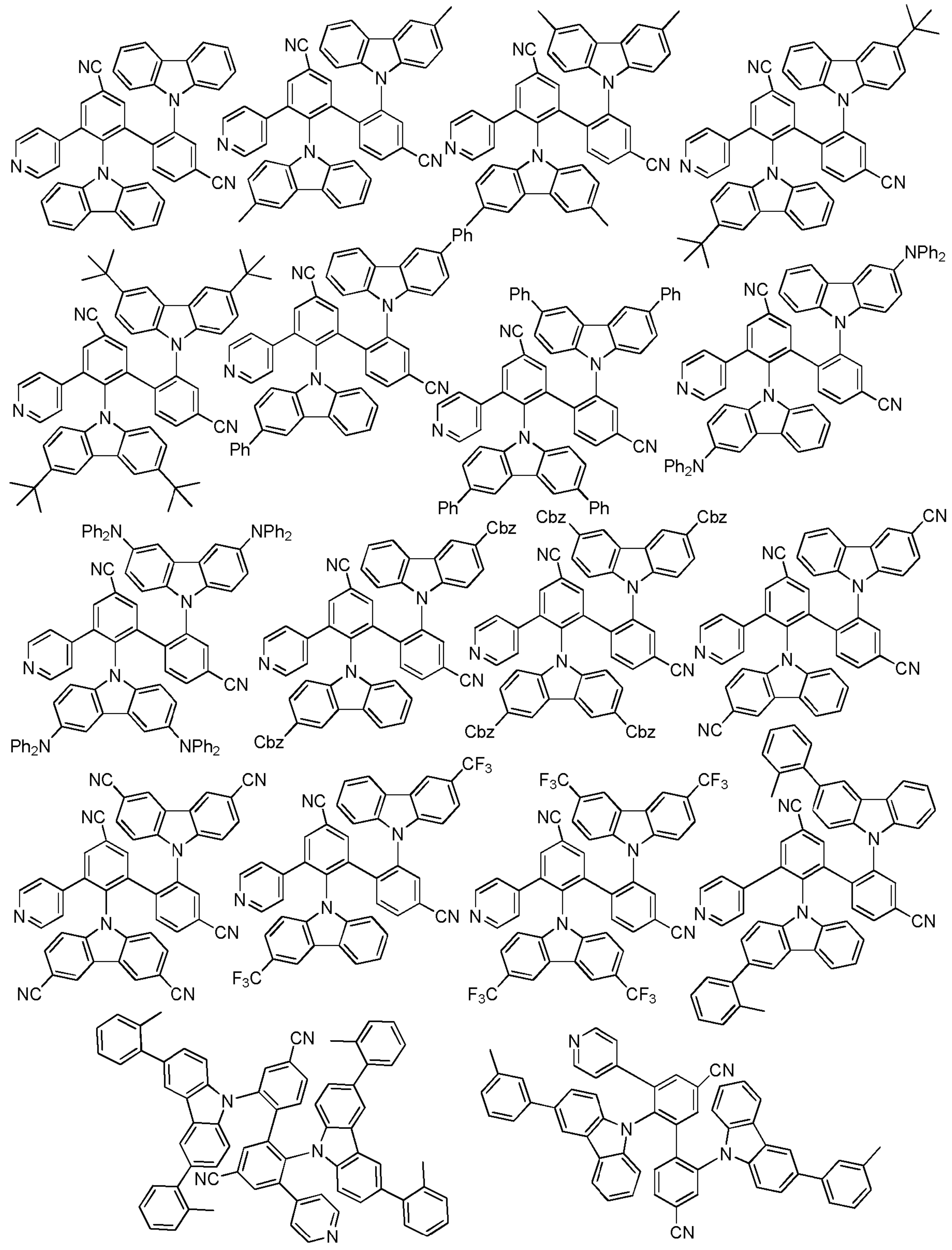

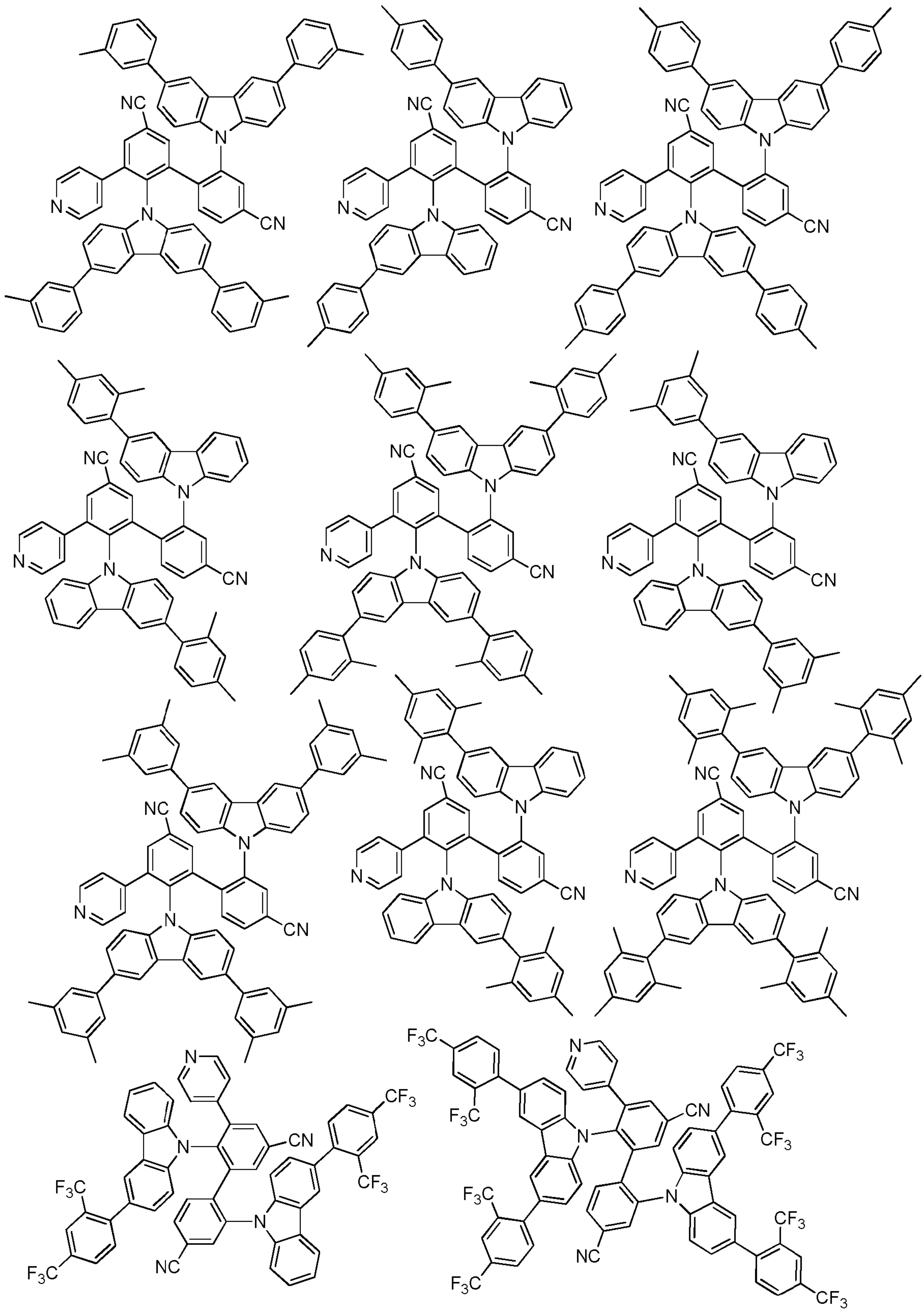

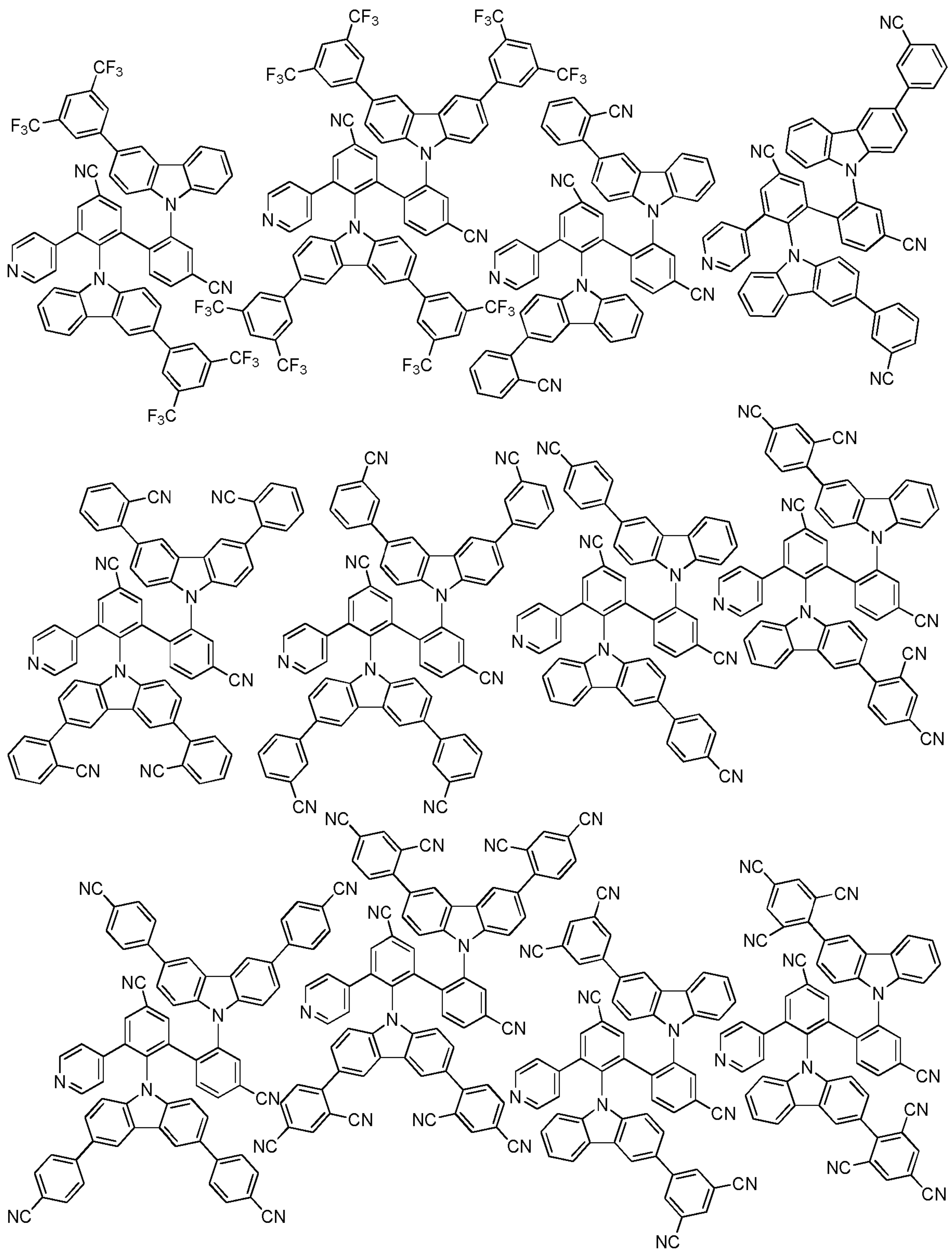

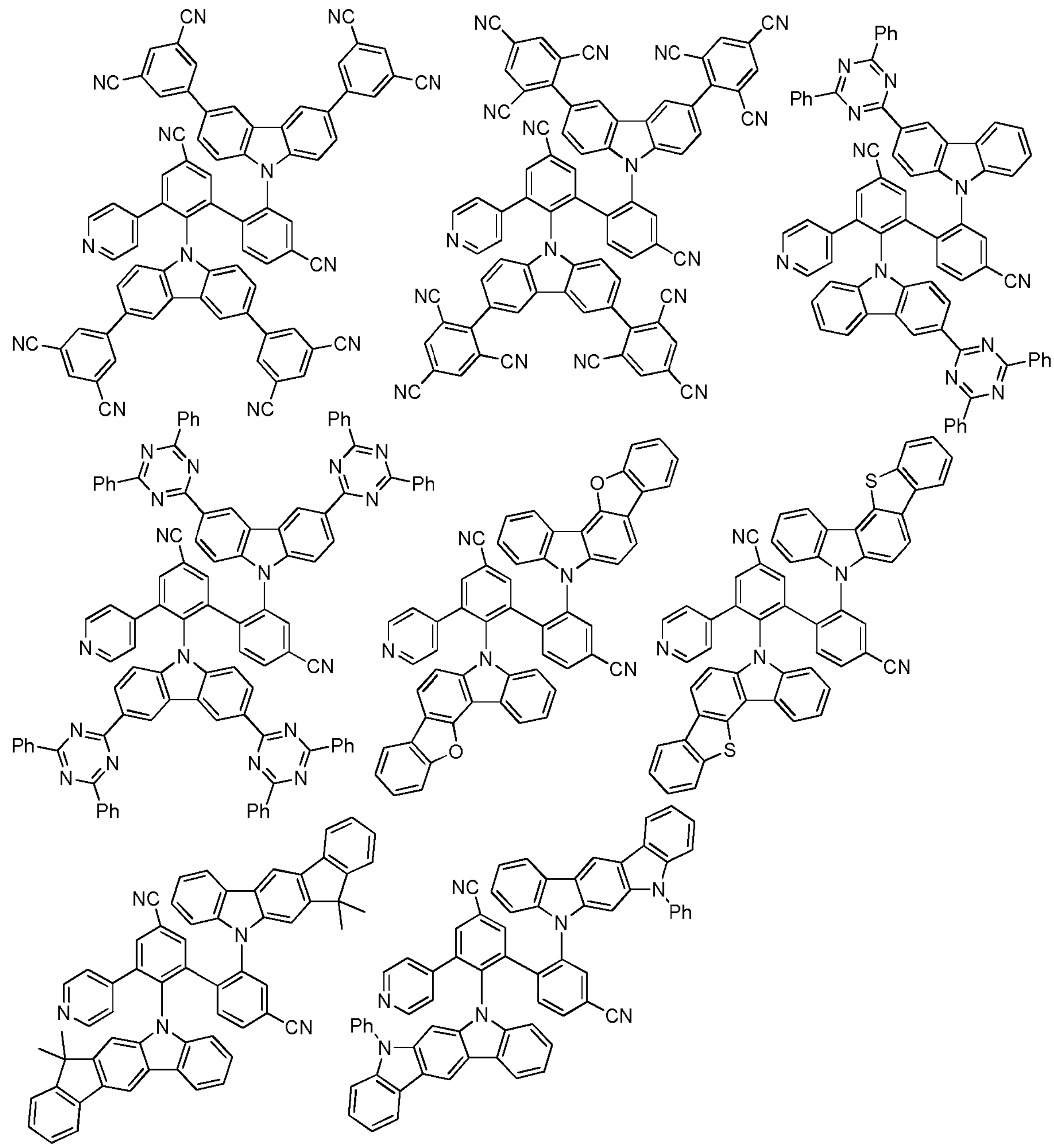

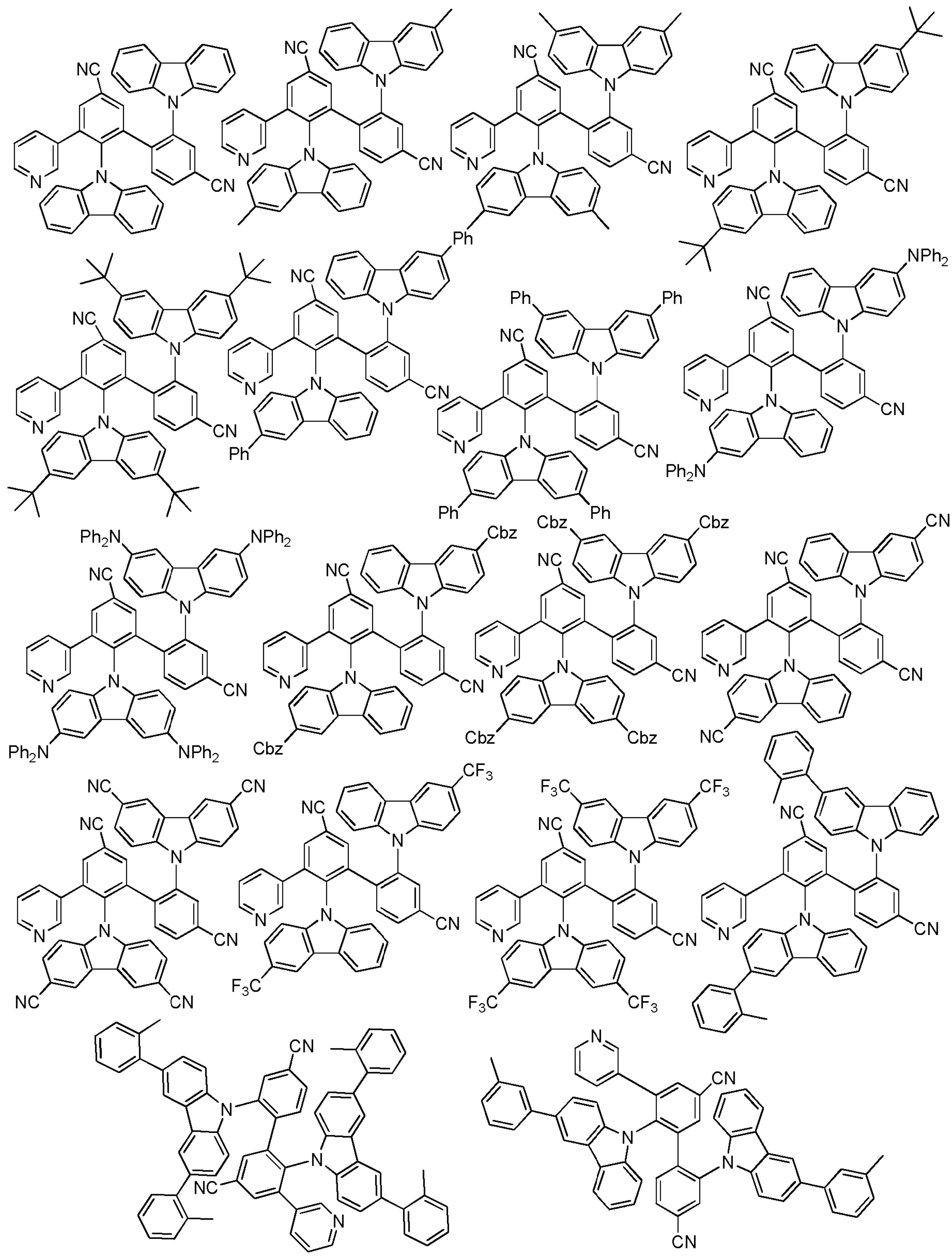

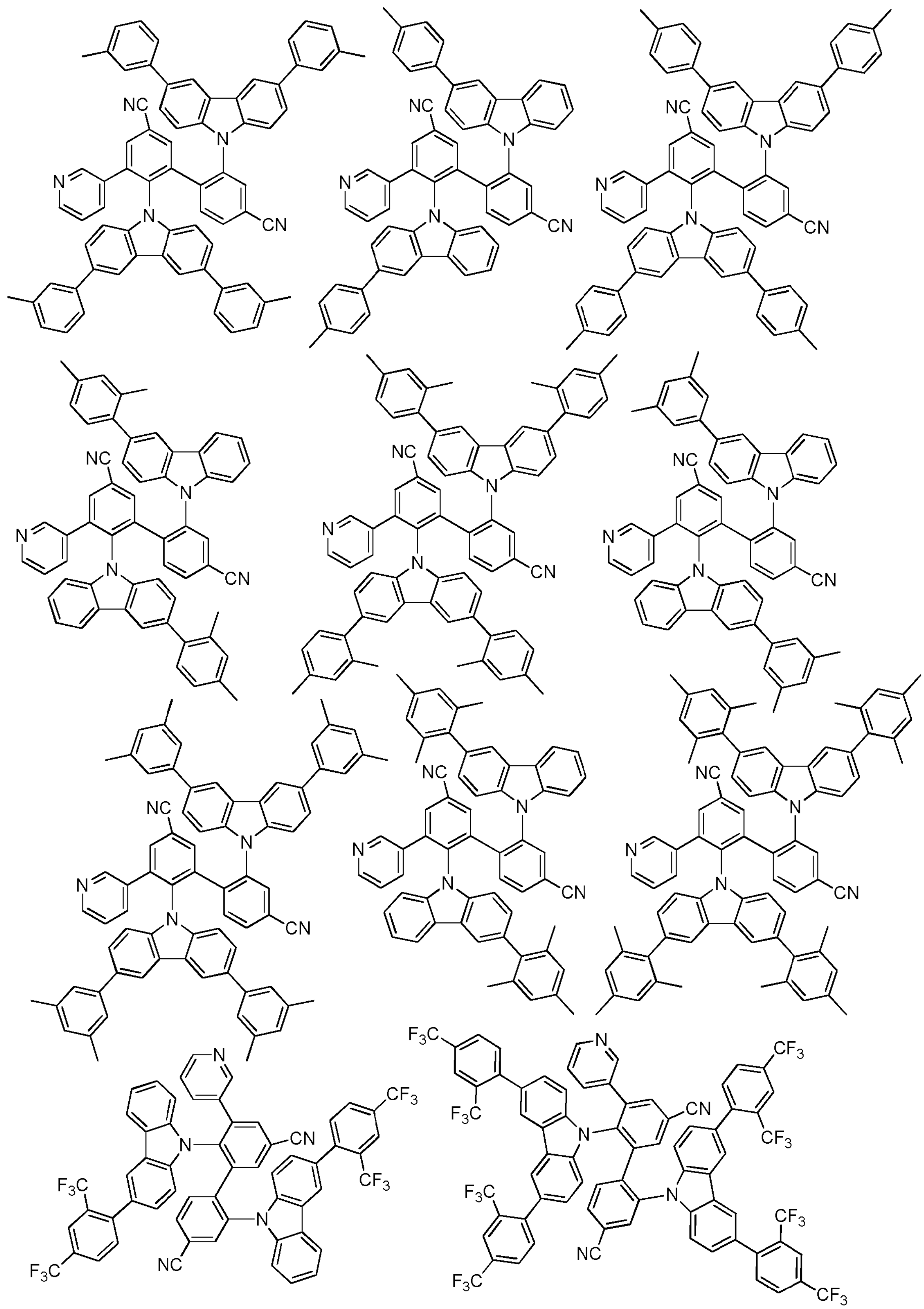

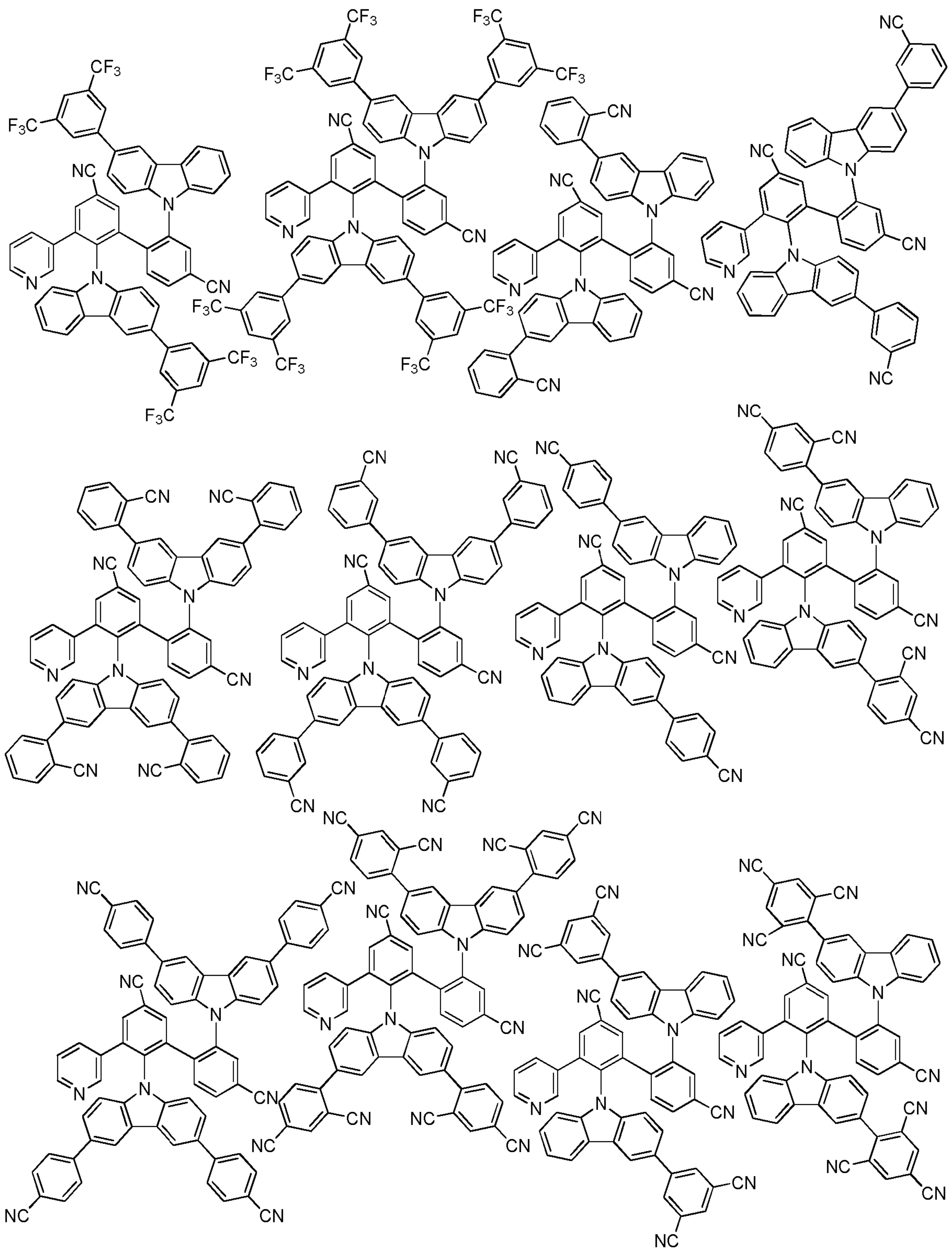

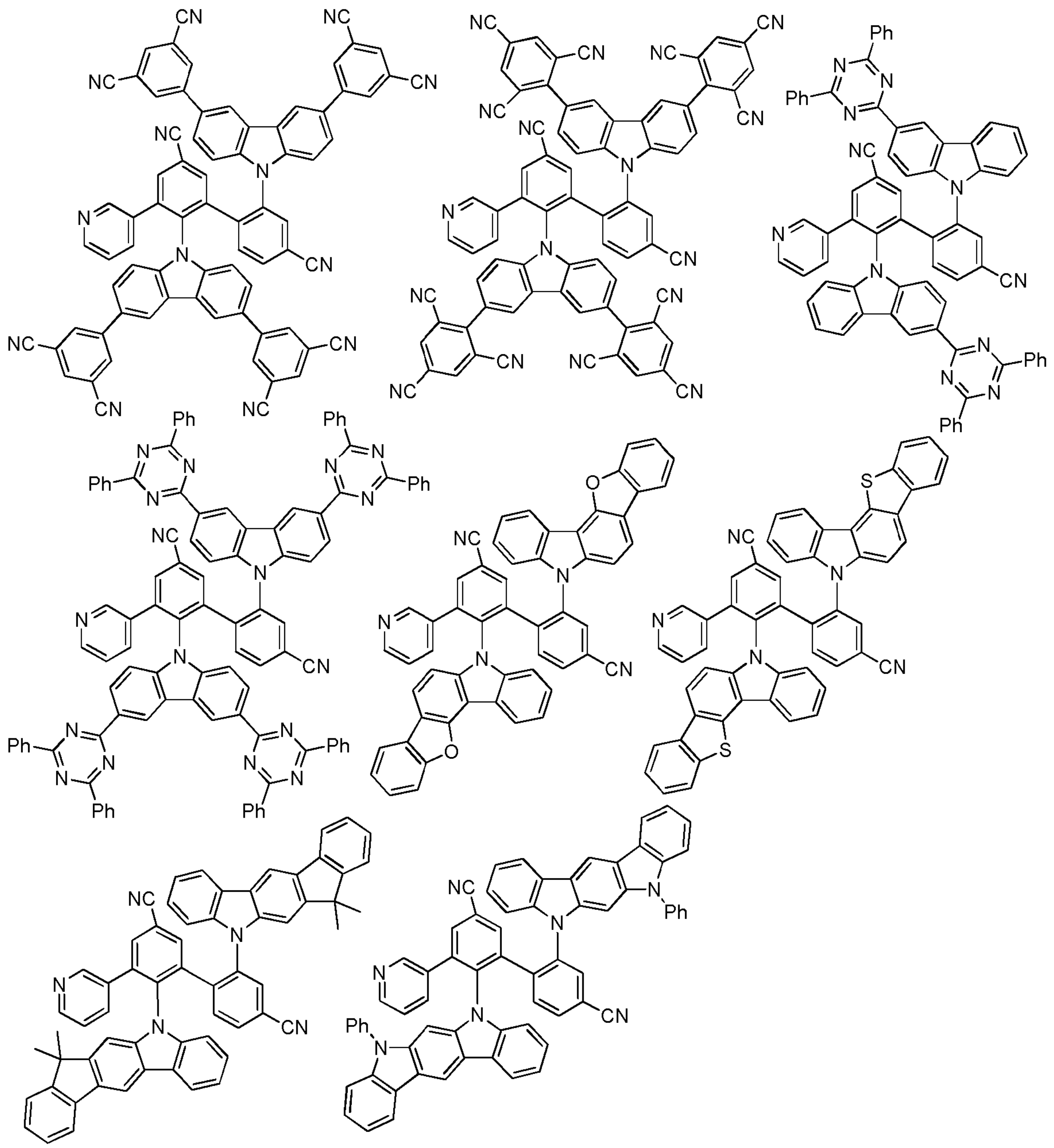

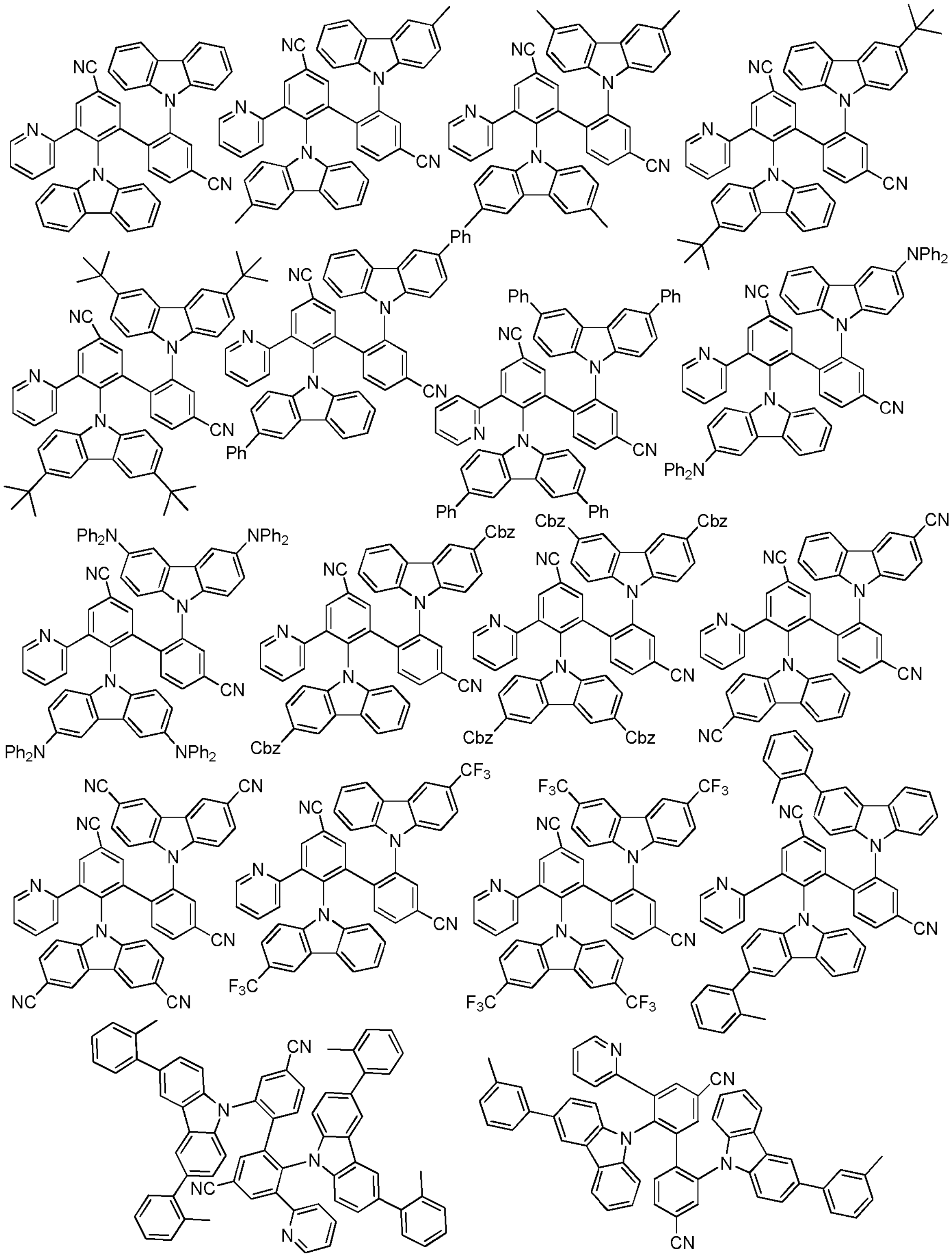

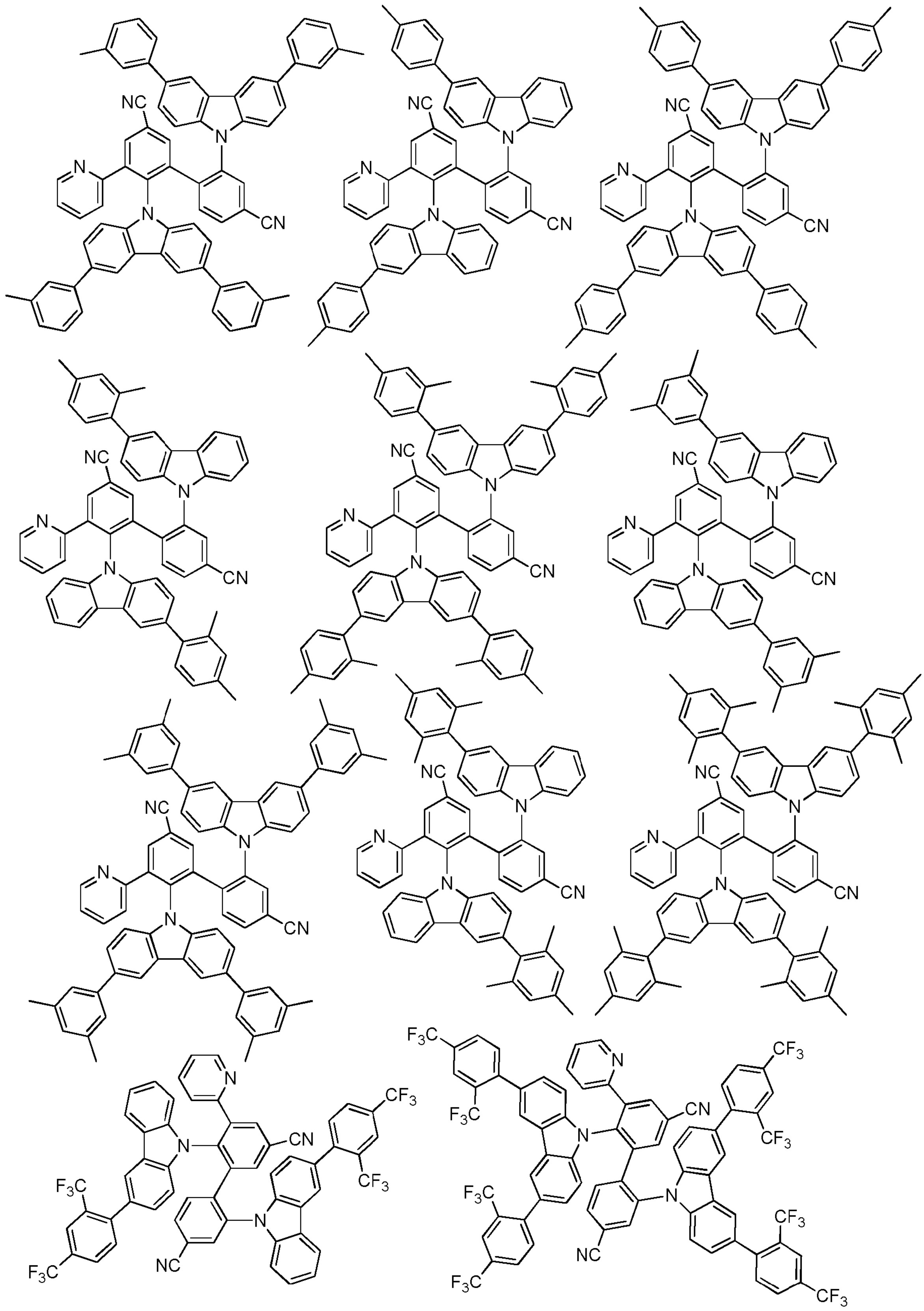

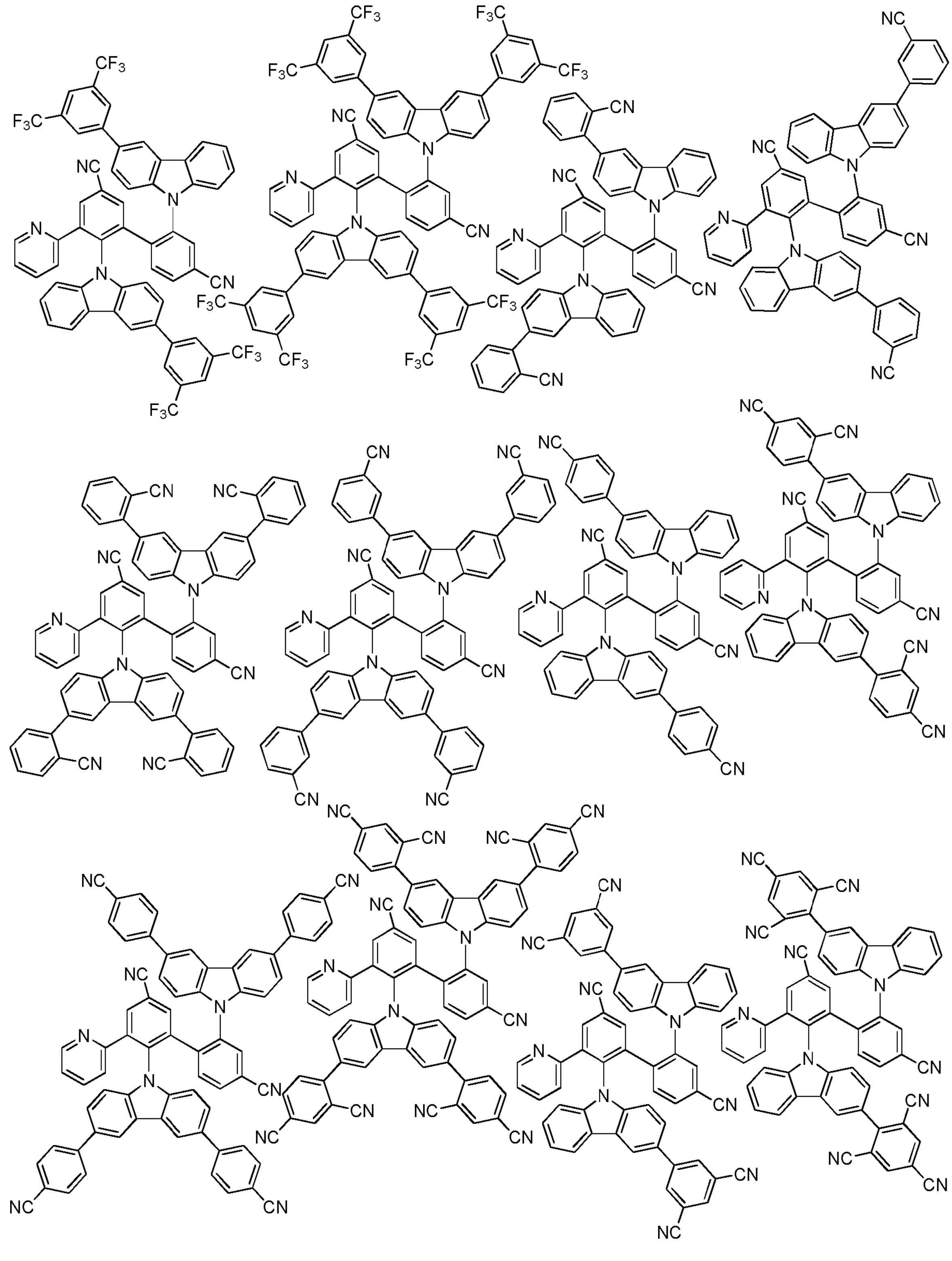

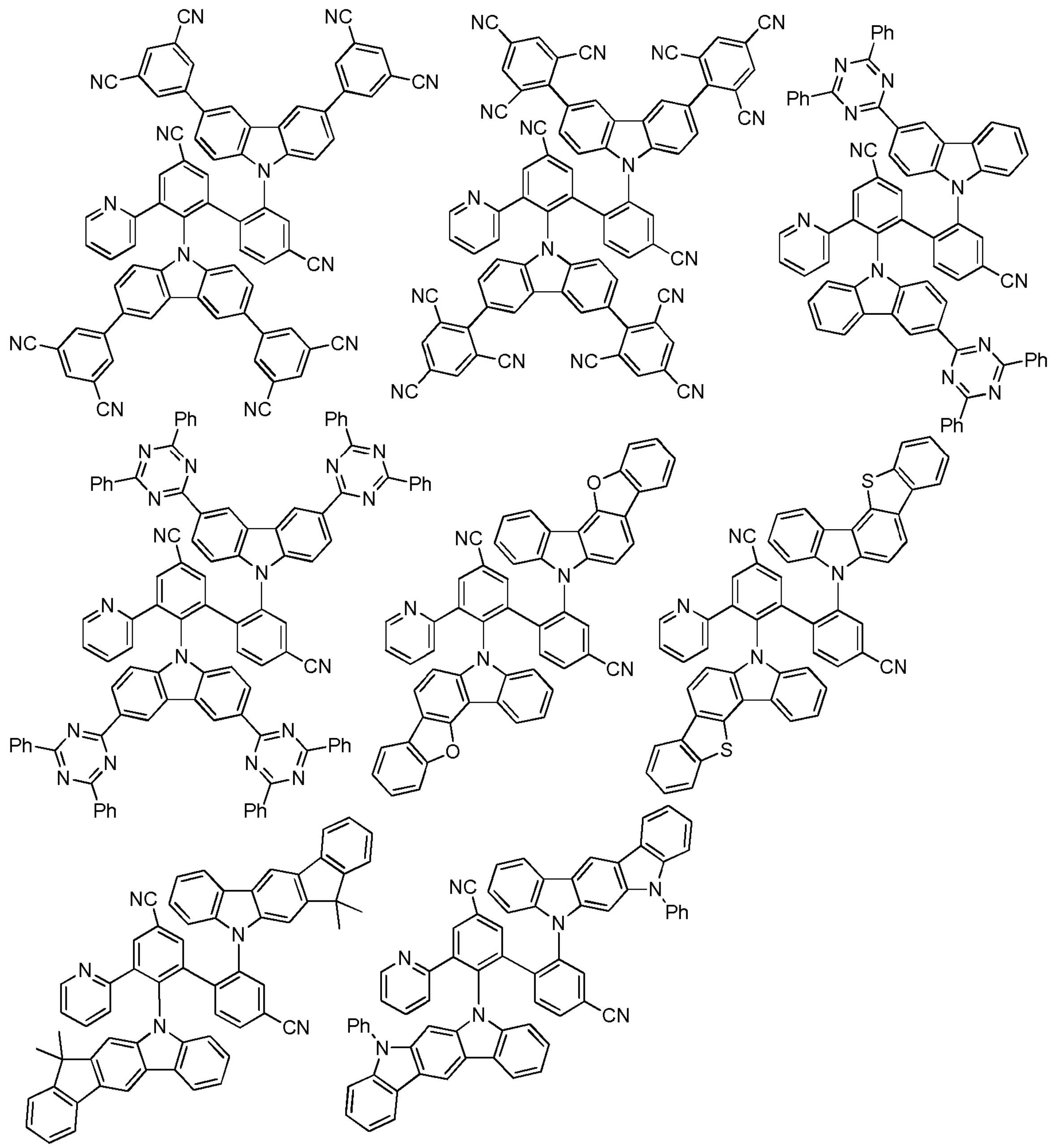

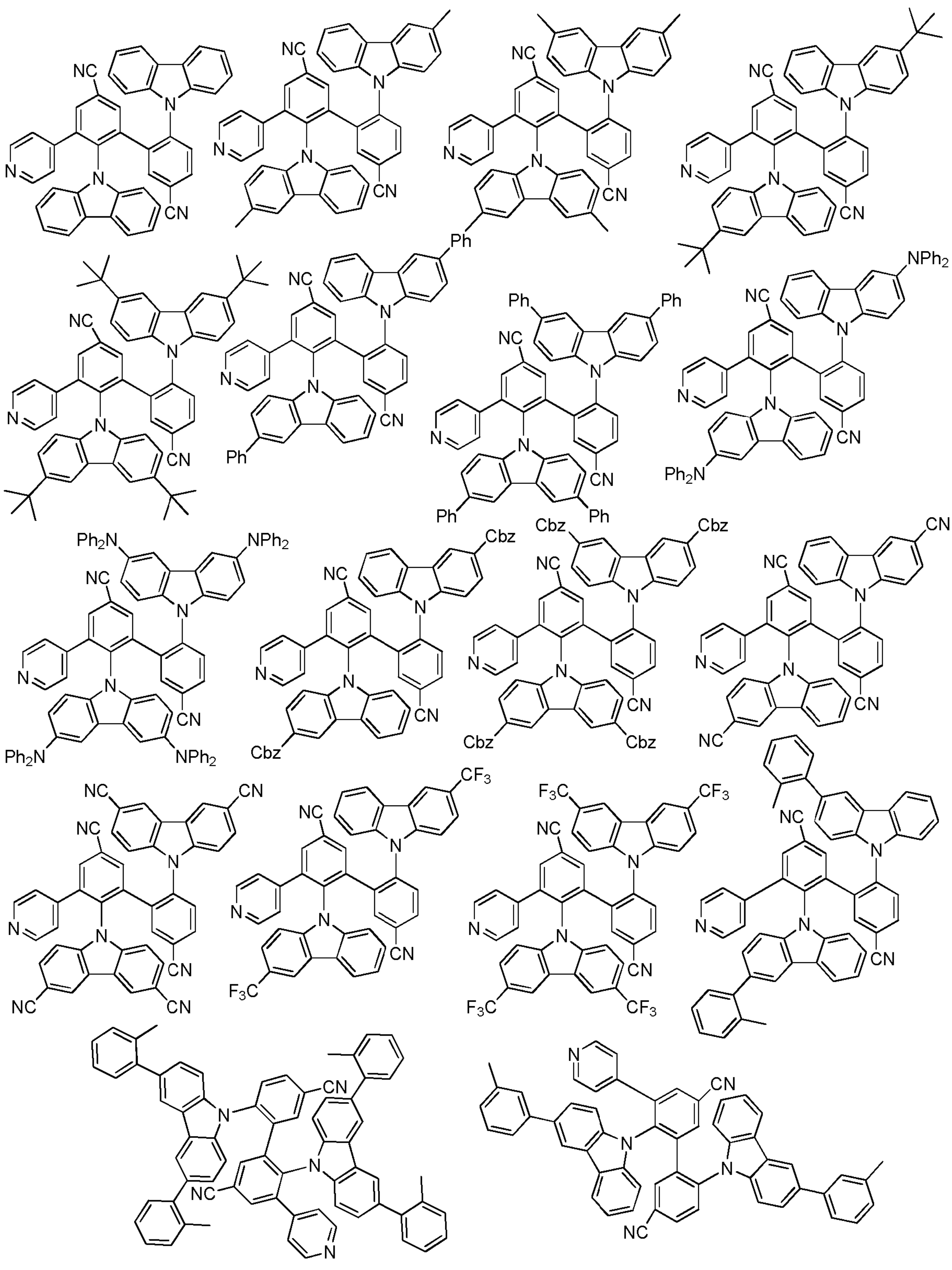

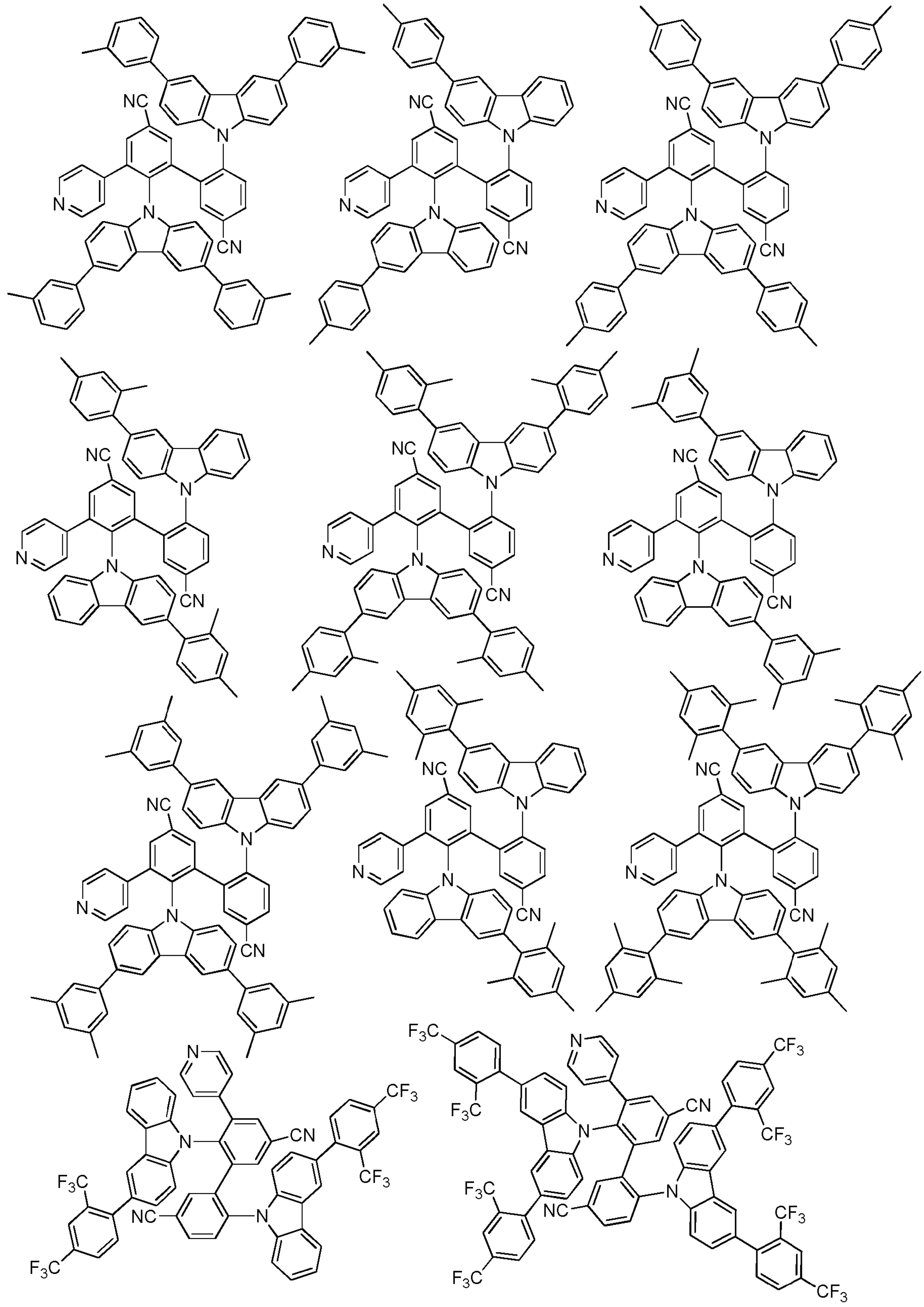

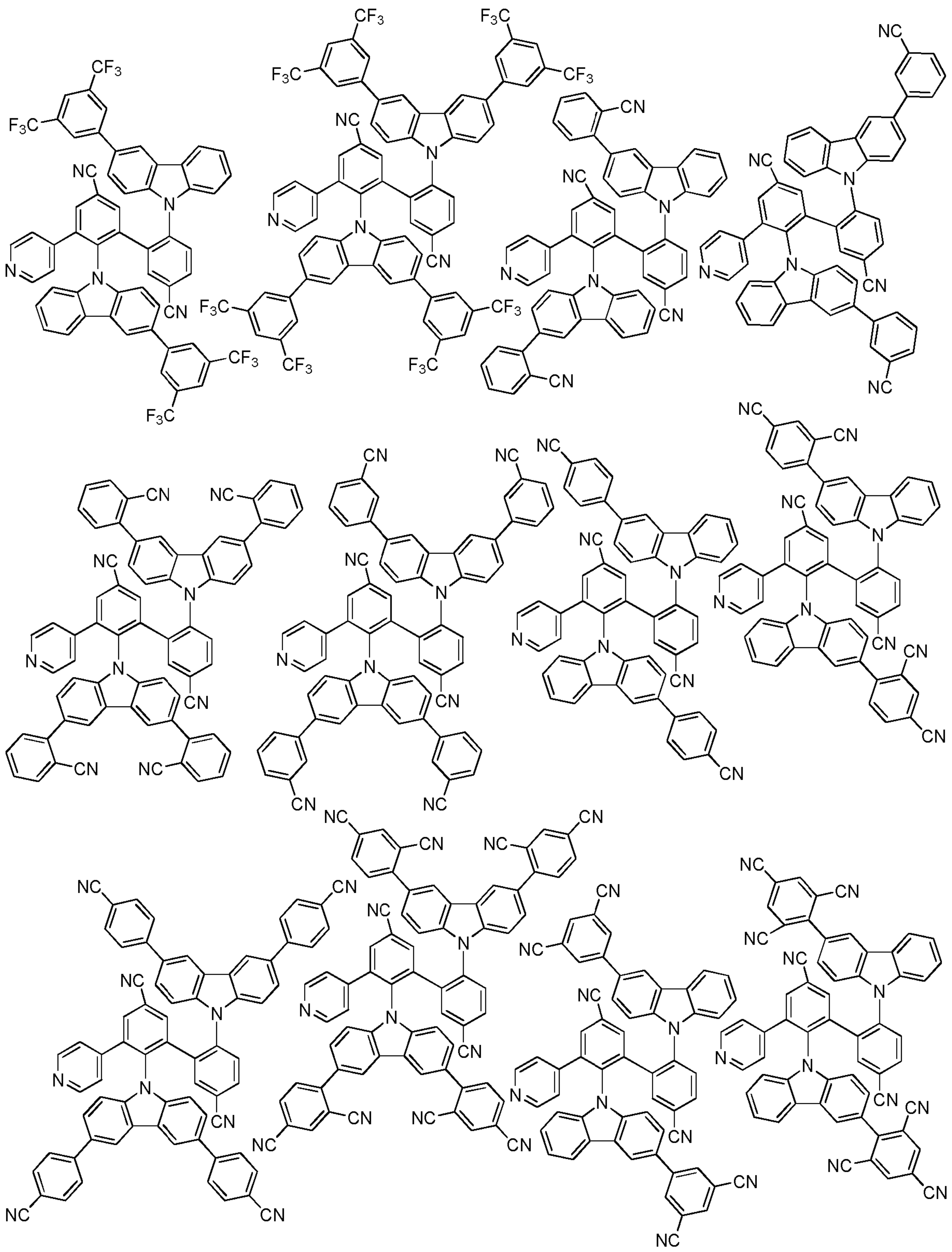

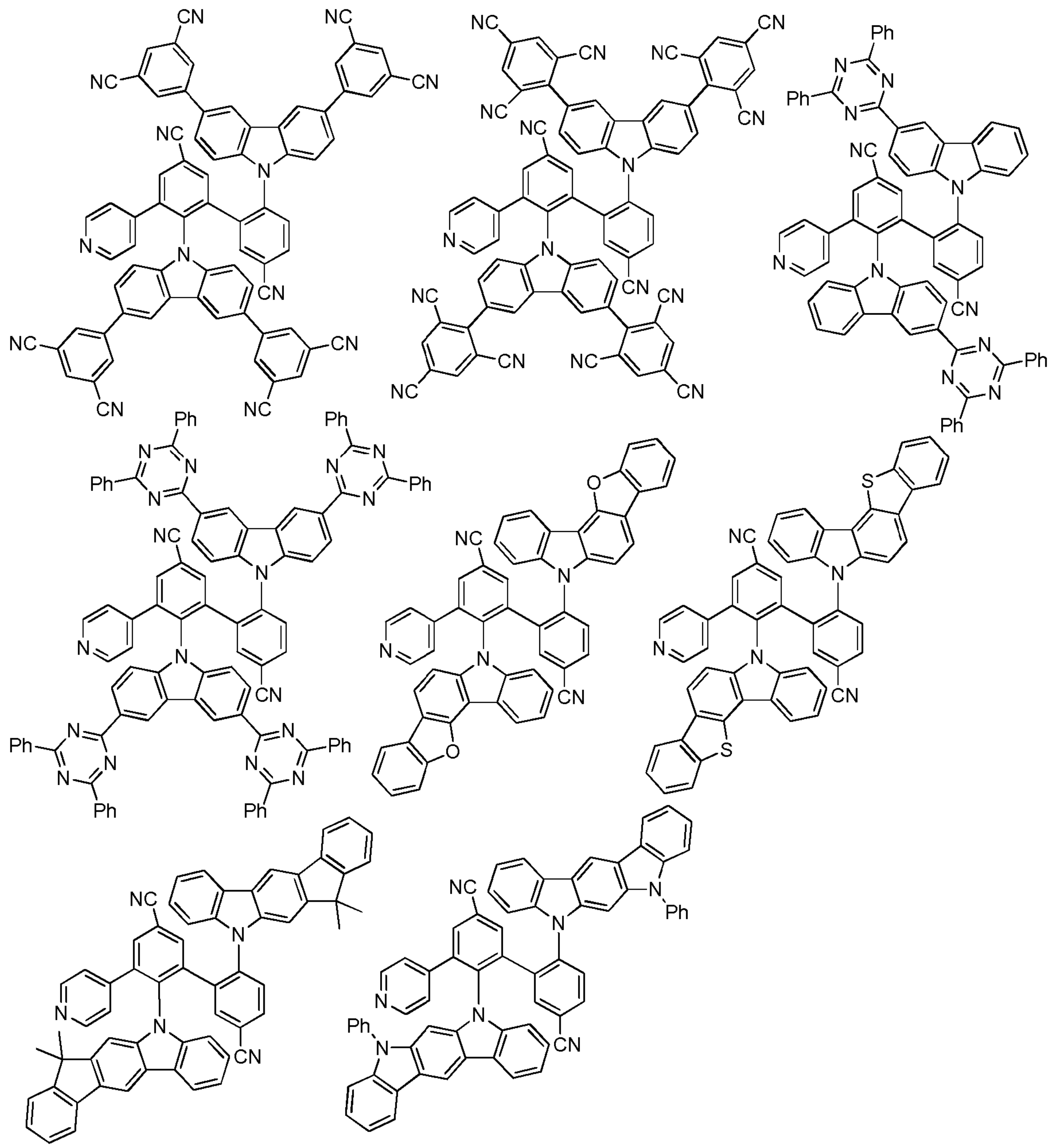

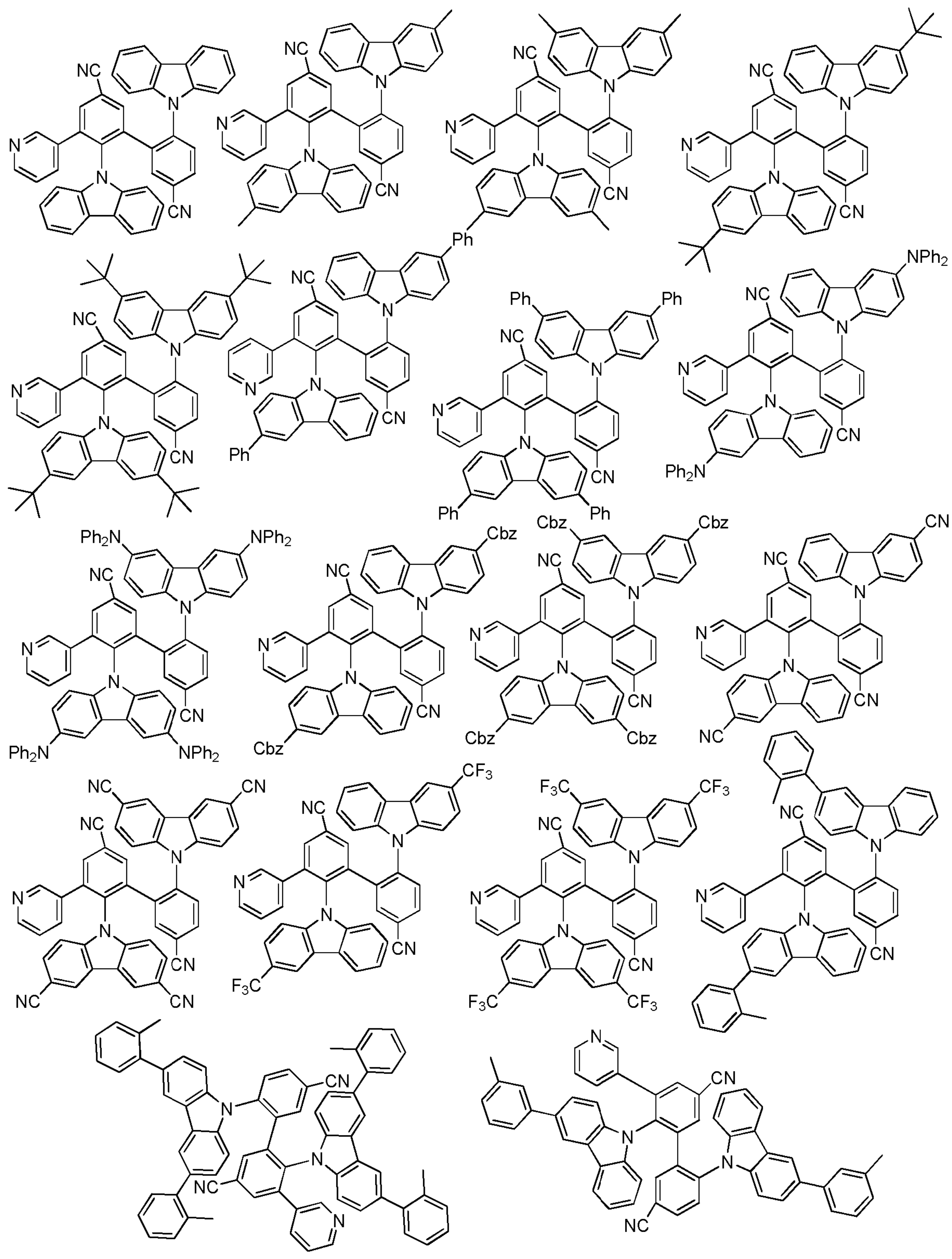

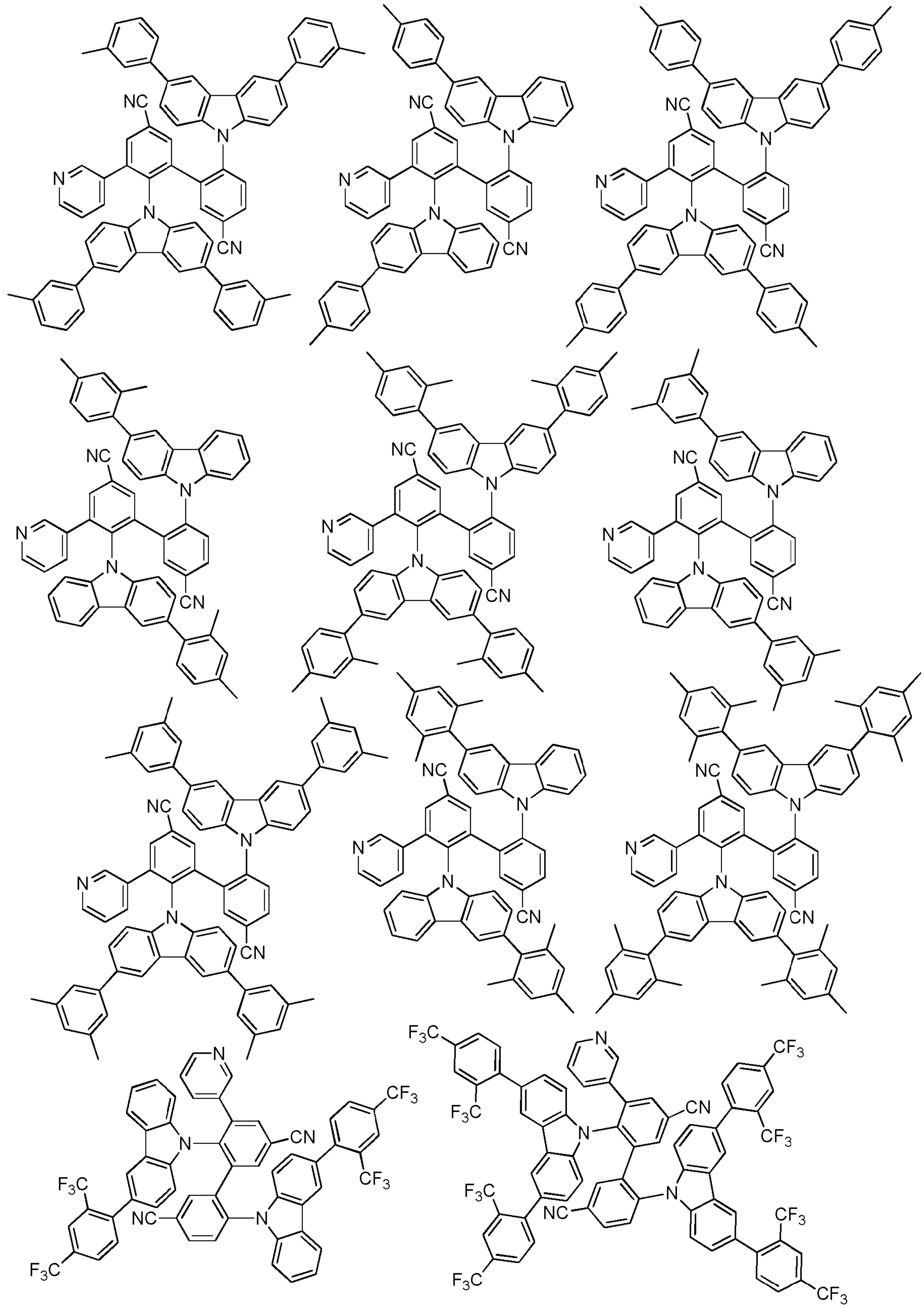

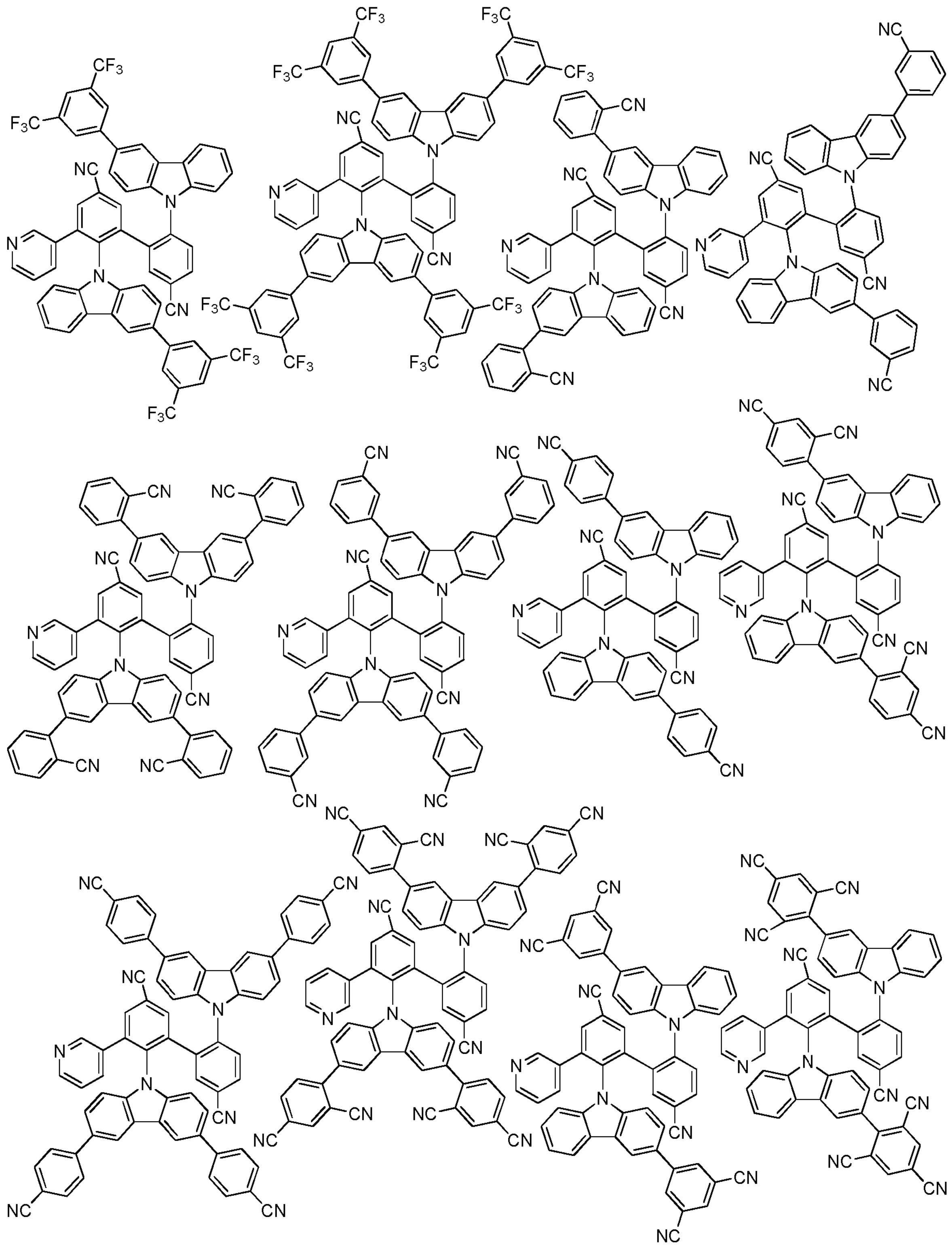

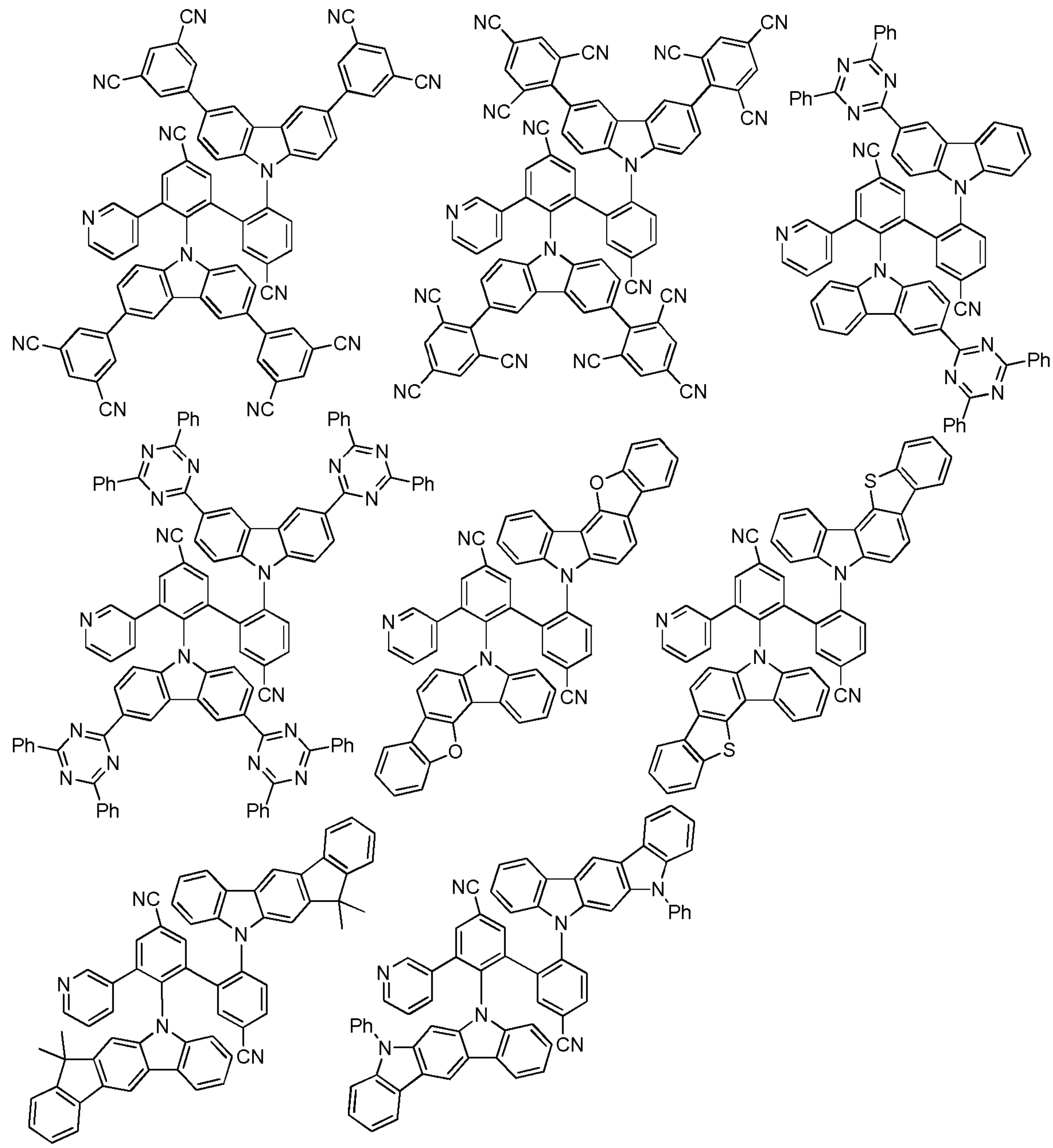

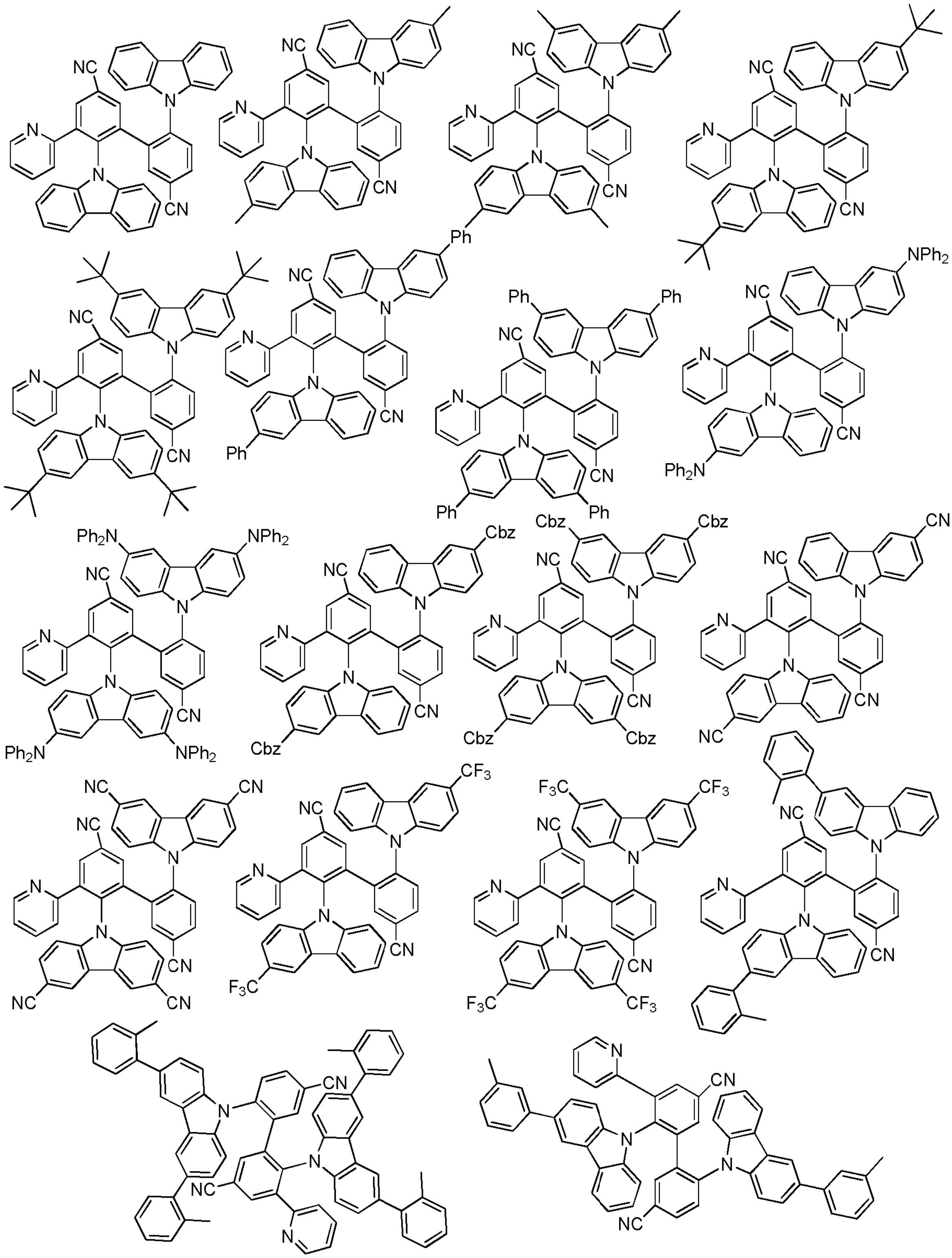

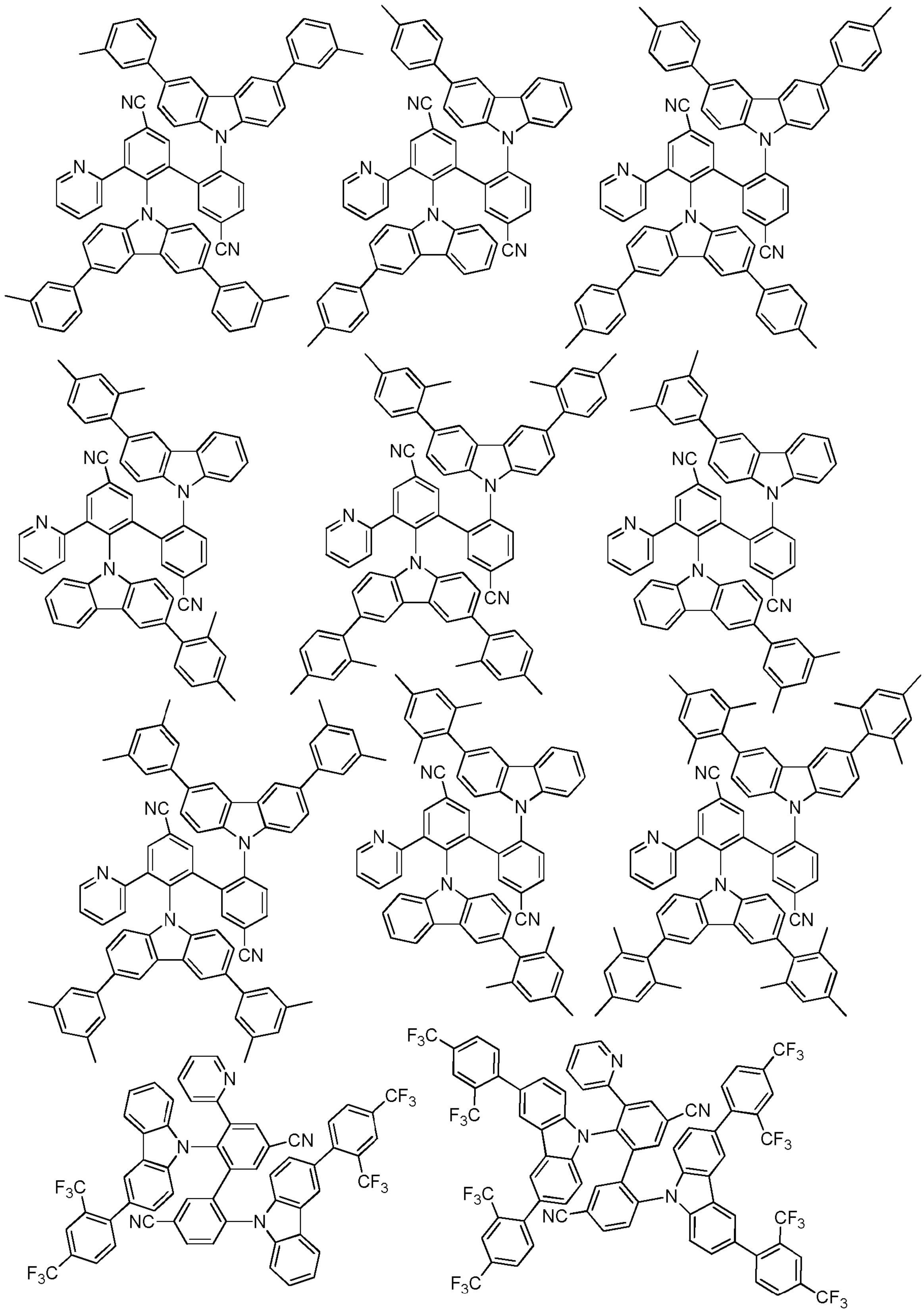

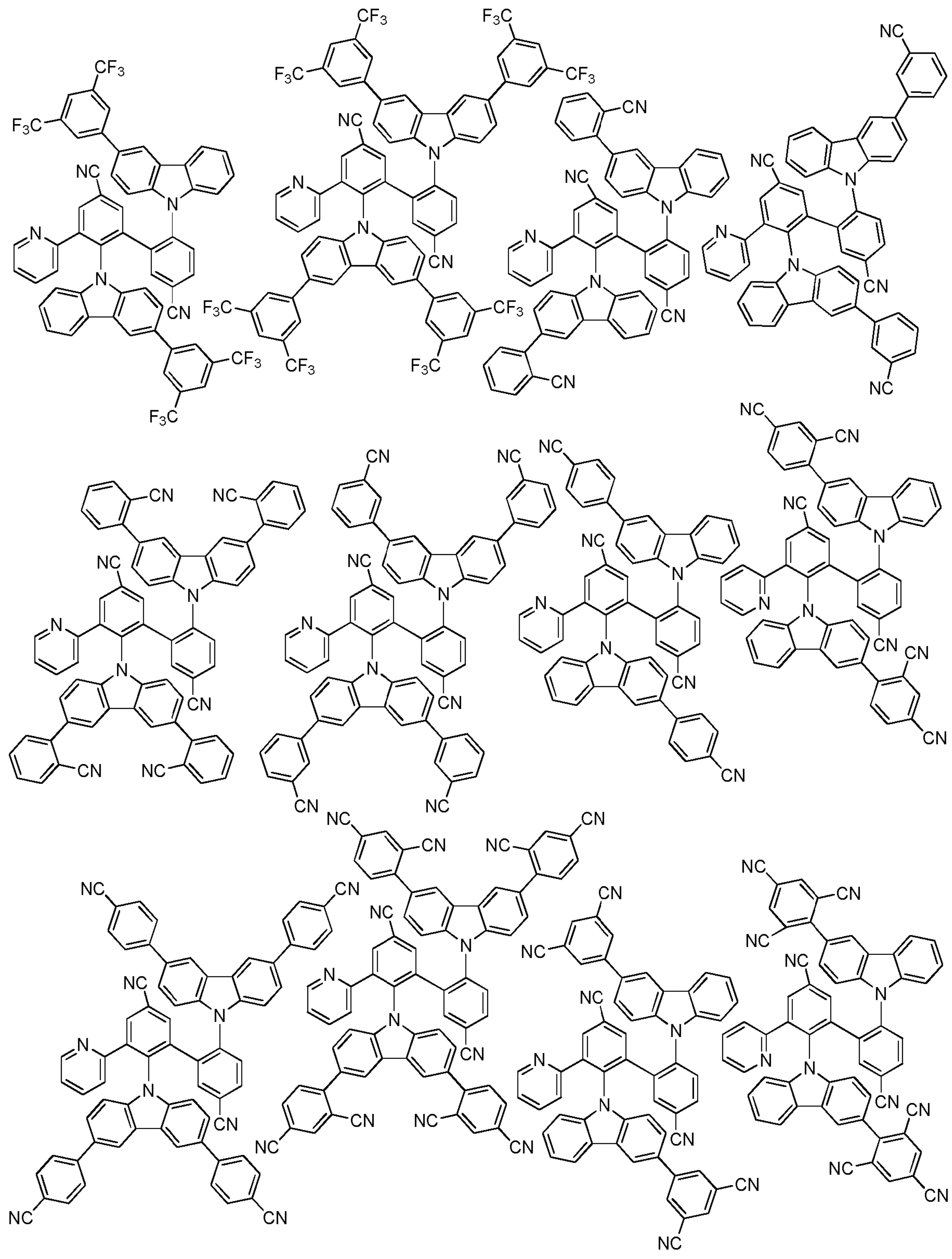

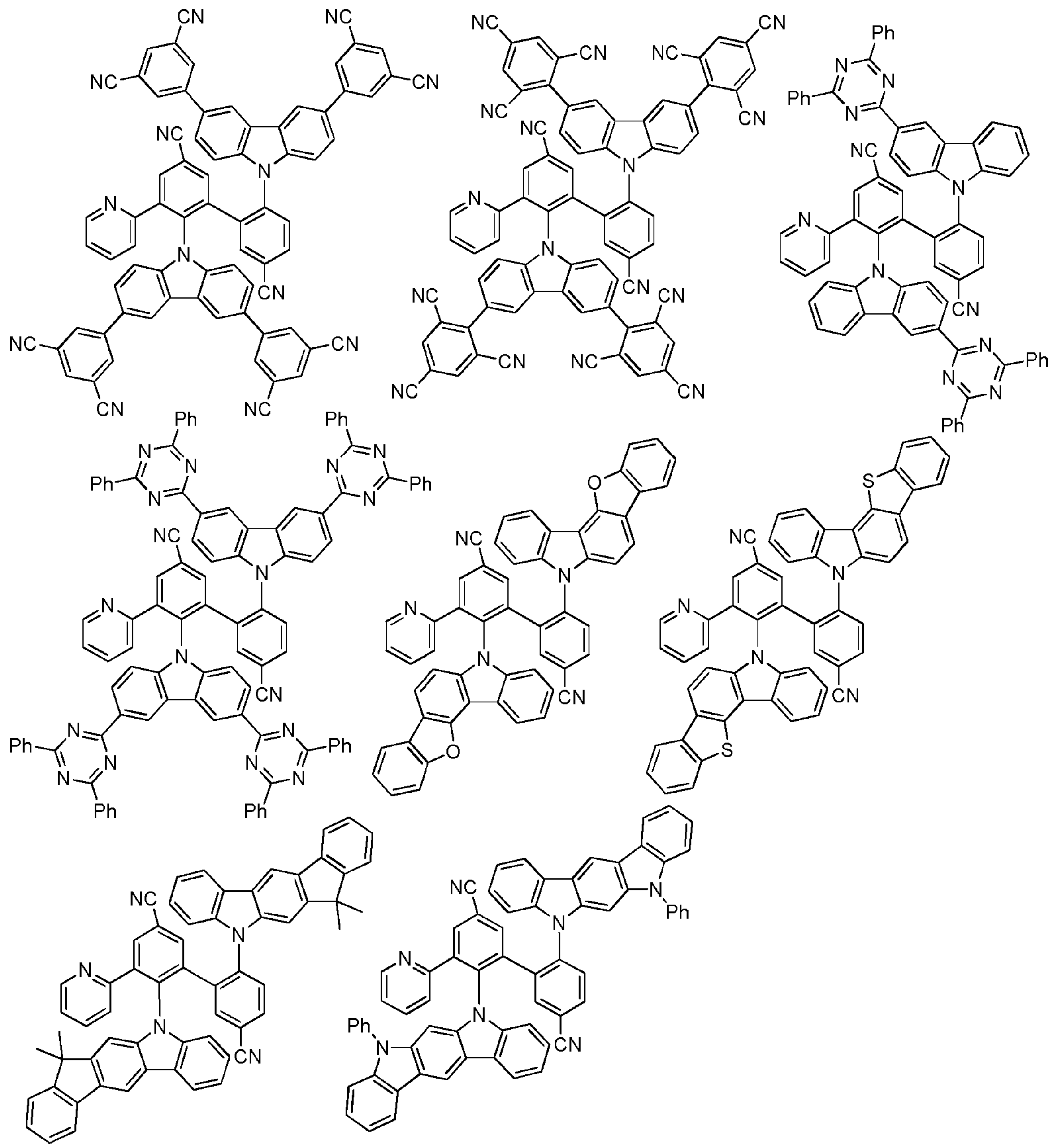

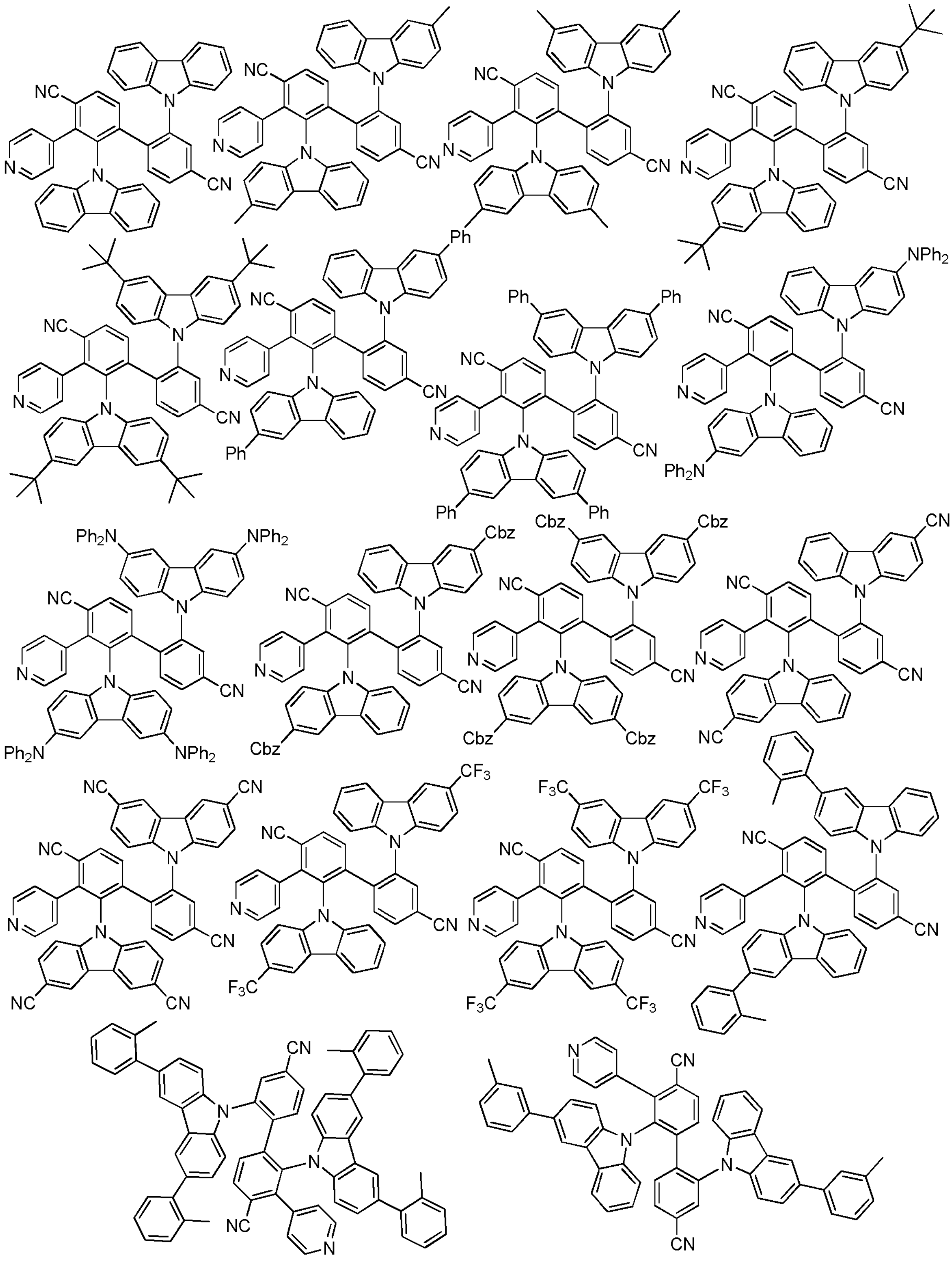

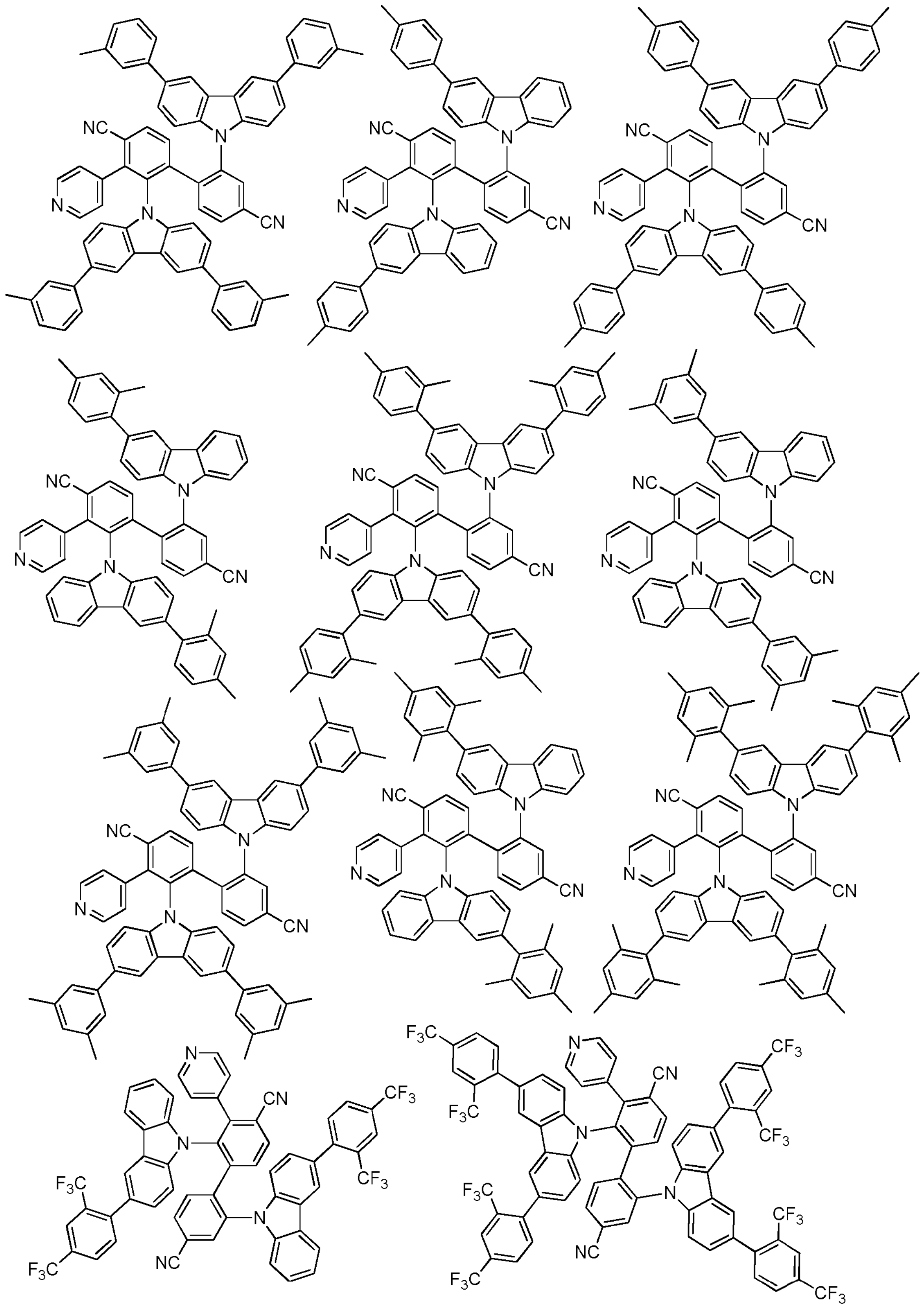

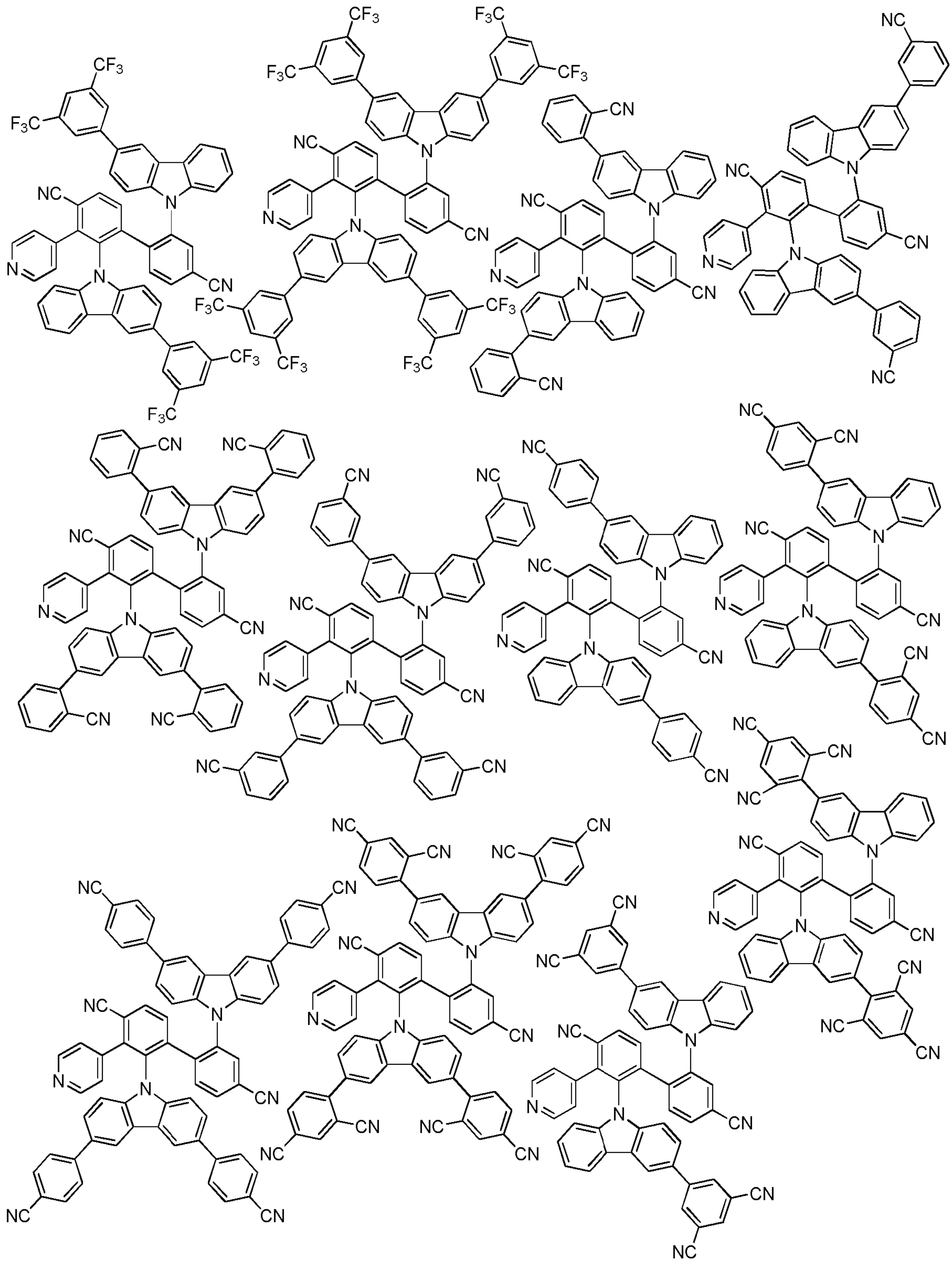

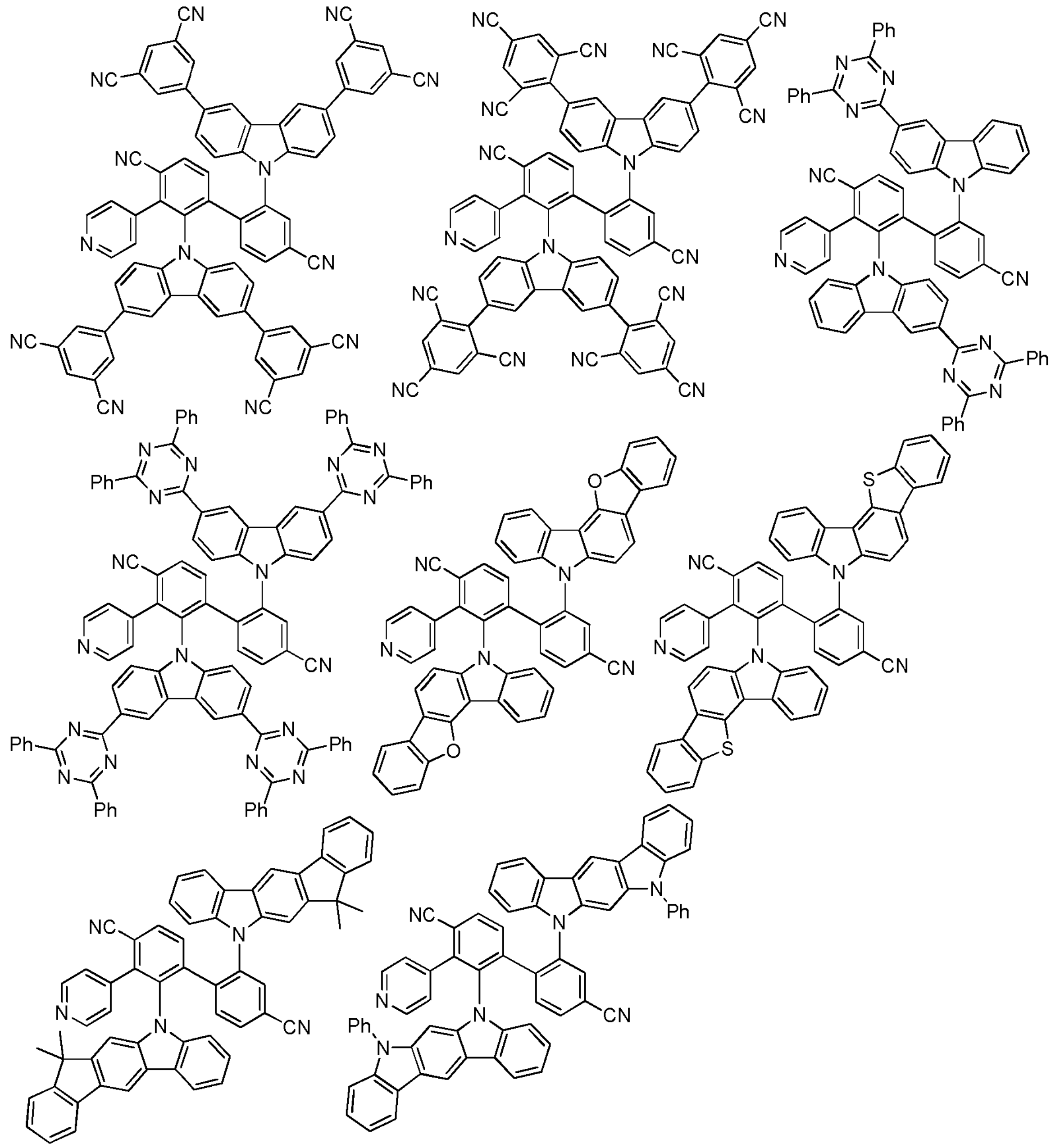

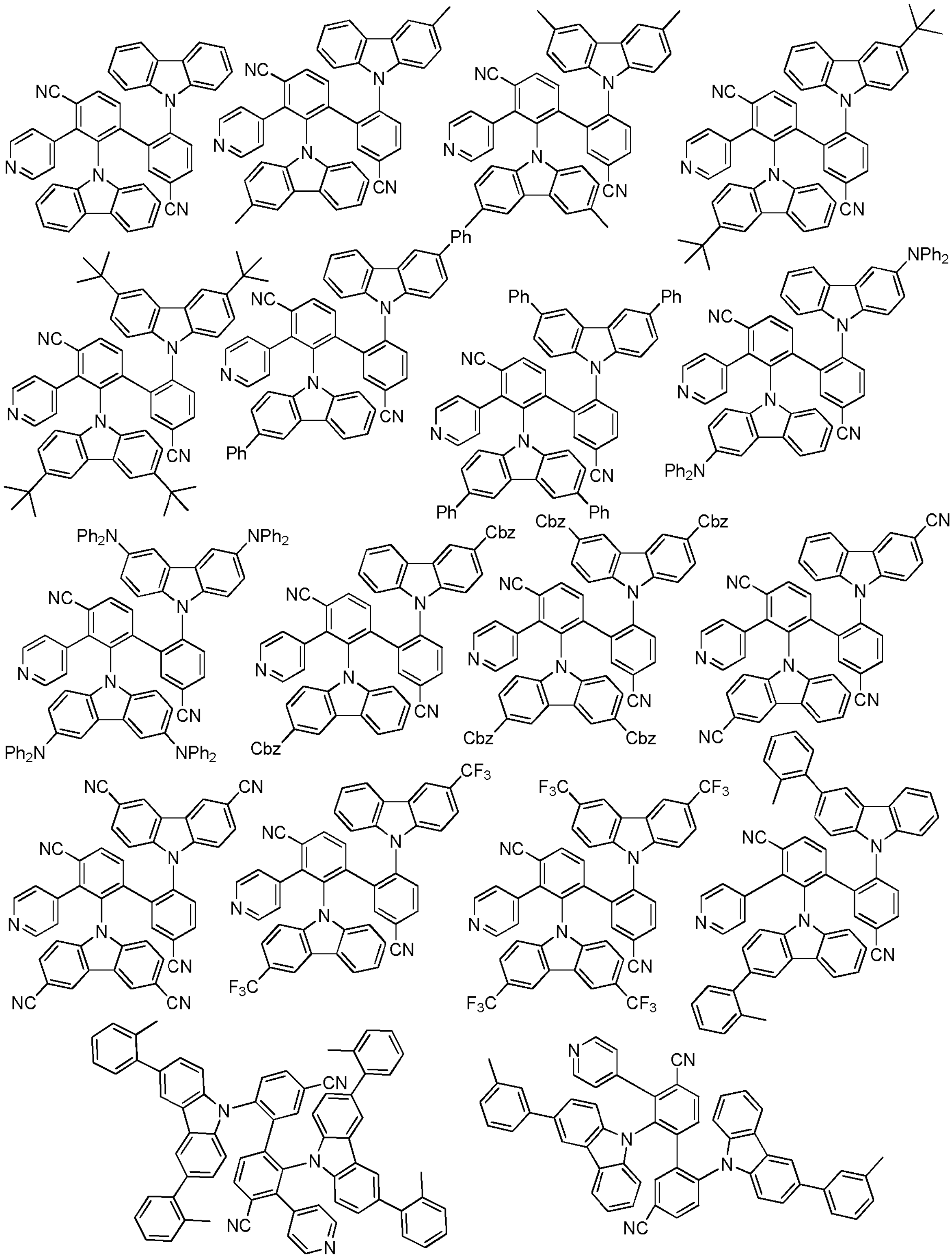

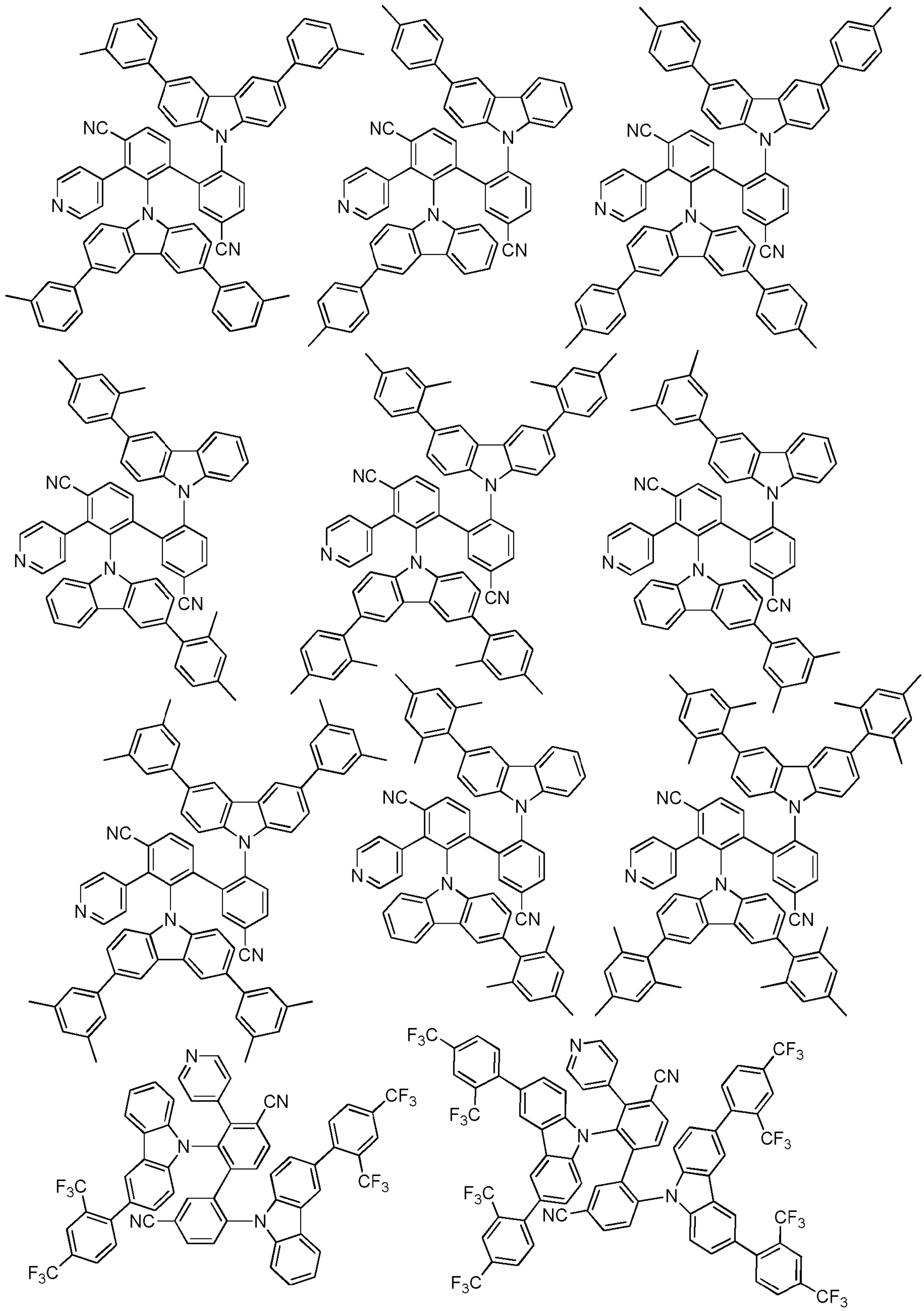

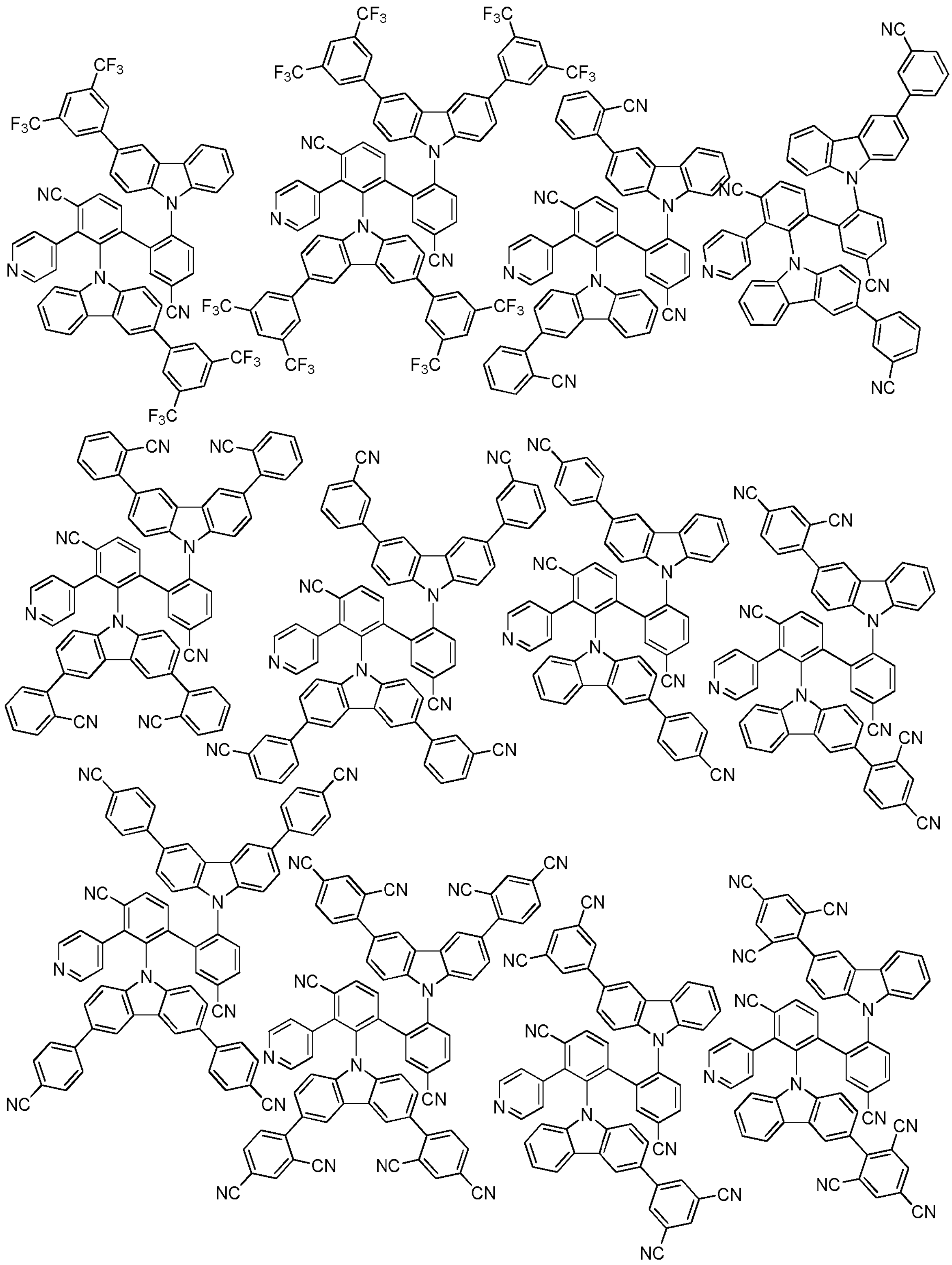

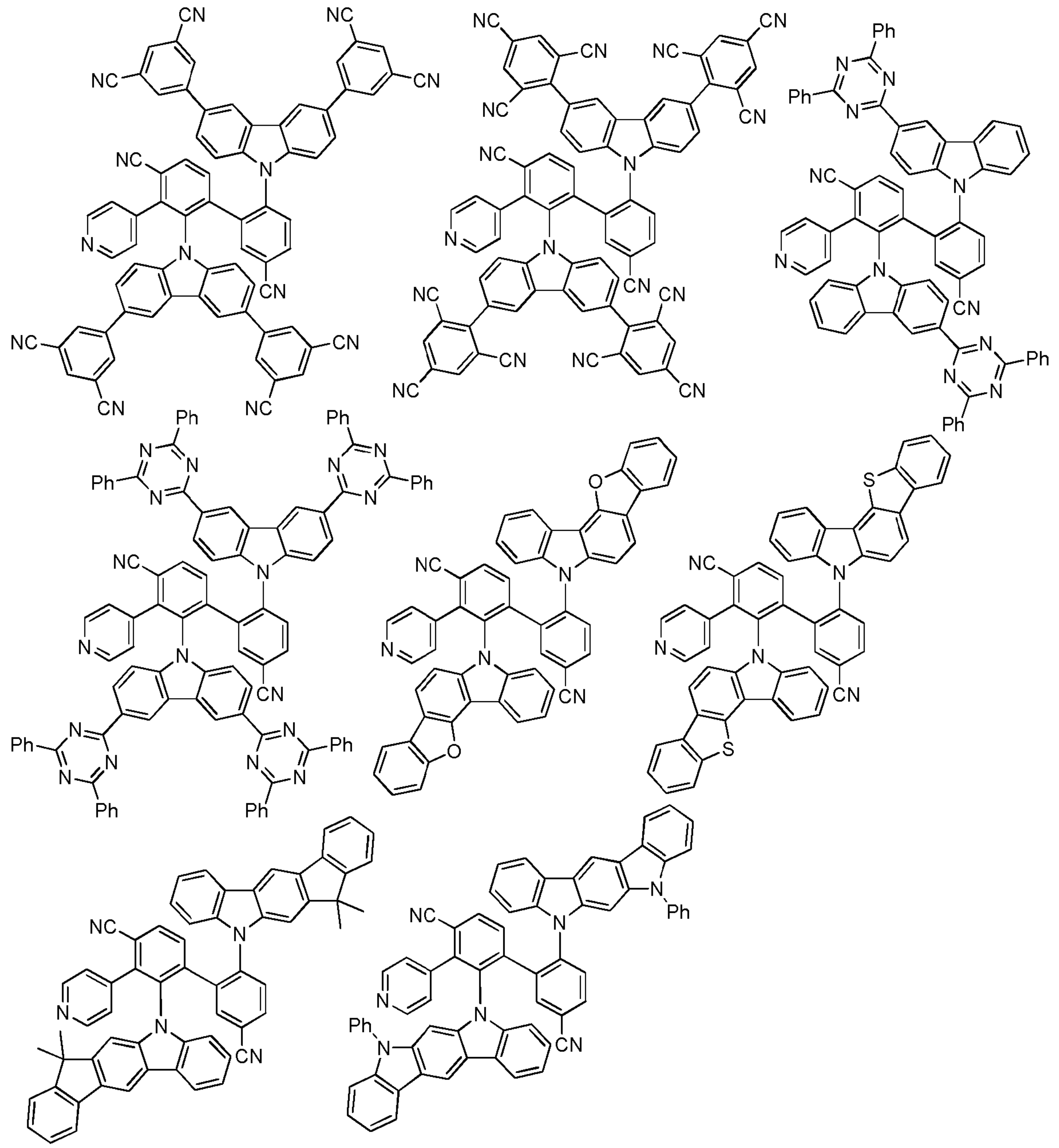

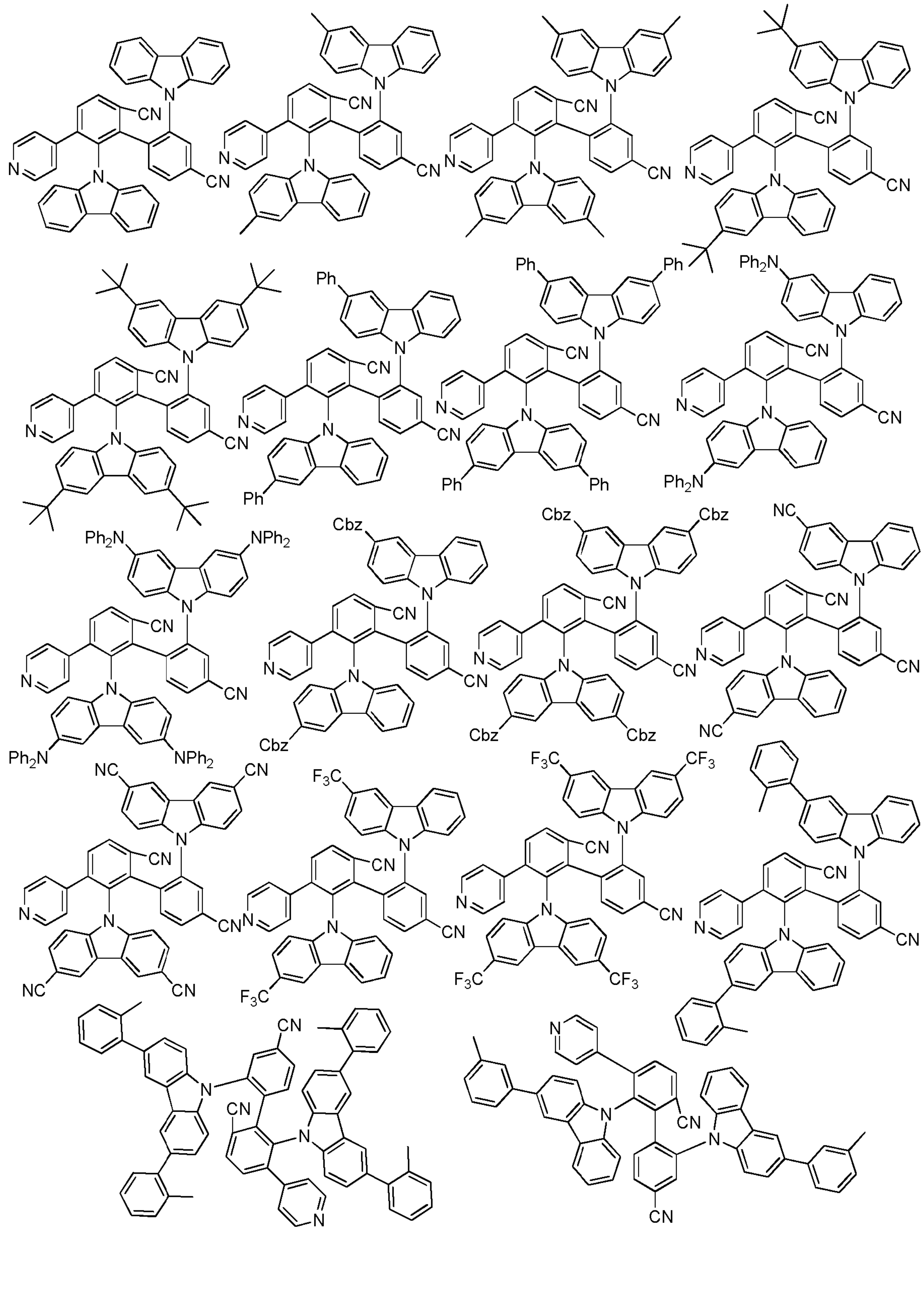

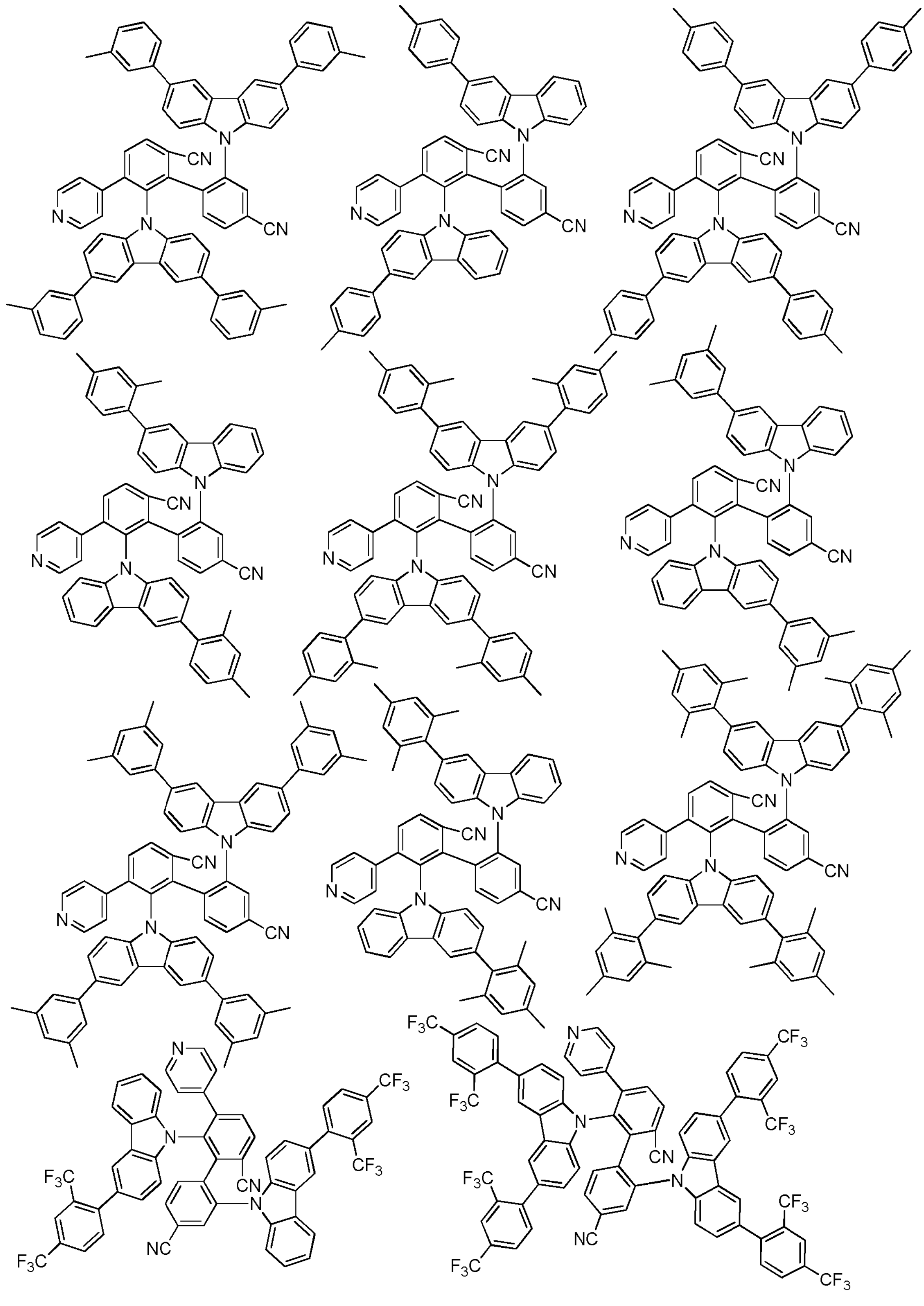

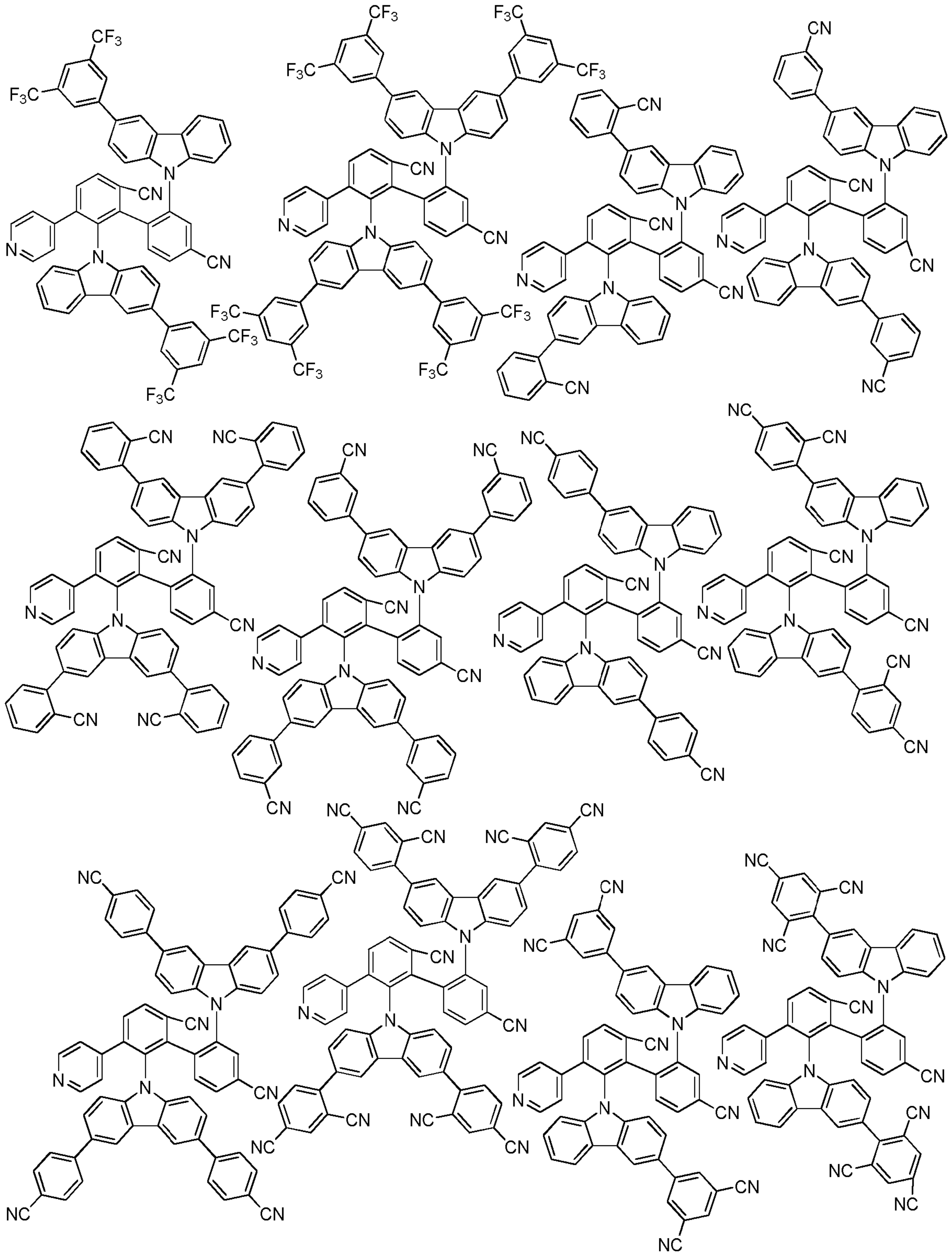

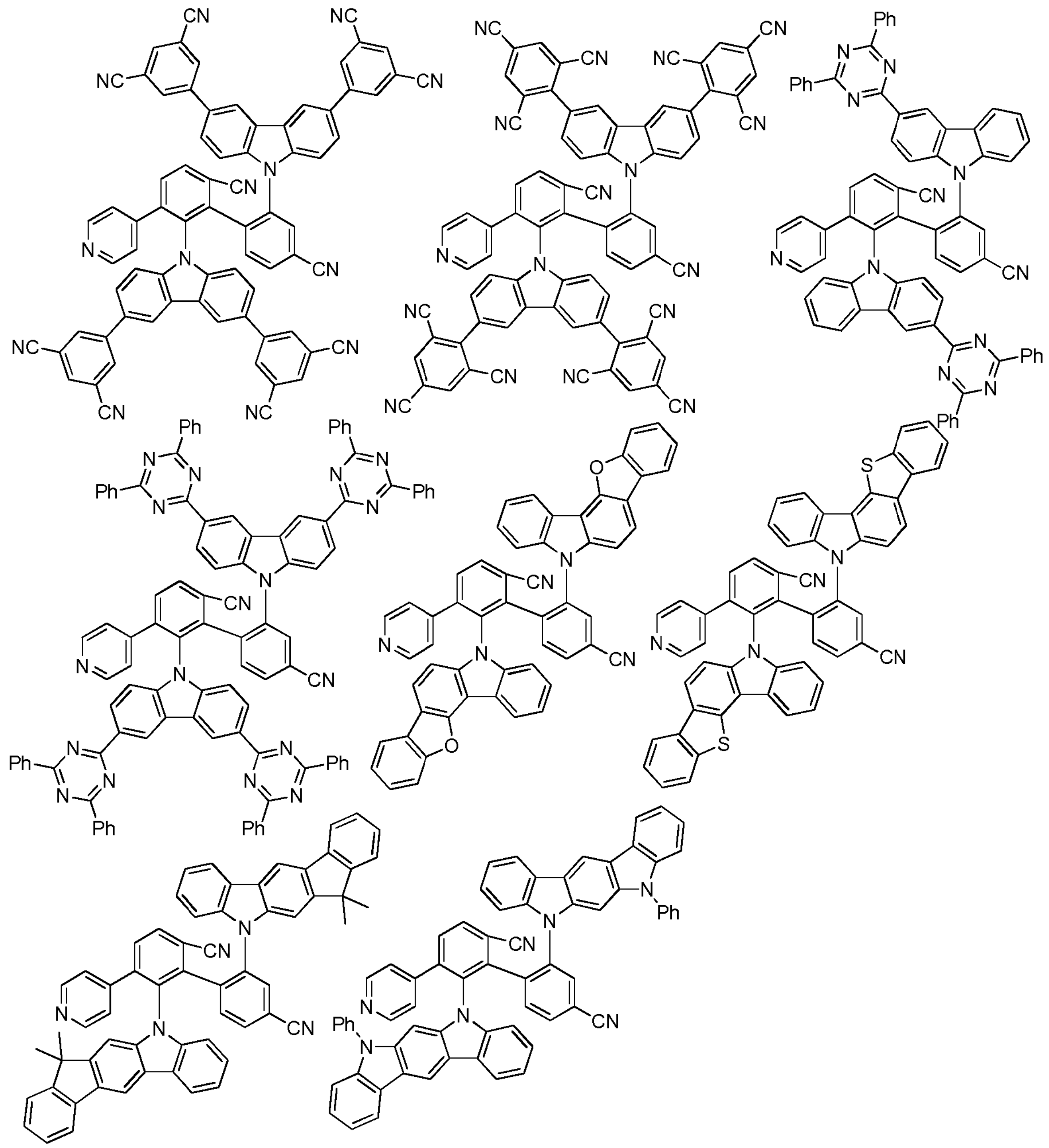

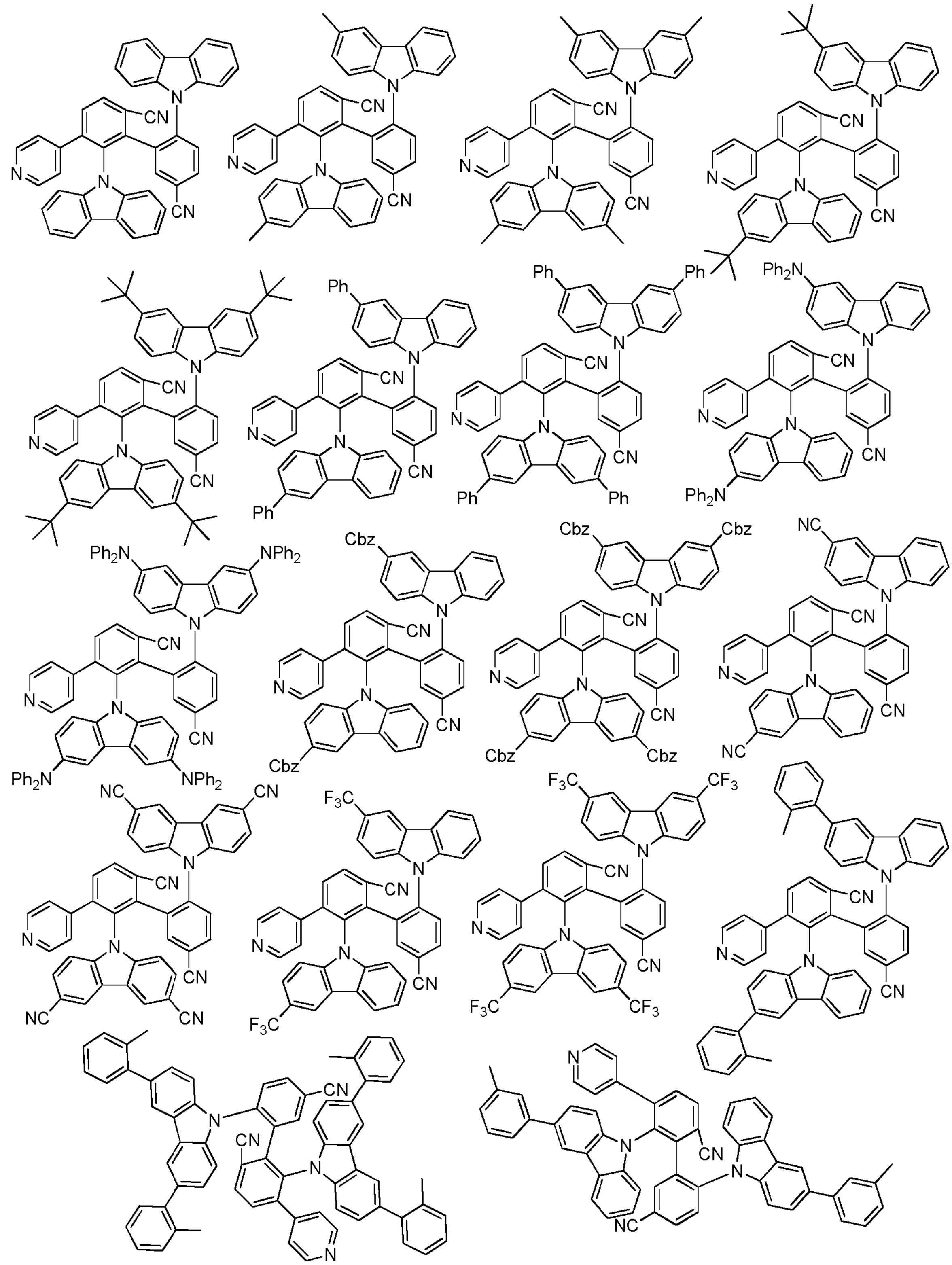

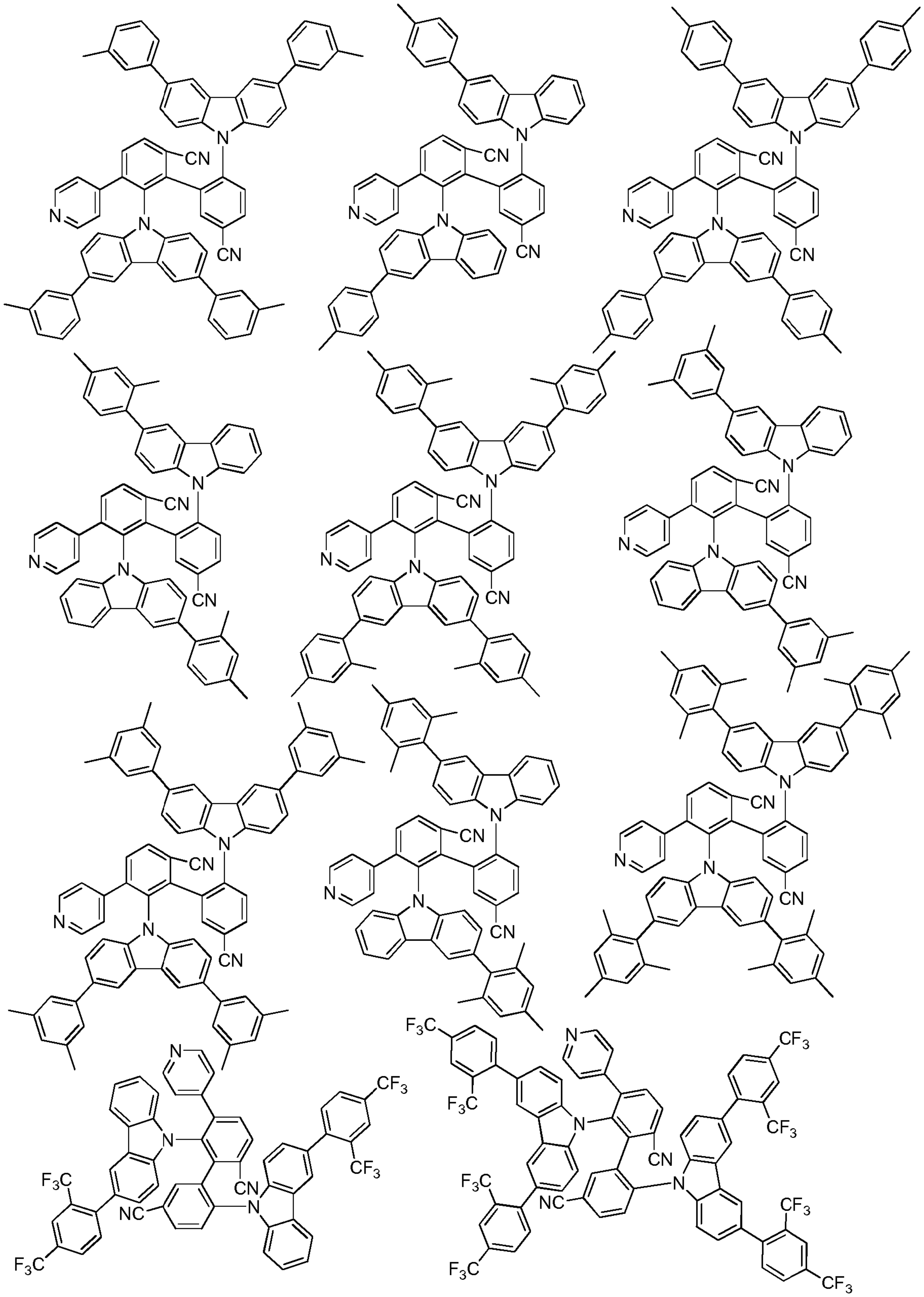

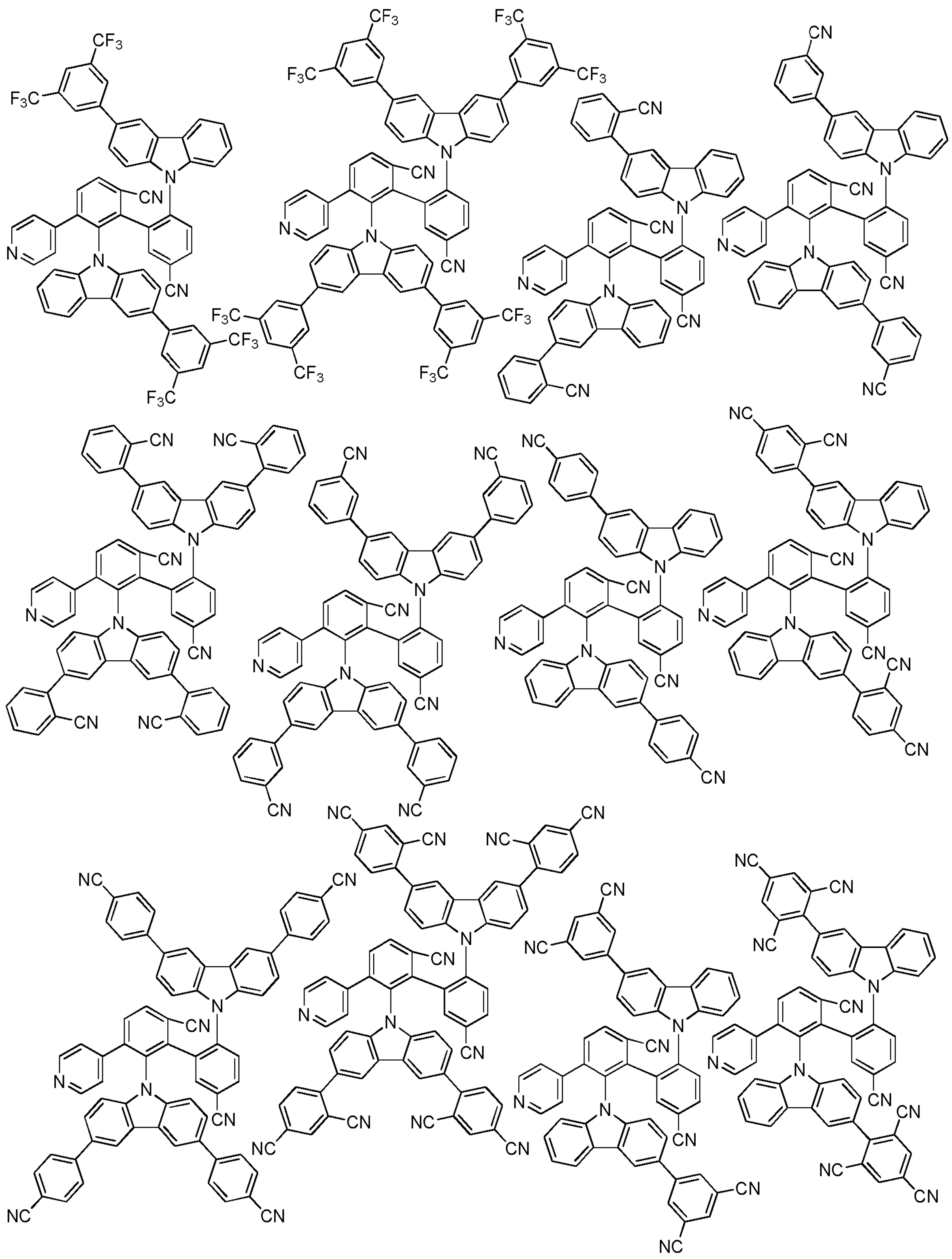

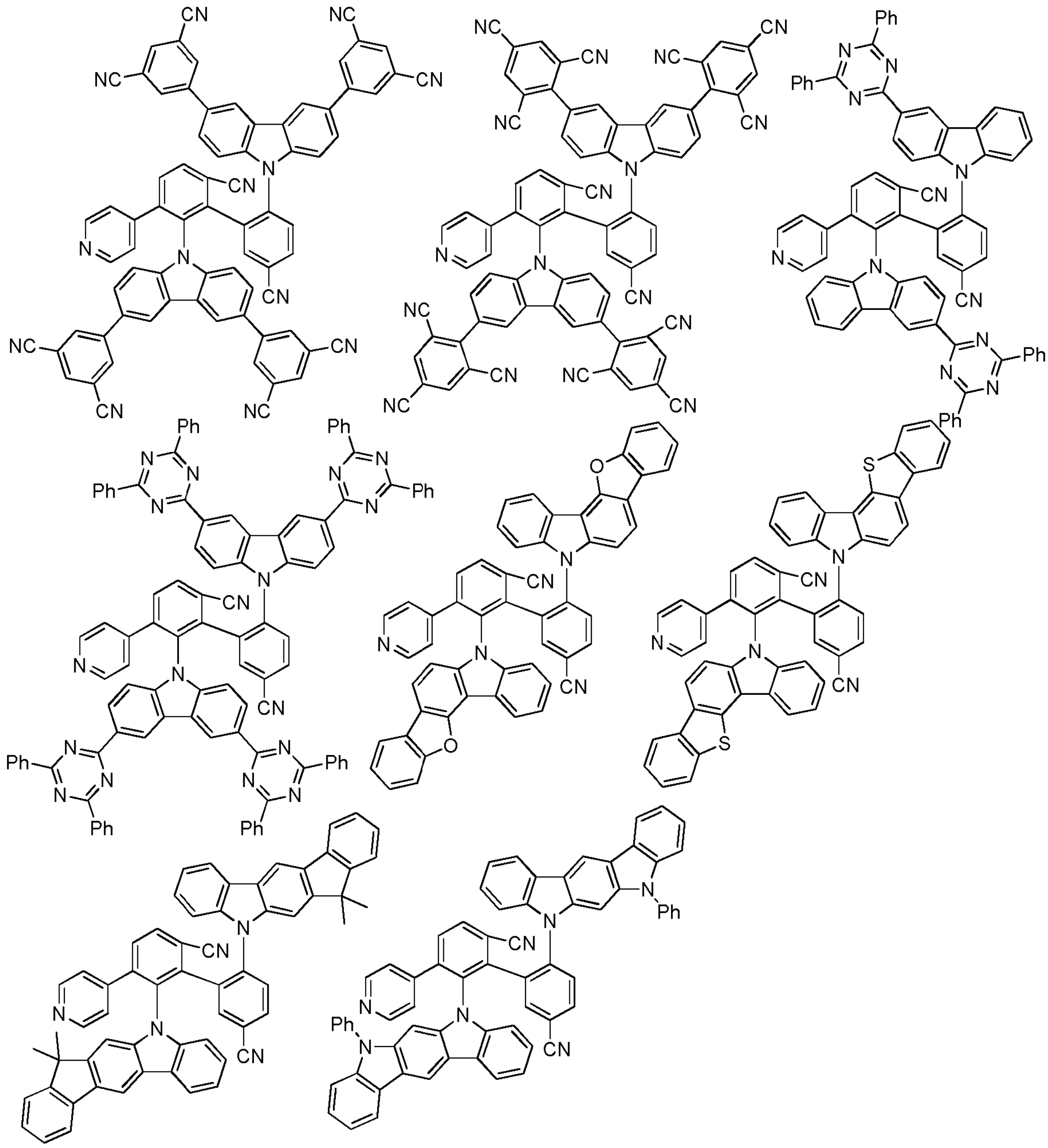

## Figuren

Es zeigen:

Figur 1 Emissionsspektrum von Beispiel **1** (10 % in PMMA).
Figur 2 Emissionsspektrum von Beispiel **2** (10 % in PMMA).
Figur 3 Emissionsspektrum von Beispiel **3** (10 % in PMMA).

## Patentansprüche

**1.** Organisches Molekül, aufweisend eine Struktur gemäß Formel I

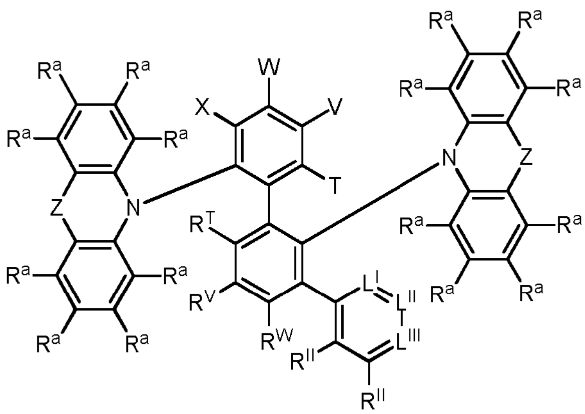

Formel I

wobei gilt:

T ist ausgewählt aus einer Gruppe bestehend aus CN und $R^1$;
V ist ausgewählt aus einer Gruppe bestehend aus CN und $R^1$;
W ist ausgewählt aus einer Gruppe bestehend aus CN und $R^1$;
X ist ausgewählt aus einer Gruppe bestehend aus CN und $R^1$;
$R^T$ ist ausgewählt aus einer Gruppe bestehend aus CN und $R^2$;
$R^V$ ist ausgewählt aus einer Gruppe bestehend aus CN und $R^2$;
$R^W$ ist ausgewählt aus einer Gruppe bestehend aus CN und $R^2$;
L' ist ausgewählt aus einer Gruppe bestehend aus N und $CR^I$;
L" ist ausgewählt aus einer Gruppe bestehend aus N und $CR^I$;
$L^{III}$ ist ausgewählt aus einer Gruppe bestehend aus N und $CR^I$;
Z ist bei jedem Auftreten gleich oder verschieden eine direkte Bindung oder ausgewählt aus der Gruppe bestehend aus $CR^3R^4$, $C=CR^3R^4$, C=O, $C=NR^3$, $NR^3$, O, $SiR^3R^4$, S, S(O) und $S(O)_2$;
$R^1$, $R^2$, $R^I$, $R^{II}$ ist bei jedem Auftreten gleich oder verschieden H, Deuterium, eine lineare Alkylgruppe mit 1 bis 5 C-Atomen, eine lineare Alkenyl- oder Alkinylgruppe mit 2 bis 8 C-Atomen, eine verzweigte oder cyclische Alkyl-, Alkenyl- oder Alkinylgruppe mit 3 bis 10 C-Atomen, wobei ein oder mehrere H-Atome durch Deuterium ersetzt sein können, oder ein aromatisches oder heteroaromatisches Ringsystem mit 5 bis 15 aromatischen Ringatomen, das jeweils durch einen oder mehrere Reste $R^6$ substituiert sein kann;
$R^a$, $R^3$ und $R^4$ ist bei jedem Auftreten gleich oder verschieden ausgewählt aus der Gruppe bestehend aus:

- H, Deuterium, $N(R^5)2$, OH, $Si(R^5)3$, $B(OR^5)2$, $OSO_2R^5$, $CF_3$, CN, F, Br, I;
- einer linearen Alkyl-, Alkoxy- oder Thioalkoxygruppe mit 1 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können;
- einer linearen Alkenyl- oder Alkinylgruppe mit 2 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können;
- einer verzweigten oder cyclischen Alkyl-, Alkenyl-, Alkinyl-, Alkoxy- oder Thioalkoxygruppe mit 3 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können;
- einem aromatischen oder heteroaromatischen Ringsystem mit 5 bis 60 aromatischen Ringatomen, das jeweils mit einem oder mehreren Resten $R^5$ substituiert sein kann;
- einer Aryloxy- oder Heteroaryloxygruppe mit 5 bis 60 aromatischen Ringatomen, die jeweils mit einem oder mehreren Resten $R^5$ substituiert sein kann; und
- eine Diarylaminogruppe, Diheteroarylaminogruppe oder Arylheteroarylaminogruppe mit 10 bis 40 aromatischen Ringatomen, welche jeweils mit einem oder mehreren Resten $R^5$ substituiert sein kann;

$R^5$ ist bei jedem Auftreten gleich oder verschieden ausgewählt aus der Gruppe bestehend aus:

- H, Deuterium, $N(R^6)_2$, OH, $Si(R^6)3$, $B(OR^6)2$, $OSO_2R^6$, $CF_3$, CN, F, Br, I;
- einer linearen Alkyl-, Alkoxy- oder Thioalkoxygruppe mit 1 bis 40 C-Atomen,die jeweils mit einem oder mehreren Resten $R^6$ substituiert sein kann, wobei eine oder mehrere nicht benachbarte $CH_2$-Gruppen durch $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sein können und wobei ein oder mehrere H-Atome durch Deuterium, CN, $CF_3$ oder $NO_2$ ersetzt sein können;
- einer linearen Alkenyl- oder Alkinylgruppe mit 2 bis 40 C-Atomen, die jeweils mit einem oder mehreren Resten $R^6$ substituiert sein kann, wobei eine oder mehrere nicht benachbarte $CH_2$-Gruppen durch $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sein können und wobei ein oder mehrere H-Atome durch Deuterium, CN, $CF_3$ oder $NO_2$ ersetzt sein können;
- einer verzweigten oder cyclischen Alkyl-, Alkenyl-, Alkinyl-, Alkoxy- oder Thioalkoxygruppe mit 3 bis 40 C-Atomen, die jeweils mit einem oder mehreren Resten $R^6$ substituiert sein kann, wobei eine oder mehrere nicht benachbarte $CH_2$-Gruppen durch $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sein können und wobei ein oder mehrere H-Atome durch Deuterium, CN, $CF_3$ oder $NO_2$ ersetzt sein können;
- einem aromatischen oder heteroaromatischen Ringsystem mit 5 bis 60 aromatischen Ringatomen, das jeweils mit einem oder mehreren Resten $R^6$ substituiert sein kann,
- einer Aryloxy- oder Heteroaryloxygruppe mit 5 bis 60 aromatischen Ringatomen, die jeweils mit einem oder mehreren Resten $R^6$ substituiert sein kann; und
- einer Diarylaminogruppe, Diheteroarylaminogruppe oder Arylheteroarylaminogruppe mit 10 bis 40 aromatischen Ringatomen, welche jeweils mit einem oder mehreren Resten $R^6$ substituiert sein kann;

$R^6$ ist bei jedem Auftreten gleich oder verschieden ausgewählt aus der Gruppe bestehend aus:

- H, Deuterium, OH, $CF_3$, CN, F;
- einer linearen Alkyl-, Alkoxy- oder Thioalkoxygruppe mit 1 bis 5 C-Atomen, wobei ein oder mehrere H-Atome durch Deuterium, CN, $CF_3$ oder $NO_2$ ersetzt sein können;
- einer linearen Alkenyl- oder Alkinylgruppe mit 2 bis 5 C-Atomen, wobei ein oder mehrere H-Atome durch Deuterium, CN, $CF_3$ oder $NO_2$ ersetzt sein können;
- einer verzweigten oder cyclischen Alkyl-, Alkenyl-, Alkinyl-, Alkoxy- oder Thioalkoxygruppe mit 3 bis 5 C-Atomen, wobei ein oder mehrere H-Atome durch Deuterium, CN, $CF_3$ oder $NO_2$ ersetzt sein können;
- einem aromatischen oder heteroaromatischen Ringsystem mit 5 bis 60 aromatischen Ringatomen;
- einer Aryloxy- oder Heteroaryloxygruppe mit 5 bis 60 aromatischen Ringatomen; und
- einer Diarylaminogruppe, Diheteroarylaminogruppe oder Arylheteroarylaminogruppe mit 10 bis 40 aromatischen Ringatomen;

wobei jeder der Reste $R^a$, $R^3$, $R^4$ oder $R^5$ auch mit einem oder mehreren weiteren Resten $R^a$, $R^3$, $R^4$ oder $R^5$ ein mono- oder polycyclisches, aliphatisches, aromatisches und/oder benzoanelliertes Ringsystem bilden kann; wobei genau ein Rest ausgewählt aus der Gruppe bestehend aus T, V, W und X gleich CN ist, genau ein Rest ausgewählt aus $R^T$, $R^V$ und $R^W$ gleich CN ist, und
genau ein Rest ausgewählt aus $L^I$, $L^{II}$ und $L^{III}$ gleich N ist.

**2.** Organisches Molekül nach Anspruch 1, wobei $R^1$, $R^2$, $R^I$ und $R^{II}$ bei jedem Auftreten gleich oder verschieden ausgewählt sind aus der Gruppe bestehend aus H, Methyl und Phenyl.

**3.** Organisches Molekül nach mindestens einem der Ansprüche 1 oder 2, wobei $L^{III}$ gleich N ist.

**4.** Organisches Molekül nach mindestens einem der Ansprüche 1 bis 3, wobei das organische Molekül eine Struktur gemäß Formel II aufweist:

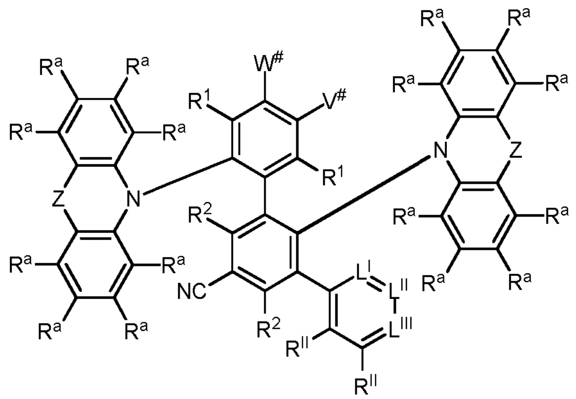

Formel II

wobei $W^\#$ ausgewählt ist aus einer Gruppe bestehend aus CN und $R^1$;
und $V^\#$ ausgewählt ist aus einer Gruppe bestehend aus CN und $R^1$;
wobei genau ein Rest ausgewählt aus $W^\#$ und $V^\#$ gleich CN ist.

**5.** Organisches Molekül nach mindestens einem der Ansprüche 1 bis 4, wobei das organische Molekül eine Struktur gemäß Formel IIa aufweist:

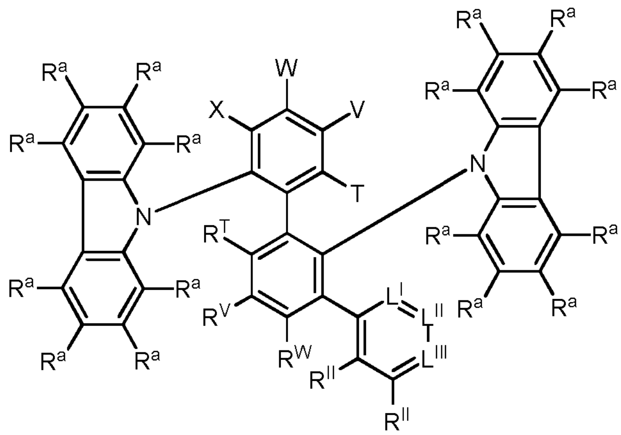

Formel IIa

**6.** Organisches Molekül nach mindestens einem der Ansprüche 1 bis 4, wobei das organische Molekül eine Struktur gemäß Formel IIaa aufweist:

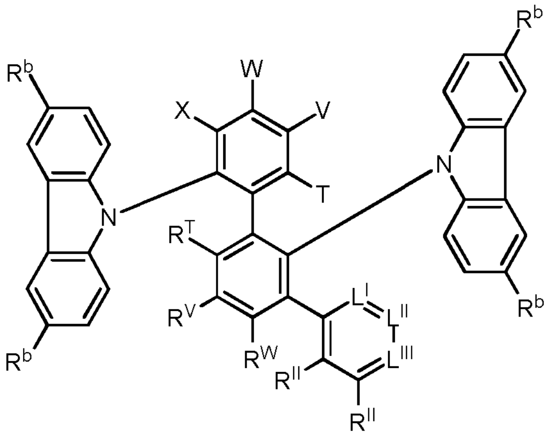

Formel IIaa

wobei
$R^b$ bei jedem Auftreten gleich oder verschieden ausgewählt ist aus der Gruppe bestehend aus:

- $N(R^5)_2$, OH, $Si(R^5)_3$, $B(OR^5)_2$, $OSO_2R^5$, $CF_3$, CN, F, Br, I;
- einer linearen Alkyl-, Alkoxy- oder Thioalkoxygruppe mit 1 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können;
- einer linearen Alkenyl- oder Alkinylgruppe mit 2 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können;
- einer verzweigten oder cyclischen Alkyl-, Alkenyl-, Alkinyl-, Alkoxy- oder Thioalkoxygruppe mit 3 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können;
- einem aromatischen oder heteroaromatischen Ringsystem mit 5 bis 60 aromatischen Ringatomen, das jeweils mit einem oder mehreren Resten $R^5$ substituiert sein kann;
- einer Aryloxy- oder Heteroaryloxygruppe mit 5 bis 60 aromatischen Ringatomen, die jeweils mit einem oder mehreren Resten $R^5$ substituiert sein kann; und
- einer Diarylaminogruppe, Diheteroarylaminogruppe oder Arylheteroarylaminogruppe mit 10 bis 40 aromatischen Ringatomen, welche jeweils mit einem oder mehreren Resten $R^5$ substituiert sein kann;

und im Übrigen die in Anspruch 1 genannten Definitionen gelten.

**7.** Organisches Molekül nach mindestens einem der Ansprüche 1 bis 5, wobei das organische Molekül eine Struktur der Formel IIaaa aufweist:

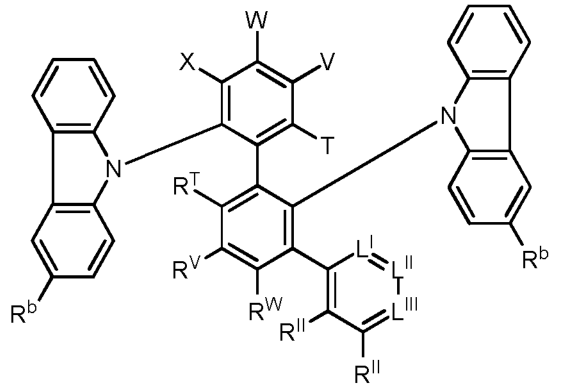

Formel IIaaa

wobei

$R^b$ bei jedem Auftreten gleich oder verschieden ausgewählt ist aus der Gruppe bestehend aus:

- $N(R^5)_2$, OH, $Si(R^5)_3$, $B(OR^5)_2$, $OSO_2R^5$, $CF_3$, CN, F, Br, I;
- einer linearen Alkyl-, Alkoxy- oder Thioalkoxygruppe mit 1 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können;
- einer linearen Alkenyl- oder Alkinylgruppe mit 2 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können;
- einer verzweigten oder cyclischen Alkyl-, Alkenyl-, Alkinyl-, Alkoxy- oder Thioalkoxygruppe mit 3 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können;
- einem aromatischen oder heteroaromatischen Ringsystem mit 5 bis 60 aromatischen Ringatomen, das jeweils mit einem oder mehreren Resten $R^5$ substituiert sein kann;
- einer Aryloxy- oder Heteroaryloxygruppe mit 5 bis 60 aromatischen Ringatomen, die jeweils mit einem oder mehreren Resten $R^5$ substituiert sein kann; und
- einer Diarylaminogruppe, Diheteroarylaminogruppe oder Arylheteroarylaminogruppe mit 10 bis 40 aromatischen Ringatomen, welche jeweils mit einem oder mehreren Resten $R^5$ substituiert sein kann;

und im Übrigen die in Anspruch 1 genannte Definitionen gelten.

**8.** Organisches Molekül nach Anspruch 6 oder 7, wobei $R^b$ bei jedem Auftreten gleich oder verschieden Me, $^i$Pr, $^t$Bu, CN, $CF_3$ oder Ph, das jeweils mit einem oder mehreren Resten ausgewählt aus Me, $^i$Pr, $^t$Bu, CN, $CF_3$ oder Ph substituiert sein kann, Pyridinyl, das jeweils mit einem oder mehreren Resten ausgewählt aus Me, $^i$Pr, $^t$Bu, CN, $CF_3$ oder Ph substituiert sein kann, Pyrimidinyl, das jeweils mit einem oder mehreren Resten ausgewählt aus Me, $^i$Pr, $^t$Bu, CN, $CF_3$ oder Ph substituiert sein kann, Triazinyl, das jeweils mit einem oder mehreren Resten ausgewählt aus Me, $^i$Pr, $^t$Bu, CN, $CF_3$ oder Ph substituiert sein kann, Carbazolyl, das jeweils mit einem oder mehreren Resten ausgewählt aus Me, $^i$Pr, $^t$Bu, CN, $CF_3$ oder Ph substituiert sein kann, oder $N(Ph)_2$ ist.

**9.** Verfahren zur Herstellung eines organischen Moleküls nach Anspruch 1 bis 8, wobei ein mit zwei Resten $R^2$ substituiertes Dichlor-Fluorbenzonitril als Edukt eingesetzt wird, wobei $R^2$ wie in Anspruch 1 definiert ist.

**10.** Verwendung eines organischen Moleküls nach Anspruch 1 bis 8 als lumineszierender Emitter und/oder als Hostmaterial und/oder als Elektronentransportmaterial und/oder als Lochinjektionsmaterial und/oder als Lochblockiermaterial in einer optoelektronischen Vorrichtung.

**11.** Verwendung nach Anspruch 10, wobei die optoelektronische Vorrichtung ausgewählt ist aus der Gruppe bestehend aus:

• organischen lichtemittierenden Dioden (OLEDs),
• lichtemittierenden elektrochemischen Zellen,
• OLED-Sensoren,
• organischen Dioden,
• organischen Solarzellen,
• organischen Transistoren,
• organischen Feldeffekttransistoren,
• organischen Lasern und
• Down-Konversions-Elementen.

**12.** Zusammensetzung, aufweisend:

(a) ein organisches Molekül nach einem der Ansprüche 1 bis 8, insbesondere in Form eines Emitters und/oder Hosts, und
(b) ein Emitter- und/oder Hostmaterial, das von dem organischen Molekül nach Anspruch 1 bis 8 verschiedenen ist und
(c) optional einen Farbstoff und/oder ein Lösungsmittel.

**13.** Optoelektronische Vorrichtung, aufweisend ein organisches Molekül nach Anspruch 1 bis 8 oder eine Zusammensetzung nach Anspruch 12, insbesondere ausgeformt als eine Vorrichtung ausgewählt aus der Gruppe bestehend aus organischer lichtemittierender Diode (OLED), lichtemittierender elektrochemischer Zelle, OLED-Sensor, organischer Diode, organischer Solarzelle, organischem Transistor, organischem Feldeffekttransistor, organischem Laser und Down-Konversions-Element.

**14.** Optoelektronische Vorrichtung nach Anspruch 13, aufweisend

- ein Substrat,
- eine Anode und
- eine Kathode, wobei die Anode oder die Kathode auf das Substrat aufgebracht sind, und
- eine lichtemittierende Schicht, die zwischen der Anode und der Kathode angeordnet ist und die das organische Molekül oder die Zusammensetzung aufweist.

**15.** Verfahren zur Herstellung eines optoelektronischen Bauelements, wobei ein organisches Molekül nach Anspruch 1 bis 8 verwendet wird, insbesondere umfassend die Verarbeitung des organischen Moleküls mittels eines Vakuumverdampfungsverfahrens oder aus einer Lösung.

**Claims**

**1.** Organic molecule comprising a structure according to formula I

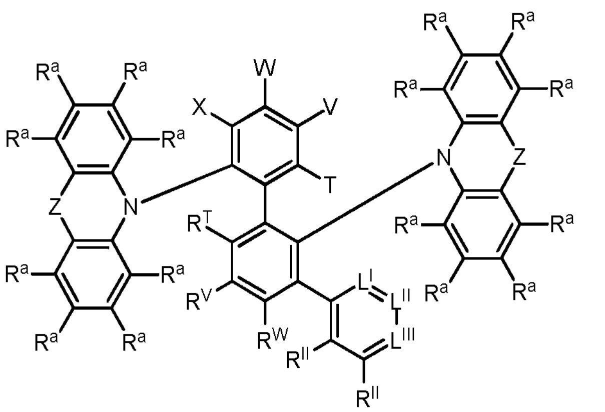

Formula I

wherein:

T is selected from the group consisting of CN and $R^1$;
V is selected from the group consisting of CN and $R^1$;
W is selected from the group consisting of CN and $R^1$;
X is selected from the group consisting of CN and $R^1$;
$R^T$ is selected from the group consisting of CN and $R^2$;
$R^V$ is selected from the group consisting of CN and $R^2$;
$R^W$ is selected from the group consisting of CN and $R^2$;
$L^I$ is selected from the group consisting of N and $CR^I$;
$L^{II}$ is selected from the group consisting of N and $CR^I$;
$L^{III}$ is selected from the group consisting of N and $CR^I$;
Z is at each occurrence the same or different and is a direct bond or selected from the group consisting of $CR^3R^4$, $C=CR^3R^4$, C=O, $C=NR^3$, $NR^3$, O, $SiR^3R^4$, S, S(O) and $S(O)_2$;
$R^1$, $R^2$, $R^I$, $R^{II}$ is at each occurrence the same or different and is H, deuterium, a linear alkyl group having 1 to 5 C atoms, a linear alkenyl or alkynyl group having 2 to 8 C atoms, a branched or cyclic alkyl, alkenyl or alkynyl group having 3 to 10 C atoms, wherein one or more H atoms may be substituted by deuterium, or an aromatic or heteroaromatic ring system having 5 to 15 aromatic ring atoms, each of which may be substituted by one or more moieties $R^6$;
$R^a$, $R^3$ and $R^4$ is at each occurrence the same or different and is selected from the group consisting of:

- H, deuterium, $N(R^5)_2$, OH, $Si(R^5)_3$, $B(OR^5)_2$, $OSO_2R^5$, $CF_3$, CN, F, Br, I;
- a linear alkyl, alkoxy or thioalkoxy group having 1 to 40 C atoms, each of which may be substituted by one or more moieties $R^5$, wherein one or more non-adjacent $CH_2$ groups may be substituted by $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$ and wherein one or more H atoms may be substituted by deuterium, CN, $CF_3$ or $NO_2$;
- a linear alkenyl or alkynyl group having 2 to 40 C atoms, each of which may be substituted by one or more moieties $R^5$, wherein one or more non-adjacent $CH_2$ groups may be substituted by $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$ and wherein one or more H atoms may be substituted by deuterium, CN, $CF_3$ or $NO_2$;
- a branched or cyclic alkyl, alkenyl, alkynyl, alkoxy or thioalkoxy group having 3 to 40 C atoms, each of which may be substituted by one or more moieties $R^5$, wherein one or more non-adjacent $CH_2$ groups may be substituted by $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$ and wherein one or more H atoms may be substituted by deuterium, CN, $CF_3$ or $NO_2$;
- an aromatic or heteroaromatic ring system having 5 to 60 aromatic ring atoms, each of which may be substituted by one or more moieties $R^5$;
- an aryloxy or heteroaryloxy group having 5 to 60 aromatic ring atoms, each of which may be substituted by one or more moieties $R^5$; and
- a diarylamino group, diheteroarylamino group or arylheteroarylamino group having 10 to 40 aromatic ring atoms, each of which may be substituted by one or more moieties $R^5$;

$R^5$ is at each occurrence the same or different and is selected from the group consisting of:

- H, deuterium, $N(R^6)_2$, OH, $Si(R^6)_3$, $B(OR^6)_2$, $OSO_2R^6$, $CF_3$, CN, F, Br, I;
- a linear alkyl, alkoxy or thioalkoxy group having 1 to 40 C atoms, each of which may be substituted by one or more moieties $R^6$, wherein one or more non-adjacent $CH_2$ groups may be substituted by $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$ and wherein one or more H atoms may be substituted by deuterium, CN, $CF_3$ or $NO_2$;
- a linear alkenyl or alkynyl group having 2 to 40 C atoms, each of which may be substituted by one or more moieties $R^6$, wherein one or more non-adjacent $CH_2$ groups may be substituted by $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$ and wherein one or more H atoms may be substituted by deuterium, CN, $CF_3$ or $NO_2$;
- a branched or cyclic alkyl, alkenyl, alkynyl, alkoxy or thioalkoxy group having 3 to 40 C atoms, each of which may be substituted by one or more moieties $R^6$, wherein one or more non-adjacent $CH_2$ groups may be substituted by $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$ and wherein one or more H atoms may be substituted by deuterium, CN, $CF_3$ or $NO_2$;
- an aromatic or heteroaromatic ring system having 5 to 60 aromatic ring atoms, each of which may be substituted by one or more moieties $R^6$;

- an aryloxy or heteroaryloxy group having 5 to 60 aromatic ring atoms, each of which may be substituted by one or more moieties $R^6$; and
- a diarylamino group, diheteroarylamino group or arylheteroarylamino group having 10 to 40 aromatic ring atoms, each of which may be substituted by one or more moieties $R^6$;

$R^6$ is at each occurrence the same or different and is selected from the group consisting of:

- H, deuterium, OH, $CF_3$, CN, F;
- a linear alkyl, alkoxy or thioalkoxy group having 1 to 5 C atoms, wherein one or more H atoms may be substituted by deuterium, CN, $CF_3$ or $NO_2$;
- a linear alkenyl or alkynyl group having 2 to 5 C atoms, wherein one or more H atoms may be substituted by deuterium, CN, $CF_3$ or $NO_2$;
- a branched or cyclic alkyl, alkenyl, alkynyl, alkoxy or thioalkoxy group having 3 to 5 C atoms, wherein one or more H atoms may be substituted by deuterium, CN, $CF_3$ or $NO_2$;
- an aromatic or heteroaromatic ring system having 5 to 60 aromatic ring atoms;
- an aryloxy or heteroaryloxy group having 5 to 60 aromatic ring atoms; and
- a diarylamino group, diheteroarylamino group or arylheteroarylamino group having 10 to 40 aromatic ring atoms;

wherein each of the moieties $R^a$, $R^3$, $R^4$ or $R^5$ may also form a mono- or polycyclic, aliphatic, aromatic and/or benzo-fused ring system with one or more further moieties $R^a$, $R^3$, $R^4$ or $R^5$; and
wherein exactly one moiety selected from the group consisting of T, V, W and X is CN,
exactly one moiety selected from $R^T$, $R^V$ and $R^W$ is CN, and
exactly one moiety selected from $L^I$, $L^{II}$ and $L^{III}$ is N.

2. Organic molecule according to claim 1, wherein $R^1$, $R^2$, $R^I$ and $R^{II}$ is at each occurrence the same or different and is selected from the group consisting of H, Methyl and Phenyl.

3. Organic molecule according to at least one of claims 1 or 2, wherein $L^{III}$ is N.

4. Organic molecule according to at least one of claims 1 to 3, wherein the organic molecule comprises a structure according to formula II:

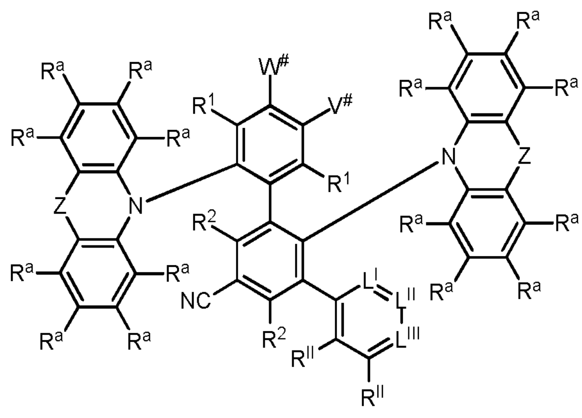

Formula II

wherein $W^\#$ is selected from the group consisting of CN and $R^1$;
and $V^\#$ is selected from the group consisting of CN and $R^1$;
wherein exactly one moiety selected from $W^\#$ and $V^\#$ is CN.

5. Organic molecule according to at least one of claims 1 to 4, wherein the organic molecule comprises a structure according to formula IIa:

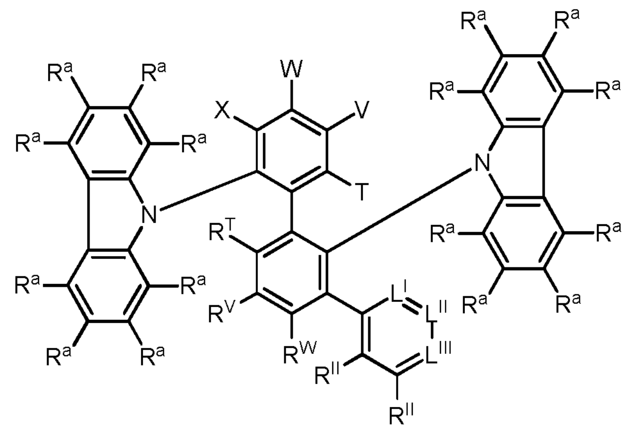

Formula IIa

**6.** Organic molecule according to at least one of claims 1 to 4, wherein the organic molecule comprises a structure according to formula IIaa:

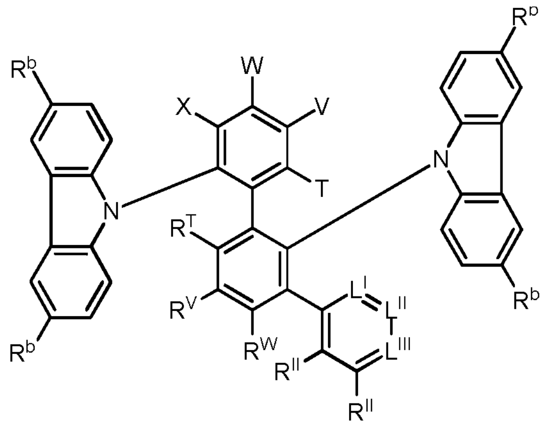

Formula IIaa

wherein
$R^b$ is at each occurrence the same or different and is selected from the group consisting of:

- $N(R^5)_2$, OH, $Si(R^5)_3$, $B(OR^5)_2$, $OSO_2R^5$, $CF_3$, CN, F, Br, I;
- a linear alkyl, alkoxy or thioalkoxy group having 1 to 40 C atoms, each of which may be substituted by one or more moieties $R^5$, wherein one or more non-adjacent $CH_2$ groups may be substituted by $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$ and wherein one or more H atoms may be substituted by deuterium, CN, $CF_3$ or $NO_2$;
- a linear alkenyl or alkynyl group having 2 to 40 C atoms, each of which may be substituted by one or more moieties $R^5$, wherein one or more non-adjacent $CH_2$ groups may be substituted by $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$ and wherein one or more H atoms may be substituted by deuterium, CN, $CF_3$ or $NO_2$;
- a branched or cyclic alkyl, alkenyl, alkynyl, alkoxy or thioalkoxy group having 3 to 40 C atoms, each of which may be substituted by one or more moieties $R^5$, wherein one or more non-adjacent $CH_2$ groups may be substituted by $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$ and wherein one or more H atoms may be substituted by deuterium, CN, $CF_3$ or $NO_2$;
- an aromatic or heteroaromatic ring system having 5 to 60 aromatic ring atoms, each of which may be substituted by one or more moieties $R^5$;
- an aryloxy or heteroaryloxy group having 5 to 60 aromatic ring atoms, each of which may be substituted by one or more moieties $R^5$; and
- a diarylamino group, diheteroarylamino group or arylheteroarylamino group having 10 to 40 aromatic ring atoms, each of which may be substituted by one or more moieties $R^5$;

and apart from that the definitions of claim 1 apply.

7. Organic molecule according to at least one of claims 1 to 5, wherein the organic molecule comprises a structure according to formula IIaaa:

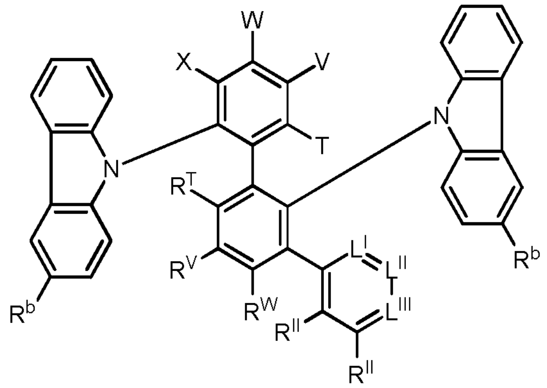

Formula IIaaa

wherein
$R^b$ is at each occurrence the same or different and is selected from the group consisting of:

- $N(R^5)_2$, OH, $Si(R^5)_3$, $B(OR^5)_2$, $OSO_2R^5$, $CF_3$, CN, F, Br, I;
- a linear alkyl, alkoxy or thioalkoxy group having 1 to 40 C atoms, each of which may be substituted by one or more moieties $R^5$, wherein one or more non-adjacent $CH_2$ groups may be substituted by $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$ and wherein one or more H atoms may be substituted by deuterium, CN, $CF_3$ or $NO_2$;
- a linear alkenyl or alkynyl group having 2 to 40 C atoms, each of which may be substituted by one or more moieties $R^5$, wherein one or more non-adjacent $CH_2$ groups may be substituted by $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$ and wherein one or more H atoms may be substituted by deuterium, CN, $CF_3$ or $NO_2$;
- a branched or cyclic alkyl, alkenyl, alkynyl, alkoxy or thioalkoxy group having 3 to 40 C atoms, each of which may be substituted by one or more moieties $R^5$, wherein one or more non-adjacent $CH_2$ groups may be substituted by $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$ and wherein one or more H atoms may be substituted by deuterium, CN, $CF_3$ or $NO_2$;
- an aromatic or heteroaromatic ring system having 5 to 60 aromatic ring atoms, each of which may be substituted by one or more moieties $R^5$;
- an aryloxy or heteroaryloxy group having 5 to 60 aromatic ring atoms, each of which may be substituted by one or more moieties $R^5$; and
- a diarylamino group, diheteroarylamino group or arylheteroarylamino group having 10 to 40 aromatic ring atoms, each of which may be substituted by one or more moieties $R^5$;

and apart from that the definitions of claim 1 apply.

8. Organic molecule according to claim 6 or 7, wherein $R^b$ is at each occurrence the same or different and is Me, $^i$Pr, $^t$Bu, CN, $CF_3$ or Ph, each of which may be substituted with one or more moieties selected from Me, $^i$Pr, $^t$Bu, CN, $CF_3$ or Ph, pyridinyl, each which of may be substituted with one or more moieties selected from Me, $^i$Pr, $^t$Bu, CN, $CF_3$ or Ph, pyrimidinyl, each which of may be substituted with one or more moieties selected from Me, $^i$Pr, $^t$Bu, CN, $CF_3$ or Ph, triazinyl, each which of may be substituted with one or more moieties selected from Me, $^i$Pr, $^t$Bu, CN, $CF_3$ or Ph, carbazolyl, each which of may be substituted with one or more moieties selected from Me, $^i$Pr, $^t$Bu, CN, $CF_3$ or Ph, or is $N(Ph)_2$.

9. Method for preparing an organic molecule according to any of claims 1 to 8, wherein a dichloro-fluoro-benzonitrile substituted with two moieties $R^2$ is used as an educt, wherein $R^2$ is as defined in claim 1.

10. Use of an organic molecule according to claims 1 to 8 as a luminescent emitter and/or as a host material and/or as an electron transport material and/or as a hole injection material and/or as a hole blocking material in an optoelectronic

device.

**11.** Use according to claim 10, wherein the optoelectronic device is selected from the group consisting of:

- organic light-emitting diodes (OLEDs),
- light-emitting electrochemical cells,
- OLED-sensors,
- organic diodes,
- organic solar cells,
- organic transistors,
- organic field-effect transistors,
- organic lasers, and
- down-conversion elements.

**12.** Composition, comprising:

(a) an organic molecule according to any one of claims 1 to 8, in particular in the form of an emitter and/or a host, and
(b) an emitter and/or host material, which differs from the organic molecule according to any one of claims 1 to 8, and
(c) optionally, a dye and/or a solvent.

**13.** Optoelectronic device, comprising an organic molecule according to claims 1 to 8 or a composition according to claim 12, in particular in form of a device selected from the group consisting of organic light-emitting diode (OLED), light-emitting electrochemical cell, OLED-sensor, organic diode, organic solar cell, organic transistor, organic field-effect transistor, organic laser, and down-conversion element.

**14.** Organic optoelectronic device according to claim 13, comprising

- a substrate,
- an anode, and
- a cathode, wherein the anode or the cathode are disposed on the substrate, and
- a light-emitting layer, which is arranged between the anode and the cathode and which comprises the organic molecule or the composition.

**15.** Method for producing an optoelectronic device, wherein an organic molecule according to claims 1 to 8 is used, in particular comprising the processing of the organic molecule by vacuum evaporation method or from a solution.

## Revendications

**1.** Molécule organique, comprenant une structure selon la formule I

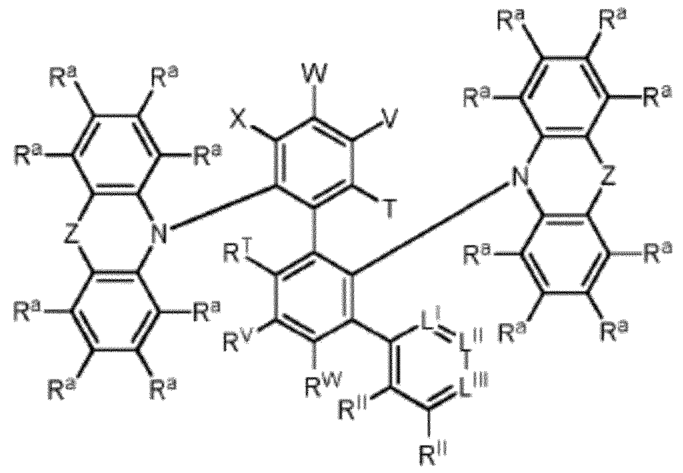

formule I

T étant choisi dans un groupe constitué par CN et $R^1$ ;
V étant choisi dans un groupe constitué par CN et $R^1$ ;
W étant choisi dans un groupe constitué par CN et $R^1$ ;
X étant choisi dans un groupe constitué par CN et $R^1$ ;
$R^T$ étant choisi dans un groupe constitué par CN et $R^2$ ;
$R^V$ étant choisi dans un groupe constitué par CN et $R^2$ ;
$R^W$ étant choisi dans un groupe constitué par CN et $R^2$ ;
$L^I$ étant choisi dans un groupe constitué par N et $CR^I$ ;
$L^{II}$ étant choisi dans un groupe constitué par N et $CR^I$ ;
$L^{III}$ étant choisi dans un groupe constitué par N et $CR^I$ ;
Z, identique ou différent en chaque occurrence, étant une liaison directe ou étant choisi dans le groupe constitué par $CR^3R^4$, $C=CR^3R^4$, C=O, $C=NR^3$, $NR^3$, O, $SiR^3R^4$, S, S(O) et $S(O)_2$ ;
$R^1$, $R^2$, $R^I$, $R^{II}$, identiques ou différents en chaque occurrence, étant H, deutérium, un groupe alkyle linéaire comportant 1 à 5 atomes de C, un groupe alcényle ou alcynyle linéaire comportant 2 à 8 atomes de C, un groupe alkyle, alcényle ou alcynyle ramifié ou cyclique comportant 3 à 10 atomes de C, un ou plusieurs atomes de H pouvant être remplacés par deutérium, ou un système cyclique aromatique ou hétéroaromatique comportant 5 à 15 atomes de cycle aromatique, qui peut à chaque fois être substitué par un ou plusieurs radicaux $R^6$ ;
$R^a$, $R^3$ et $R^4$, en chaque occurrence identiques ou différents, étant choisis dans le groupe constitué par :

- H, deutérium, $N(R^5)_2$, OH, $Si(R^5)_3$, $B(OR^5)_2$, $OSO_2R^5$, $CF_3$, CN, F, Br, I ;
- un groupe alkyle, alcoxy ou thioalcoxy linéaire comportant 1 à 40 atomes de C, qui peut à chaque fois être substitué par un ou plusieurs radicaux $R^5$, un ou plusieurs groupes $CH_2$ non adjacents pouvant être remplacés par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ et un ou plusieurs atomes de H pouvant être remplacés par deutérium, CN, $CF_3$ ou $NO_2$ ;
- un groupe alcényle ou alcynyle linéaire comportant 2 à 40 atomes de C, qui peut à chaque fois être substitué par un ou plusieurs radicaux $R^5$, un ou plusieurs groupes $CH_2$ non adjacents pouvant être remplacés par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ et un ou plusieurs atomes de H pouvant être remplacés par deutérium, CN, $CF_3$ ou $NO_2$ ;
- un groupe alkyle, alcényle, alcynyle, alcoxy ou thioalcoxy ramifié ou cyclique comportant 3 à 40 atomes de C, qui peut à chaque fois être substitué par un ou plusieurs radicaux $R^5$, un ou plusieurs groupes $CH_2$ non adjacents pouvant être remplacés par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ et un ou plusieurs atomes de H pouvant être remplacés par deutérium, CN, $CF_3$ ou $NO_2$;
- un système cyclique aromatique ou hétéroaromatique comportant 5 à 60 atomes de cycle aromatique, qui peut à chaque fois être substitué par un ou plusieurs radicaux $R^5$;
- un groupe aryloxy ou hétéroaryloxy comportant 5 à 60 atomes de cycle aromatique, qui peut à chaque fois être substitué par un ou plusieurs radicaux $R^5$ ; et
- un groupe diarylamino, un groupe dihétéroarylamino ou un groupe arylhétéroarylamino comportant 10 à

40 atomes de cycle aromatique, qui peut à chaque fois être substitué par un ou plusieurs radicaux $R^5$ ;

$R^5$, en chaque occurrence identique ou différent, étant choisi dans le groupe constitué par :

- H, deutérium, $N(R^6)_2$, OH, $Si(R^6)_3$, $B(OR^6)_2$, $OSO_2R^6$, $CF_3$, CN, F, Br, I ;
- un groupe alkyle, alcoxy ou thioalcoxy linéaire comportant 1 à 40 atomes de C, qui peut à chaque fois être substitué par un ou plusieurs radicaux $R^6$, un ou plusieurs groupes $CH_2$ non adjacents pouvant être remplacés par $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S ou $CONR^6$ et un ou plusieurs atomes de H pouvant être remplacés par deutérium, CN, $CF_3$ ou $NO_2$ ;
- un groupe alcényle ou alcynyle linéaire comportant 2 à 40 atomes de C, qui peut à chaque fois être substitué par un ou plusieurs radicaux $R^6$, un ou plusieurs groupes $CH_2$ non adjacents pouvant être remplacés par $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S ou $CONR^6$ et un ou plusieurs atomes de H pouvant être remplacés par deutérium, CN, $CF_3$ ou $NO_2$ ;
- un groupe alkyle, alcényle, alcynyle, alcoxy ou thioalcoxy ramifié ou cyclique comportant 3 à 40 atomes de C, qui peut à chaque fois être substitué par un ou plusieurs radicaux $R^6$, un ou plusieurs groupes $CH_2$ non adjacents pouvant être remplacés par $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S ou $CONR^6$ et un ou plusieurs atomes de H pouvant être remplacés par deutérium, CN, $CF_3$ ou $NO_2$ ;
- un système cyclique aromatique ou hétéroaromatique comportant 5 à 60 atomes de cycle aromatique, qui peut à chaque fois être substitué par un ou plusieurs radicaux $R^6$;
- un groupe aryloxy ou hétéroaryloxy comportant 5 à 60 atomes de cycle aromatique, qui peut à chaque fois être substitué par un ou plusieurs radicaux $R^6$ ; et
- un groupe diarylamino, un groupe dihétéroarylamino ou un groupe arylhétéroarylamino comportant 10 à 40 atomes de cycle aromatique, qui peut à chaque fois être substitué par un ou plusieurs radicaux $R^6$ ;

$R^6$, en chaque occurrence identique ou différent, étant choisi dans le groupe constitué par :

- H, deutérium, OH, $CF_3$, CN, F ;
- un groupe alkyle, alcoxy ou thioalcoxy linéaire comportant 1 à 5 atomes de C, un ou plusieurs atomes de H pouvant être remplacés par deutérium, CN, $CF_3$ ou $NO_2$ ;
- un groupe alcényle ou alcynyle linéaire comportant 2 à 5 atomes de C, un ou plusieurs atomes de H pouvant être remplacés par deutérium, CN, $CF_3$ ou $NO_2$ ;
- un groupe alkyle, alcényle, alcynyle, alcoxy ou thioalcoxy ramifié ou cyclique comportant 3 à 5 atomes de C, un ou plusieurs atomes de H pouvant être remplacés par deutérium, CN, $CF_3$ ou $NO_2$ ;
- un système cyclique aromatique ou hétéroaromatique comportant 5 à 60 atomes de cycle aromatique ;
- un groupe aryloxy ou hétéroaryloxy comportant 5 à 60 atomes de cycle aromatique ; et
- un groupe diarylamino, un groupe dihétéroarylamino ou un groupe arylhétéroarylamino comportant 10 à 40 atomes de cycle aromatique ;

chacun des radicaux $R^a$, $R^3$, $R^4$ ou $R^5$ pouvant former également avec un ou plusieurs autres radicaux $R^a$, $R^3$, $R^4$ ou $R^5$ un système cyclique monocyclique ou polycyclique, aliphatique, aromatique et/ou benzo-annelé ;
exactement un radical choisi dans le groupe constitué par T, V, W et X étant égal à CN, exactement un radical choisi parmi $R^T$, $R^V$ et $R^W$ étant égal à CN et
exactement un radical choisi parmi $L^I$, $L^{II}$ et $L^{III}$ étant égal à N.

2. Molécule organique selon la revendication 1, $R^1$, $R^2$, $R^I$ et $R^{II}$, identiques ou différents en chaque occurrence, étant choisis dans le groupe constitué par H, méthyle et phényle.

3. Molécule organique selon au moins l'une des revendications 1 et 2, $L^{III}$ étant égal à N.

4. Molécule organique selon au moins l'une des revendications 1 à 3, la molécule organique comprenant une structure selon la formule II :

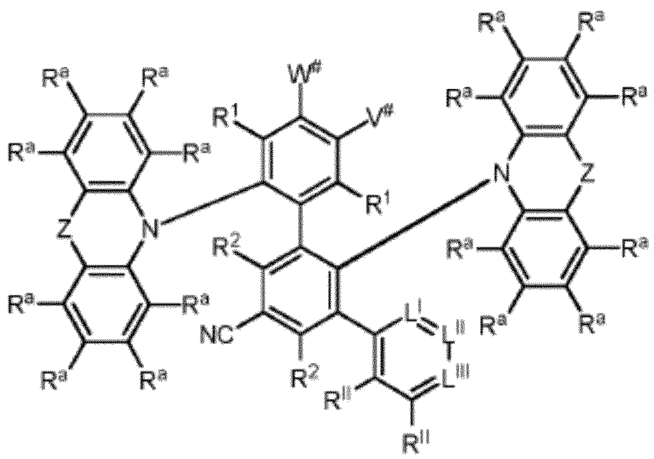

formule II

W# étant choisi dans un groupe constitué par CN et $R^1$ ;
et V# étant choisi dans un groupe constitué par CN et $R^1$ ;
exactement un radical choisi parmi W# et V# étant égal à CN.

5. Molécule organique selon au moins l'une des revendications 1 à 4, la molécule organique comprenant une structure selon la formule IIa :

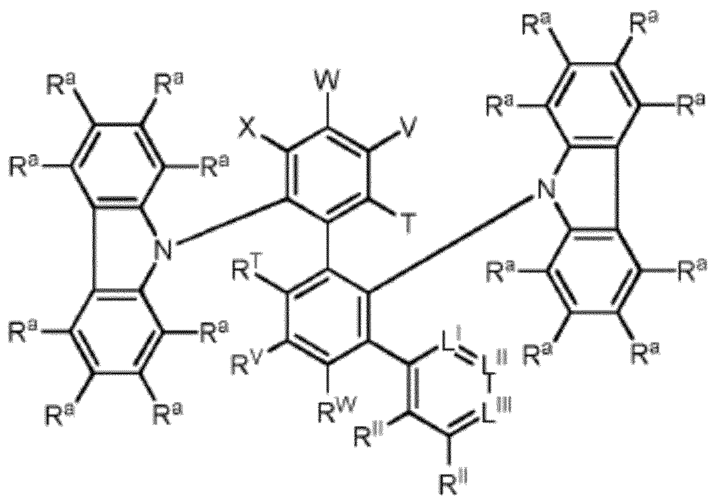

formule IIa.

6. Molécule organique selon au moins l'une des revendications 1 à 4, la molécule organique comprenant une structure selon la formule IIaa :

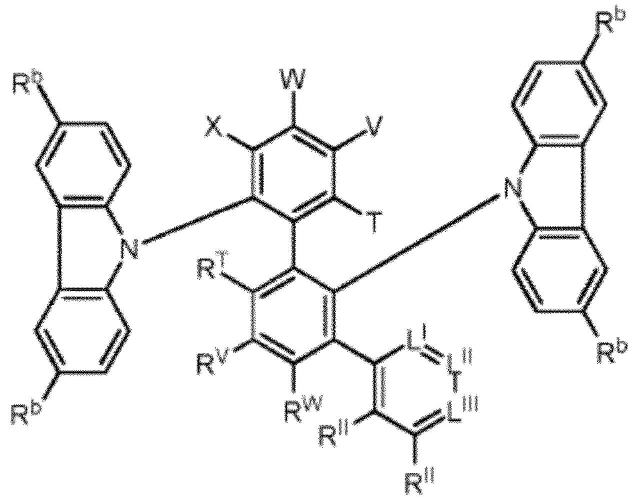

formule IIaa

$R^b$, en chaque occurrence identique ou différent, étant choisi dans le groupe constitué par :

- $N(R^5)_2$, OH, $Si(R^5)_3$, $B(OR^5)_2$, $OSO_2R^5$, $CF_3$, CN, F, Br, I ;
- un groupe alkyle, alcoxy ou thioalcoxy linéaire comportant 1 à 40 atomes de C, qui peut à chaque fois être substitué par un ou plusieurs radicaux $R^5$, un ou plusieurs groupes $CH_2$ non adjacents pouvant être remplacés par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ et un ou plusieurs atomes de H pouvant être remplacés par deutérium, CN, $CF_3$ ou $NO_2$ ;
- un groupe alcényle ou alcynyle linéaire comportant 2 à 40 atomes de C, qui peut à chaque fois être substitué par un ou plusieurs radicaux $R^5$, un ou plusieurs groupes $CH_2$ non adjacents pouvant être remplacés par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ et un ou plusieurs atomes de H pouvant être remplacés par deutérium, CN, $CF_3$ ou $NO_2$ ;
- un groupe alkyle, alcényle, alcynyle, alcoxy ou thioalcoxy ramifié ou cyclique comportant 3 à 40 atomes de C, qui peut à chaque fois être substitué par un ou plusieurs radicaux $R^5$, un ou plusieurs groupes $CH_2$ non adjacents pouvant être remplacés par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ et un ou plusieurs atomes de H pouvant être remplacés par deutérium, CN, $CF_3$ ou $NO_2$ ;
- un système cyclique aromatique ou hétéroaromatique comportant 5 à 60 atomes de cycle aromatique, qui peut à chaque fois être substitué par un ou plusieurs radicaux $R^5$;
- un groupe aryloxy ou hétéroaryloxy comportant 5 à 60 atomes de cycle aromatique, qui peut à chaque fois être substitué par un ou plusieurs radicaux $R^5$ ; et
- un groupe diarylamino, un groupe dihétéroarylamino ou un groupe arylhétéroarylamino comportant 10 à 40 atomes de cycle aromatique, qui peut à chaque fois être substitué par un ou plusieurs radicaux $R^5$ ;

et les définitions mentionnées dans la revendication 1 s'appliquant pour le reste.

**7.** Molécule organique selon au moins l'une des revendications 1 à 5, la molécule organique comprenant une structure selon la formule IIaaa :

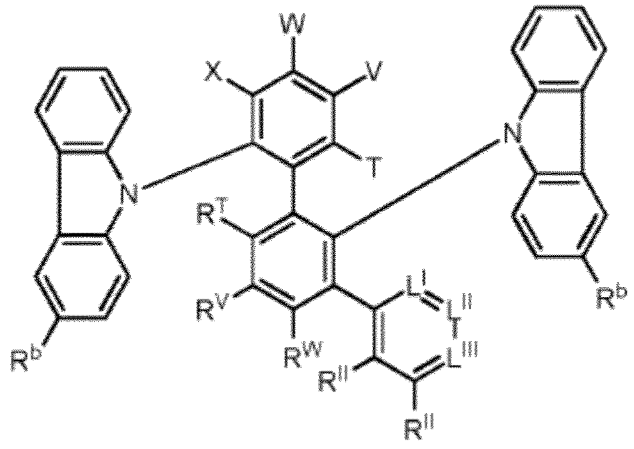

formule IIaaa

$R^b$, en chaque occurrence identique ou différent, étant choisi dans le groupe constitué par :

- $N(R^5)_2$, OH, $Si(R^5)_3$, $B(OR^5)_2$, $OSO_2R^5$, $CF_3$, CN, F, Br, I ;
- un groupe alkyle, alcoxy ou thioalcoxy linéaire comportant 1 à 40 atomes de C, qui peut à chaque fois être substitué par un ou plusieurs radicaux $R^5$, un ou plusieurs groupes $CH_2$ non adjacents pouvant être remplacés par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ et un ou plusieurs atomes de H pouvant être remplacés par deutérium, CN, $CF_3$ ou $NO_2$ ;
- un groupe alcényle ou alcynyle linéaire comportant 2 à 40 atomes de C, qui peut à chaque fois être substitué par un ou plusieurs radicaux $R^5$, un ou plusieurs groupes $CH_2$ non adjacents pouvant être remplacés par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ et un ou plusieurs atomes de H pouvant être remplacés par deutérium, CN, $CF_3$ ou $NO_2$ ;
- un groupe alkyle, alcényle, alcynyle, alcoxy ou thioalcoxy ramifié ou cyclique comportant 3 à 40 atomes de C, qui peut à chaque fois être substitué par un ou plusieurs radicaux $R^5$, un ou plusieurs groupes $CH_2$ non adjacents pouvant être remplacés par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ et un ou plusieurs atomes de H pouvant être remplacés par deutérium, CN, $CF_3$ ou $NO_2$ ;
- un système cyclique aromatique ou hétéroaromatique comportant 5 à 60 atomes de cycle aromatique, qui peut à chaque fois être substitué par un ou plusieurs radicaux $R^5$;
- un groupe aryloxy ou hétéroaryloxy comportant 5 à 60 atomes de cycle aromatique, qui peut à chaque fois être substitué par un ou plusieurs radicaux $R^5$ ; et
- un groupe diarylamino, un groupe dihétéroarylamino ou un groupe arylhétéroarylamino comportant 10 à 40 atomes de cycle aromatique, qui peut à chaque fois être substitué par un ou plusieurs radicaux $R^5$ ;

et les définitions mentionnées dans la revendication 1 s'appliquant pour le reste.

**8.** Molécule organique selon la revendication 6 ou 7, $R^b$, identique ou différent en chaque occurrence, étant Me, $^i$Pr, $^t$Bu, CN, $CF_3$ ou Ph, qui peut à chaque fois être substitué par un ou plusieurs radicaux choisis parmi Me, $^i$Pr, $^t$Bu, CN, $CF_3$ et Ph, pyridinyle, qui peut à chaque fois être substitué par un ou plusieurs radicaux choisis parmi Me, $^i$Pr, $^t$Bu, CN, $CF_3$ et Ph, pyrimidinyle, qui peut à chaque fois être substitué par un ou plusieurs radicaux choisis parmi Me, $^i$Pr, $^t$Bu, CN, $CF_3$ et Ph, triazinyle, qui peut à chaque fois être substitué par un ou plusieurs radicaux choisis parmi Me, $^i$Pr, $^t$Bu, CN, $CF_3$ et Ph, carbazolyle, qui peut à chaque fois être substitué par un ou plusieurs radicaux choisis parmi Me, $^i$Pr, $^t$Bu, CN, $CF_3$ et Ph, ou $N(Ph)_2$.

**9.** Procédé pour la préparation d'une molécule organique selon les revendications 1 à 8, un dichloro-fluorobenzonitrile substitué par deux radicaux $R^2$ étant utilisé en tant qu'éduit, $R^2$ étant tel que défini dans la revendication 1.

**10.** Utilisation d'une molécule organique selon les revendications 1 à 8 en tant qu'émetteur luminescent et/ou en tant que matériau hôte et/ou en tant que matériau de transport d'électrons et/ou en tant que matériau d'injection de trous et/ou en tant que matériau de blocage de trous dans un dispositif optoélectronique.

**11.** Utilisation selon la revendication 10, le dispositif optoélectronique étant choisi dans le groupe constitué par :

- des diodes organiques luminescentes (OLED),
- des cellules électrochimiques luminescentes,
- des capteurs OLED,
- des diodes organiques,
- des cellules solaires organiques,
- des transistors organiques,
- des transistors organiques à effet de champ,
- des lasers organiques et
- des éléments de conversion par abaissement.

**12.** Composition, comprenant:

(a) une molécule organique selon l'une quelconque des revendications 1 à 8, en particulier sous forme d'un émetteur et/ou d'un hôte, et
(b) un matériau émetteur et/ou un matériau hôte, qui sont différents de la molécule selon les revendications 1 à 8 et
(c) éventuellement un colorant et/ou un solvant.

**13.** Dispositif optoélectronique, comprenant une molécule organique selon les revendications 1 à 8 ou une composition selon la revendication 12, en particulier sous la forme d'un dispositif choisi dans le groupe constitué par une diode luminescente organique (OLED), une cellule électrochimique luminescente, un capteur OLED, une diode organique, une cellule solaire organique, un transistor organique, un transistor organique à effet de champ, un laser organique et un élément de conversion par abaissement.

**14.** Dispositif optoélectronique selon la revendication 13, présentant

- un substrat,
- une anode et
- une cathode, l'anode ou la cathode étant déposée sur le substrat, et
- une couche luminescente, qui est disposée entre l'anode et la cathode et qui présente la molécule organique ou la composition.

**15.** Procédé pour la fabrication d'un composant optoélectronique, une molécule organique selon les revendications 1 à 8 étant utilisée, en particulier comprenant le traitement de la molécule organique au moyen d'un procédé d'évaporation sous vide ou à partir d'une solution.

**Figur 1**

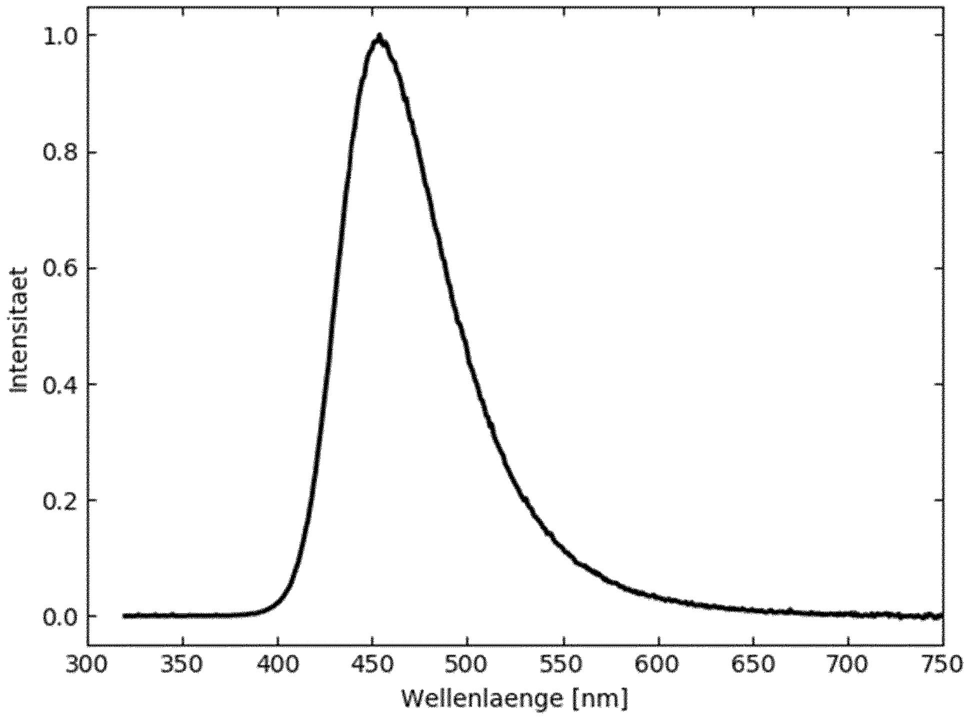


**Figur 2**

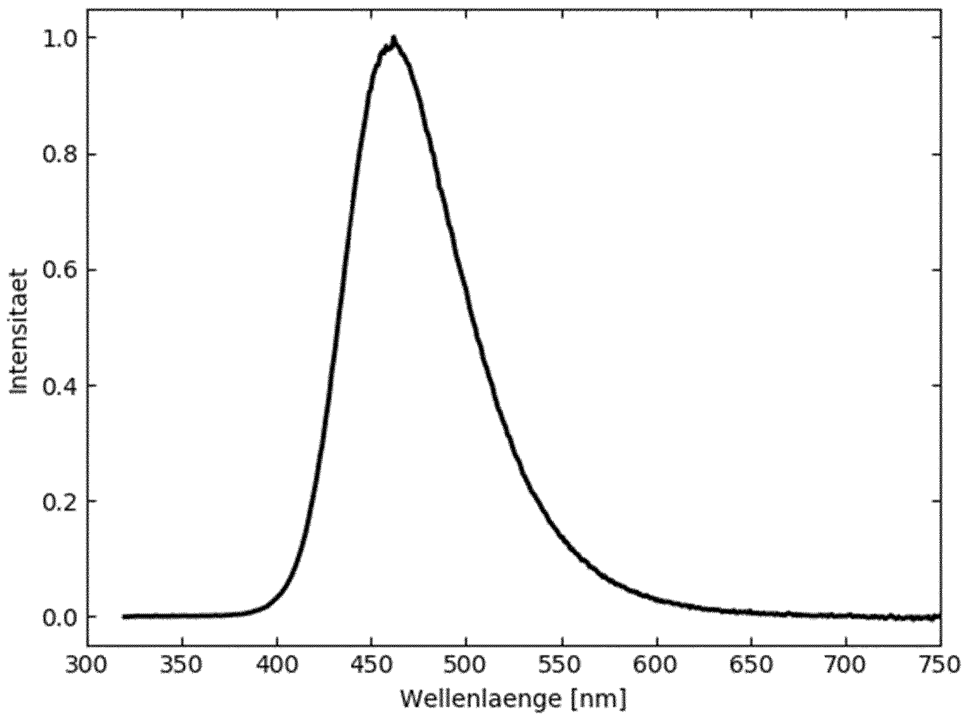

Figur 3

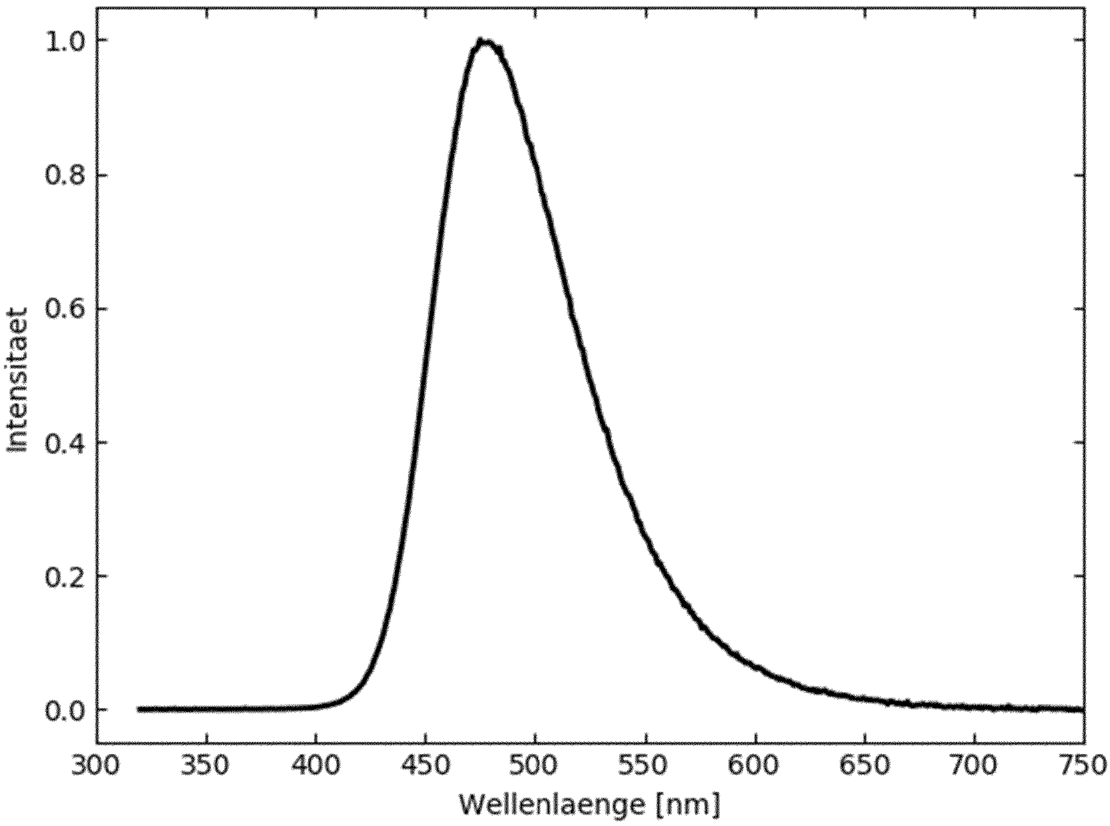
Intensitaet
1.0
0.8
0.6
0.4
0.2
0.0
300
350
400
450
500
550
600
650
700
750
Wellenlaenge [nm]

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- **M.E. THOMPSON et al.** *Chem. Mater.,* 2004, vol. 16, 4743
- **OKINAKA et al.** Invited Paper: New Fluorescent Blue Host Materials for Achieving Low Voltage in OLEDs. *SID Symposium Digest of Technical Papers,* 2015, vol. 46
- *CHEMICAL ABSTRACTS,* 73183-34-3